# EUROPEAN PATENT SPECIFICATION

(11) **EP 2 057 122 B1**
(45) Date of publication and mention of the grant of the patent: **01.09.2010**
(21) Application number: 07819060.0
(22) Date of filing: 17.10.2007
(51) Int. Cl.: C07D 215/22, C07C 217/60, A61K 31/435, A61K 31/137, A61P 9/00, A61P 11/06, A61P 29/00, A61P 27/06

(54) **DERIVATIVES OF 4-(2-AMINO-1-HYDROXYETHYL)PHENOL AS AGONISTS OF THE 2 ADRENERGIC RECEPTOR**
4-( 2-AMINO-1-HYDROXYETHYL)- PHENOLDERIVATIVE ALS AGONISTEN DES 2-ADRENERGISCHEN REZEPTORS
DÉRIVÉS DE 4-(2-AMINO-1-HYDROXYÉTHYL)PHÉNOL EN TANT QU'AGONISTES DU RÉCEPTEUR ADRÉNERGIQUE 2

(30) Priority: 20.10.2006 ES 200602676
(43) Date of publication of application: 13.05.2009
(73) Proprietor: Almirall, S.A., 08022 Barcelona (ES)
(72) Inventor: BACH TANA, Jordi, 08500 Vic (ES); CRESPO CRESPO, Maria Isabel, 08036 Barcelona (ES); PUIG DURAN, Carlos, 08024 Barcelona (ES); GUAL ROIG, Silvia, 08349 Cabrera de Mar (ES); ORTEGA MUÑOZ, Alberto, Barcelona (ES)
(74) Representative: Srinivasan, Ravi Chandran
(86) International application number: PCT/EP2007/008992
(87) International publication number: WO 2008/046598

(56) References cited:
- EP-B1- 1 235 787
- WO-A-03/042160
- WO-A-03/099764
- WO-A-2004/089892
- WO-A-2005/121065
- WO-A-2006/023457
- WO-A1-99/64035
- US-A- 4 022 776
- US-A1- 2002 055 651
- US-A1- 2003 153 597
- US-A1- 2004 167 167
- US-A1- 2005 192 316
- US-A1- 2005 272 769
- US-A1- 2006 019 991
- US-A1- 2006 035 931
- US-A1- 2006 178 410
- DEYRUP M D ET AL: "STRUCTURE-AFFINITY PROFILE OF 8-HYDROXYCARBOSTYRIL-BASED AGONISTS THAT DISSOCIATE SLOWLY FROM THE BETA2-ADRENOCEPTOR" NAUNYN-SCHMIEDEBERG'S ARCHIVES OF PHARMACOLOGY, SPRINGER, BERLIN, DE, vol. 359, no. 3, 1999, pages 168-177, XP001199515 ISSN: 0028-1298

## Description

### FIELD OF THE INVENTION

The present invention is directed to novel β2 adrenergic receptor agonists. The invention is also directed to pharmaceutical compositions comprising such compounds, methods of using such compounds to treat diseases associated with β2 adrenergic receptor activity, and processes and intermediates useful for preparing such compounds.

### BACKGROUND OF THE INVENTION

β2 adrenergic receptor agonists are recognized as effective drugs for the treatment of pulmonary diseases such as asthma and chronic obstructive pulmonary disease (including chronic bronchitis and emphysema). β2 adrenergic receptor agonists are also useful for treating pre-term labor, glaucoma and are potentially useful for treating neurological disorders and cardiac disorders.

WO-A-03099764 discloses alkoxy aryl b2 adrenergic receptor agonists.

In spite of the success that has been achieved with certain β2 adrenergic receptor agonists, current agents possess less than desirable potency, selectivity, onset, and/or duration of action. Thus, there is a need for additional β2 adrenergic receptor agonists having improved properties. Preferred agents may possess, among other properties, improved potency, selectivity, onset, improved safety margins, improved therapeutic window and/or duration of action.

### SUMMARY OF THE INVENTION

The invention provides novel compounds that possess β2 adrenergic receptor agonist activity. Accordingly, there is provided a compound of the invention which is a compound of formula (I): 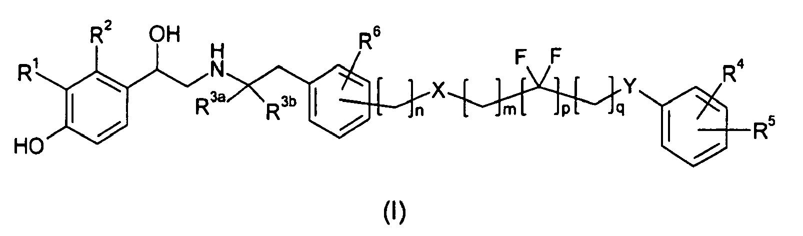 wherein:
- R¹ is a group selected from -CH₂OH, -NH(CO)H and
- R² is a hydrogen atom; or
- R¹ together with R² form the group -NH-C(O)-CH=CH-, wherein the nitrogen atom is bound to the carbon atom in the phenyl ring holding R¹ and the carbon atom is bound to the carbon atom in the phenyl ring holding R²
- R^{3a} and R^{3b} are independently selected from the group consisting of hydrogen atoms and C₁₋₄ alkyl groups
- X and Y are independently selected from the group consisting of direct bond and oxygen atom
- n, m and q each independently has a value selected from 0, 1, 2 and 3
- p has a value selected from 1, 2 and 3
- R⁴ and R⁵ are independently selected from hydrogen atoms, halogen atoms, C₁₋₄ alkyl, C₁₋₄ alkoxy, -CONH₂, -NHCONH₂, -SR⁷, -SOR⁷, -SO₂R⁷, -SO₂NHR⁸ and the groups 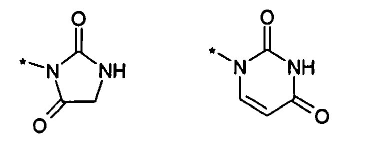 wherein R⁷ is selected from C₁₋₄ alkyl and C₃₋₈ cycloalkyl and R⁸ is selected from hydrogen atoms and C₁₋₄ alkyl groups
- R⁶ is selected from the group consisting of hydrogen atoms, halogen atoms, C₁₋₄ alkyl and C₁₋₄ alkoxy
or a pharmaceutically-acceptable salt or solvate or stereoisomer thereof.

The invention also provides a pharmaceutical composition comprising a compound of the invention and a pharmaceutically-acceptable carrier. The invention further provides combinations comprising a compound of the invention and one or more other therapeutic agents and pharmaceutical compositions comprising such combinations.

In separate and distinct aspects, the invention also provides synthetic processes and intermediates described herein, which are useful for preparing compounds of the invention.

The invention also provides a compound of the invention as described herein for use in medical therapy, as well as the use of a compound of the invention in the manufacture of a formulation or medicament for treating a disease or condition associated with β2 adrenergic receptor activity (e.g. a pulmonary disease, such as asthma or chronic obstructive pulmonary disease, pre-term labor, glaucoma, a neurological disorder, a cardiac disorder, or inflammation) in a mammal.

### DETAILED DESCRIPTION OF THE INVENTION

When describing the compounds, compositions and methods of the invention, the following terms have the following meanings, unless otherwise indicated.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with β2 adrenergic receptor activity" includes all disease states and/or conditions that are acknowledged now, or that are found in the future, to be associated with β2 adrenergic receptor activity. Such disease states include, but are not limited to, pulmonary diseases, such as asthma and chronic obstructive pulmonary disease (including chronic bronchitis and emphysema), as well as neurological disorders and cardiac disorders. β2 adrenergic receptor activity is also known to be associated with pre-term labor (see International Patent Application Publication Number WO 98/09632), glaucoma and some types of inflammation (see International Patent Application Publication Number WO 99/30703 and Patent Application Publication Number EP 1 078 629).

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, fumaric, gluconic, glutamic, hydrobromic, hydrochloric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

The term "solvate" refers to a complex or aggregate formed by one or more molecules of a solute, i.e. a compound of the invention or a pharmaceutically-acceptable salt thereof, and one or more molecules of a solvent. Such solvates are typically crystalline solids having a substantially fixed molar ratio of solute and solvent. Representative solvents include by way of example, water, methanol, ethanol, isopropanol, acetic acid, and the like. When the solvent is water, the solvate formed is a hydrate.

It will be appreciated that the term "or a pharmaceutically-acceptable salt or solvate of stereoisomer thereof" is intended to include all permutations of salts, solvates and stereoisomers, such as a solvate of a pharmaceutically-acceptable salt of a stereoisomer of a compound of formula (I).

The term "amino-protecting group" refers to a protecting group suitable for preventing undesired reactions at an amino nitrogen. Representative amino-protecting groups include, but are not limited to, formyl; acyl groups, for example alkanoyl groups, such as acetyl; alkoxycarbonyl groups, such as tert-butoxycarbonyl (Boc); arylmethoxycarbonyl groups, such as benzyloxycarbonyl (Cbz) and 9-fluorenylmethoxycarbonyl (Fmoc); arylmethyl groups, such as benzyl (Bn), trityl (Tr), and 1,1-di-(4'-methoxyphenyl)methyl; silyl groups, such as trimethylsilyl (TMS) and tert-butyldimethylsilyl (TBS); and the like.

The term "hydroxy-protecting group" refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl; arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

The compounds of the invention contain at least a chiral center. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

In an embodiment the compounds of formula (I) have a p value of 1

In another embodiment the compounds of formula (I) have an n value of 0.

In still another embodiment the compounds of formula (I) have an m value of 1 or 2, preferably of 1.

In yet another embodiment the compounds of formula (I) have a q value of 0 or 1, preferably of 0.

In another embodiment in the compounds of formula (I) X represents a oxygen atom.

In still another embodiment in the compounds of formula (I) Y represents a direct bond.

In still another embodiment in the compounds of formula (I) R^{3a} represents a hydrogen atom and R^{3b} is selected from the group consisting of hydrogen atom and methyl group, preferably both R^{3a} and R^{3b} represent a hydrogen atom.

In yet another embodiment in the compounds of formula (I) R⁴ represents a hydrogen atom.

In another embodiment in the compounds of formula (I) R⁴ represents a hydrogen atom and R⁵ is selected from hydrogen atoms, halogen atoms, -CONH₂, - NHCONH₂, -SR⁷, -SOR⁷, -SO₂R⁷ and -SO₂NHR⁸, more preferably, R⁴ represents a hydrogen atom and R⁵ is a group selected from hydrogen atoms and groups - CONH₂ and -NHCONH₂.

In still another embodiment in the compounds of formula (I) R⁶ is selected from the group consisting of hydrogen atom, fluorine atom, methyl group and methoxy group, preferably R⁶ represents a hydrogen atom and a methoxy group, most preferably a hydrogen atom.

In another embodiment in the compounds of formula (I) R¹ together with R² form the group -NH-C(O)-CH=CH-, wherein the nitrogen atom is bound to the carbon atom in the phenyl ring holding R¹ and the carbon atom is bound to the carbon atom in the phenyl ring holding R².

In still another embodiment in the compounds of formula (I) R¹ together with R² form the group -NH-C(O)-CH=CH-, wherein the nitrogen atom is bound to the carbon atom in the phenyl ring holding R¹ and the carbon atom is bound to the carbon atom in the phenyl ring holding R², n and q have a value of 0 and m and p have a value of 1.

In yet another embodiment in the compounds of formula (I) X represents an oxygen atom, Y represents a direct bond and R⁴, R⁵ and R⁶ independently represent hydrogen atoms.

In still another embodiment in the compounds of formula (I) R¹ together with R² form the group -NH-C(O)-CH=CH-, wherein the nitrogen atom is bound to the carbon atom in the phenyl ring holding R¹ and the carbon atom is bound to the carbon atom in the phenyl ring holding R², n and q have a value of 0, m and p have a value of 1, X represents an oxygen atom, Y represents a direct bond and R⁴, R⁵ and R⁶ independently represent hydrogen atoms.

Particular individual compounds of the invention include:
5-[(1*R*,*S*)-2-({(1*R*,S)-2-[4-(2,2-difluoro-2-phenylethoxy)phenyl]-1-methyl ethyl}amino)-1-hydroxyethyl]-8-hydroxyquinolin-2(1*H*)-one
4-[(1*R*,*S*)-2-({(1*R*,*S*)-2-[4-(2,2-Difluoro-2-phenylethoxy)phenyl]-1-methyl ethyl}amino)-1-hydroxyethyl]-2-(hydroxymethyl)phenol acetate
Formic acid - {5-[(1*R*,*S*)-2-({(1*R*,*S*)-2-[4-(2,2-Difluoro-2-phenylethoxy) phenyl]-1-methylethyl}amino)-1-hydroxyethyl]-2-hydroxyphenyl}formamide (1:1)
5-((1*R*)-2-({(1*R*,*S*)-2-[3-(2,2-difluoro-2-phenylethoxy)phenyl]-1-methylethyl}amino)-1-hydroxyethyl]-8-hydroxyquinolin-2(1*H*)-one
4-[(1*R*)-2-({(1*R*,*S*)-2-[3-(2,2-difluoro-2-phenylethoxy)phenyl]-1-methylethyl}amino)-1-hydroxyethyl]-2-(hydroxymethyl)phenol
4-{(1*R*)-2-[((1*R*,*S*)-2-{3-[(2,2-Difluoro-2-phenylethoxy)methyl]phenyl}-1-methylethyl)amino]-1-hydroxyethyl}-2-(hydroxymethyl)phenol acetate
5-[2-({2-[(1*R*,S)-4-(2,2-difluoro-2-phenylethoxy)phenyl]ethyl}amino)-1-hydroxyethyl]-8-hydroxyquinolin-2(1H)-one
5-[(1*R*)-2-({2-[4-(2,2-Difluoro-2-phenylethoxy)phenyl]ethyl}amino)-1-hydroxyethyl]-8-hydroxyquinolin-2(1*H*)-one
4-[(1*R*,*S*)-2-({2-[4-(2,2-Difluoro-2-phenylethoxy)phenyl]ethyl}amino)-1-hydroxyethyl]-2-(hydroxymethyl)phenol
{5-[(1*R*,*S*)-2-({2-[4-(2,2-Difluoro-2-phenylethoxy)phenyl]-1-methylethyl} amino)-1-hydroxyethyl]-2-hydroxyphenyl}formamide - formate
5-[(1*R*,*S*)-2-({2-[3-(2,2-Difluoro-2-phenylethoxy)phenyl]ethyl}amino)-1-hydroxyethyl]-8-hydroxyquinolin-2(1*H*)-one
5-[(1R)-2-({2-[3-(2,2-difluoro-2-phenylethoxy)phenyl]ethyl}amino)-1-hydroxyethyl]-8-hydroxyquinolin-2(1H)-one
4-[(1*R*,*S*)-2-({2-[3-(2,2-Difluoro-2-phenylethoxy)phenyl]ethyl}amino)-1-hydroxyethyl]-2-(hydroxymethyl)phenol
{5-[(1*R*,*S*)-2-({2-[3-(2,2-Difluoro-2-phenylethoxy)phenyl]ethyl}amino)-1-hydroxyethyl]-2-hydroxyphenyl}formamide
5-{(1*R*,*S*)-2-[(2-{4-[2,2-Difluoro-2-(2-methoxyphenyl)ethoxy]phenyl} ethyl)amino]-1-hydroxyethyl}-8-hydroxyquinolin-2(1*H*)-one
5-[(1*R*)-2-({(1*R*,*S*)-2-[4-(2,2-difluoro-3-phenylpropoxy)phenyl]-1-methyl-ethyl}amino)-1-hydroxyethyl]-8-hydroxyquinolin-2(1*H*)-one
5-[(1*R*,*S*)-[2-{[4-(3,3-Difluoro-3-phenylpropoxy)benzyl]amino}-1-hydroxyethyl)]-8-hydroxyquinolin-2(1H)-one
5-[(1*R*,*S*)-[2-({2-[4-(2,2-difluoro-3-phenoxypropoxy)phenyl]ethyl}-amino)-1-hydroxyethyl]-8-hydroxyquinolin-2(1*H*)-one
5-[(1*R*,*S*)-[[2-({2-[4-(2,2-difluoro-2-phenylethoxy)-3-methoxyphenyl]-ethyl}amino)-1-hydroxyethyl]-8-hydroxyquinolin-2(1*H*)-one, formate
5-[(1*R*,*S*)-2-({2-[4-(2,2-difluoro-3-phenylpropoxy)phenyl]ethyl}amino)-1-hydroxyethyl]-8-hydroxyquinolin-2(1*H*)-one, formate
5-[(1R,S)-2-({2-[4-(2,2-difluoro-4-phenylbutoxy)phenyl]-1-methylethyl}amino)-1-hydroxyethyl]-8-hydroxyquinolin-2(1H)-one,
5-[(1 R)-2-({(1*R*,*S*)-2-[4-(1,1-difluoro-2-phenoxyethyl)phenyl]-1-methylethyl}amino)-1-hydroxyethyl]-8-hydroxyquinolin-2(1H)-one
5-[(1R)-2-({2-[4-(3,3-difluoro-3-phenylpropoxy)phenyl]ethyl}amino)-1-hydroxyethyl]-8-hydroxyquinolin-2(1H)-one
5-[(1*R*,*S*)-2-({2-[4-(4,4-difluoro-4-phenylbutoxy)phenyl]ethyl}amino)-1-hydroxyethyl]-8-hydroxyquinolin-2(1H)-one
5-[(1*R*,*S*)-2-({2-[4-(2,2-difluoro-2-phenylethoxy)-3-methylphenyl]ethyl}amino)-1-hydroxyethyl]-8-hydroxyquinolin-2(1H)-one
5-[(1*R*,*S*)-2-({2-[4-(2,2-difluoro-2-phenylethoxy)-3-fluorophenyl]ethyl}amino)-1-hydroxyethyl]-8-hydroxyquinolin-2(1H)-one
3-{1,1-difluoro-2-[4-((1*R*,*S*)-2-{[2-hydroxy-2-(8-hydroxy-2-oxo-1,2-dihydroquinolin-5-yl)ethyl]amino}ethyl)phenoxy]ethyl}benzamide
N-((1*R*,*S*)-3-{1,1-difluoro-2-[3-(2-{[(2R)-2-hydroxy-2-(8-hydroxy-2-oxo-1,2-dihydroquinolin-5-yl)ethyl]amino}propyl)phenoxy]ethyl}phenyl)urea
5-{(1*R*,*S*)-2-[(2-{4-[2,2-difluoro-2-(3-fluorophenyl)ethoxy]phenyl}ethyl)amino]-1-hydroxyethyl}-8-hydroxyquinolin-2(1H)-one
5-((1*R*,*S*)-(2-{[2-(4-{2-[3-(cyclopentylthio)phenyl]-2,2-difluoroethoxy}phenyl)ethyl]amino}-1-hydroxyethyl)-8-hydroxyquinolin-2(1H)-one
5-((1R,S)-(2-{[2-(4-{2-[3-(cyclopentylsulfonyl)phenyl]-2,2-difluoroethoxy}phenyl)ethyl]amino}-1-hydroxyethyl)-8-hydroxyquinolin-2(1H)-one
5-[(1*R*,*S*)-2-({2-[4-(2,2-difluoro-2-phenylethoxy)phenyl]-1,1-dimethylethyl}amino)-1-hydroxyethyl]-8-hydroxyquinolin-2(1H)-one
and pharmaceutically-acceptable salts and solvates thereof.

Of outstanding interest are the compounds:
4-[(1*R*,*S*)-2-({(1*R*,*S*)-2-[4-(2,2-Difluoro-2-phenylethoxy)phenyl]-1-methyl ethyl}amino)-1-hydroxyethyl]-2-(hydroxymethyl)phenol acetate
Formic acid - {5-[(1*R*,*S*)-2-({(1*R*,*S*)-2-[4-(2,2-Difluoro-2-phenylethoxy) phenyl]-1-methylethyl}amino)-1-hydroxyethyl]-2-hydroxyphenyl} formamide (1:1)
5-[2-({2-[(1*R*,*S*)-4-(2,2-difluoro-2-phenylethoxy)phenyl]ethyl}-amino)-1-hydroxyethyl]-8-hydroxyquinolin-2(1*H*)-one
5-[(1*R*)-2-({2-[4-(2,2-Difluoro-2-phenylethoxy)phenyl]ethyl}amino)-1-hydroxyethyl]-8-hydroxyquinolin-2(1*H*)-one
5-{(1*R*,*S*)-2-[(2-{4-[2,2-Difluoro-2-(2-methoxyphenyl)ethoxy]phenyl} ethyl)amino]-1-hydroxyethyl}-8-hydroxyquinolin-2(1*H*)-one
5-[(1*R*,*S*)-2-{[4-(3,3-Difluoro-3-phenylpropoxy)benzyl]amino}-1-hydroxyethyl)]-8-hydroxyquinolin-2(1H)-one
5-{(1*R*,*S*)-2-[(2-{4-[2,2-difluoro-2-(3-fluorophenyl)ethoxy]phenyl}-ethyl)amino]-1-hydroxyethyl}-8-hydroxyquinolin-2(1H)-one
5-[(1*R*,*S*)-2-({2-[4-(2,2-difluoro-2-phenylethoxy)phenyl]-1,1-dimethylethyl}amino)-1-hydroxyethyl]-8-hydroxyquinolin-2(1H)-one

The invention comprises also pharmaceutical compositions comprising a therapeutically effective amount of a compound as hereinabove defined and a pharmaceutically acceptable carrier.

In an embodiment of the present invention the pharmaceutical composition further comprises a therapeutically effective amount of one or more other therapeutic agents.

It is also an embodiment of the present invention that the pharmaceutical composition is formulated for administration by inhalation.

The compounds of the present invention as hereinabove defined may also be combined with one or more other therapeutic agents, in particular one or more drugs selected from the group consisting of corticosteroids, an antichlolinergic agents and PDE4 inhibitors.

In a preferred embodiment of the present invention the combination comprises a compound of formula (I) as hereinabove defined and a drug selected from the group consisting of fluticasone propionate, 6α,9α-difluoro-17α-[-(2-furanylcarbonyl)oxy]-11β-hydroxy-16α-methyl-3-oxo-androsta-1,4-diene-17β-carbothioic acid S-fluoromethyl ester, and 6α,9α-difluoro-11β-hydroxy-16α-methyl-3-oxo-17α-propionyloxy-androsta-1,4-diene-17β-carbothioic acid S-(2-oxo-tetrahydro-furan-3S-yl) ester, mometasone furoate, 3(R)-(2-hydroxy-2,2-dithien-2-ylacetoxy)-1-(3-phenoxypropyl)-1-azoniabicyclo[2.2.2]octane bromide and (3R)-1-phenetyl-3-(9H-xanthene-9-carbonyloxy)-1-azoniabicyclo[2.2.2]octane bromide.

The invention is also directed to a compound or composition of the invention for use in treating a disease or condition in a mammal associated with β2 adrenergic receptor activity. It is of particular relevance when the disease or condition is a pulmonary disease, preferably asthma or chronic obstructive pulmonary disease.

The disease can also be a disease or condition selected from the group consisting of pre-term labor, glaucoma, neurological disorders, cardiac disorders, and inflammation.

### General Synthetic Procedures

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal, are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

In general terms the compounds of formula (I) may be obtained by reaction of a compound of formula (II) wherein: 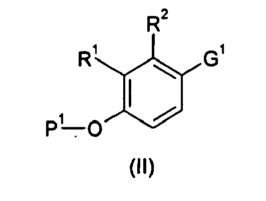
R¹ is a group selected from -CH₂OH, -NH-C(O)H and R² is a hydrogen atom or
R¹ together with R² form the group -NH-C(O)-CH=CH- wherein the nitrogen atom is bound to the carbon atom in the phenyl ring holding R¹ and the carbon atom is bound to the carbon atom in the phenyl ring holding R² and
P¹ is a conventional hydroxy protecting group such as benzyl group or *p-*methoxybenzyl group or
R¹ together with P¹ form the group -CH₂O-C(CH₃)₂- wherein the carbon atom bearing two hydrogen atoms is bound to the carbon atom in the phenyl ring holding R¹ and the carbon atom bearing two methyl groups is bound to the oxygen atom holding P¹ and R² is a hydrogen atom
with a compound of formula (III): 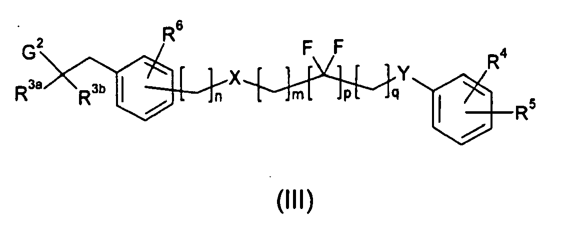 wherein:
R^{3a}, R^{3b} are independently selected from hydrogen atoms and lower alkyl groups and G² is a group selected from -NH₂ and NHP² wherein P² is a conventional amino protecting group such as benzyl group
   or
R^{3a} is a hydrogen atom or a lower alkyl group and R^{3b} together with G²:form and =O (oxo) group and
R⁴ and R⁵ are selected from hydrogen or halogen atoms or groups selected from C₁₋₄ alkyl, C₁₋₄ alkoxy, -CONH₂,-NHCONH₂, -SR⁷, -SOR⁷, -SO₂R⁷, SO₂NHR⁸ and the groups 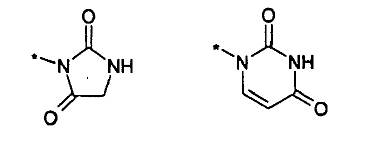
R⁶ is selected from hydrogen or halogen atoms or groups selected from C₁₋₄ alkyl and C₁₋₄ alkoxy
R⁷ is a C₁₋₄ alkyl or C₃₋₆ cycloalkyl group
R⁸ a hydrogen atom or a C₁₋₄ alkyl group
and X, Y, m, n, p and q are as defined above

The nature of the reacting groups G¹ and G² depends on the coupling reaction that is employed to obtain the compounds of formula (I). The different coupling reactions between compound (II) (the phenylethanolamine moiety) and the corresponding compound (III) (the fluorinated tail) are summarised in Scheme 1 and described below. 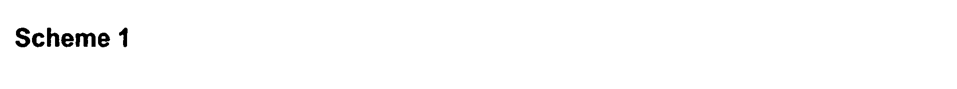 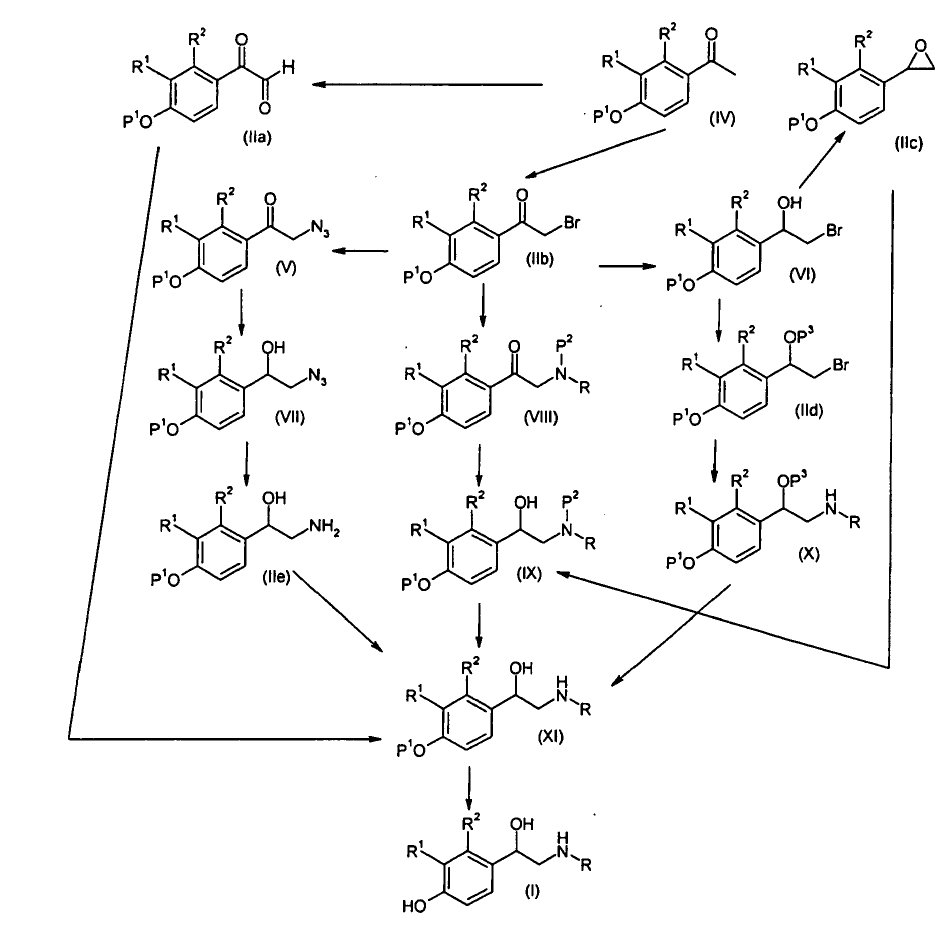 wherein R represents a group of formula:

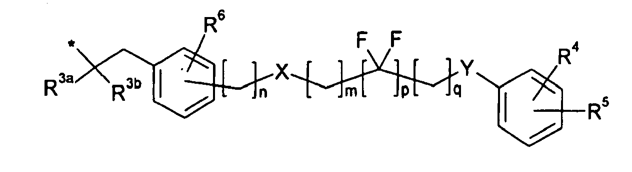

In a first alternative, phenylglyoxals of formula (IIa) (corresponding to compounds of general formula II wherein G¹ is a -CO-CHO group) may react with a compound of formula (IIIa) (corresponding to compounds of general formula (III) wherein G² is a group -NH₂) to give, in a reductive alkylation step, intermediates of formula (XI).

This step may be achieved in a variety of solvents, such as tetrahydrofuran, alcohols as methanol, ethanol or isopropyl alcohol, as well as a mixture of solvents such as methanol/tetrahydrofuran, ethanol/tetrahydrofuran or dimethylsulfoxide/methanol, the temperature range being between 5° and 100° C; more specifically between 15° and 70° C. The reducing agent may be a hydride such as sodium borohydride, sodium cyanoborohydride or sodium triacetoxyborohydride as well as hydrogen plus a hydrogenation catalyst such as palladium on charcoal.

In a second alternative, aminoalcohols of formula (IIe) (corresponding to compounds of general formula II wherein G¹ is a -CH(OH)CH₂NH₂ group) may react with an aldehyde or ketone of formula (IIIb) (corresponding to compounds of general formula III wherein R^{3b} together with G² form a group =O) to give, in an analogous reductive alkylation process, the same intermediates of formula (XI). This step is carried out under similar conditions and solvents as the previously described.

In a third alternative, the phenacyl bromides of formula (IIb) (corresponding to compounds of general formula II wherein G¹ is a -CO-CH₂-Br group) may react with protected amines of formula (IIIc) (corresponding to compounds of general formula III wherein G² is a group NHP² (being P² a conventional amine protecting group such as a benzyl group)) to give ketoamines of formula (VIII). This process may be carried out in many solvents such as tetrahydrofuran or dichloromethane in the presence of an acid scavenger such as a tertiary amine as triethylamine, and at temperatures between 5 and 60° C. The compounds of formula (VIII) may then be reduced to yield the aminoalcohols of formula (IX). The reaction may be carried out in a solvent such as tetrahydrofuran, methanol, ethanol or isopropyl alcohol or in a mixture of solvents such as methanol/tetrahydrofuran, ethanol/tetrahydrofuran or dimethylsulfoxide/methanol and at a temperature from 5° to 100° C. The reducing agent may be a hydride such as sodium borohydride, sodium triacetoxyborohydride or sodium cyanoborohydride as well as hydrogen plus a hydrogenation catalyst such as palladium on charcoal. Finally the protecting group P² -usually being a benzyl group- may be removed by means of hydrogenation with palladium on charcoal or platinum dioxide as catalysts in a solvent such as methanol, ethanol, ethyl acetate, acetic acid or dimethylformamide, in a neutral or slightly acidic media, at a temperature from room temperature to 70 °C and at a pressure from 1 to 3 bar to yield the compounds of formula (XI).

In a forth alternative, protected bromohydrins of formula (IId) (corresponding to compounds of general formula II wherein G¹ is a group -CH(OP³)-CH₂-Br (P³ being a conventional hydroxy protecting group such as a silyl ether)) may alkylate primary amines of formula (IIa) (corresponding to compounds of general formula III wherein G² is a group -NH₂) to give intermediates of formula (X). This reaction is carried out in the presence of an acid scavenger, such as a tertiary amine, potassium carbonate or sodium bicarbonate, in a variety of solvents such as dioxane, dimethylsulfoxide or also without solvent and in a range of temperatures between 60 ° and 140°C. The removal of the protecting group P³, usually a silyl ether, is achieved by means of the fluoride anion, for example in the form of a quaternary ammonium salt such as tetrabutylammonium fluoride, to give intermediates of formula (XI).

In a fifth alternative, epoxides of formula (IIc) (corresponding to compounds of general formula II wherein G¹ is an oxyran group) may also react with protected amines of formula (IIIc) (corresponding to compounds of general formula III wherein G² is a group -NHP² (being P² a conventional amine protecting group such as a benzyl group)) to give intermediates of formula (IX). This process may be carried out in many solvents such as alcohols, tetrahydrofuran or without solvents at all, in a range of temperatures between 20° and 140 °C.

As a final step, compounds of formula (XI) are deprotected to the target compounds of formula (I) by conventional methods. When the protecting group P¹ is a benzyl group, the debenzylation is carried out with hydrogen and a hydrogenation catalyst such as palladium on charcoal. This step is achieved using a variety of solvents such as alcohols, tetrahydrofuran or mixtures of them and in a neutral or slightly acidic media. The pressure of hydrogen lies between 0,6 and 3 bar and the temperature between 10 ° and 30 °C. When the protecting group P¹ is a p-methoxybenzyl group, this may be cleaved by hydrogenolysis (using the same catalysts and conditions described above) or by treatment with an acid, for example acetic acid, trifluoroacetic acid or hydrochloric acid, optionally in the presence of a solvent such as water, methylene chloride, chloroform, tetrahydrofuran or dioxane and a temperature from room temperature to the boiling point of the solvent. If R¹ together with P¹ form the group -CH₂O-C(CH₃)₂-, deprotection of the isopropylidene acetal group may be achieved by treatment with an acid (using the same acids and conditions as described above).

The intermediates of formulae (IIa), (IIb), (IIc), (IId) and (IIe) are either commercially available or may be obtained by methods well known in the literature starting from the phenylglyoxals of formula (IIa) or the corresponding hydrates-prepared from the corresponding acetophenones of formula (IV) (for ex., see EP 147719, example 2; US 4,753,962 description 54 or GB 1247370, example 1).

For example the phenylethanolamines of formula (IIe) may be obtained following methods described in J. Med. Chem., 1976, 19(9), 1138, compound 19; DE 2461861, example 24. The phenacyl bromides of formula (IIb) may be obtained following methods described in Chem. Pharm. Bull., 1977, 25(6), 1368, compound II; J. Med. Chem., 1974, 17(1), 49; EP 147719, example 1. The protected bromohydrines of formula (IId) may be obtained following methods described in the document US2004059116, example 9C; the document WO2004/011416, example 2 and the document WO2004/016578, example 1ii. The oxyranes of formula (IIc) may be obtained following methods described in WO 01036375, preparation 12; J. Med. Chem., 1974, 17(1), 55.

Many of these intermediates may also exist in an enantiomerically pure form (see, for ex., Organic Process Research & Development 1998, 2, 96; Tetrahedron Lett., 1994, 35(50), 9375; WO 02070490 example 1/X; EP 0147719).

As it has been explained before, the nature of the G² group in the compounds of formula (III) depends on the coupling reaction followed to obtain compounds (I) of the present invention on one side and on the nature of groups R^{3a}, R^{3b}, R⁴, R⁵ and R⁶ on the other side.

**Schemes 2, 3, 4** and **5** illustrate the preparation of compounds of formula (III) having different G², R^{3a}, R^{3b}, R⁴ and R⁵ groups:

The path shown in Scheme 2 may be used to obtain the compounds of formula (IIIe) (corresponding to the compounds of formula (III)) wherein G² is a -NH₂ group, R^{3b} is hydrogen R^{3a}, is C₁₋₄ alkyl, each R⁴ and R⁵ are independently selected from the group consisting of hydrogen, fluorine atom, C₁₋₄ alkyl, C₁₋₄ alkoxy, -CONH₂, -SR⁷, -SOR⁷, -SO₂R⁷, -SO₂NHR⁸ and R⁶ selected from the group comprising hydrogen, C₁₋₄ alkyl, C₁₋₄ alkoxy. 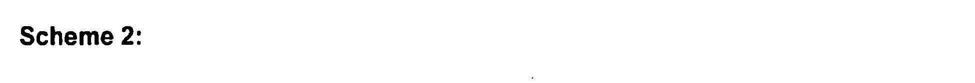 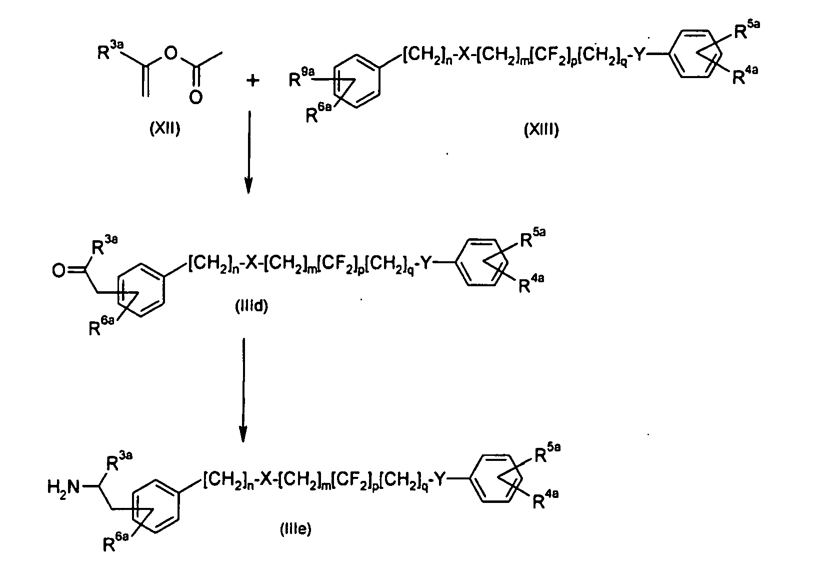 wherein:
R^{3a} is a C₁₋₄ alkyl
R^{4a} and R^{5a} are selected from hydrogen or fluorine atom or groups selected from C₁₋₄ alkyl or C₁₋₄ alkoxy, -CONH₂, -S-R⁷, -SOR⁷, -SO₂R⁷ and -SO₂NHR⁸
R^{6a} is selected from hydrogen atom or groups selected from C₁₋₄ alkyl and C₁₋₄ alkoxy
R^{9a} represents a halogen atom or an alkyl or aryl sulfonate such as methylsulfonate, trifluromethylsulfonate or p-toluensulfonate
and R⁷, n, m, p, q, X and Y are as defined above

Ketones of formula (IIId) (corresponding to compounds of general formula III wherein R^{3b} together with G² form and =O (oxo) group) may be prepared by a palladium-mediated coupling of an aryl halide or sulfonate of formula (XIII) wherein R^{9a} stands for a halogen atom or a sulfonate group. In a standard procedure, the compound of formula (XIII) is reacted with a tin enolate generated in-situ by treatment of a substituted vinylacetate of formula (XII) with tri-n-butyltin methoxide in the presence of a suitable palladium catalyst such as palladium acetate and a phospine such as tri-ortho-tolylphosphine in a non-polar solvent such as toluene. Preferably, the reaction is carried out at a temperature comprised between 80 °C and 110 °C.

Ketones of formula (IIId) (corresponding to compounds of general formula III wherein R^{3b} together with G² form and =O (oxo) group) may be easily converted to the corresponding amines of formula (IIIe) (corresponding to compounds of general formula III wherein R^{3b} is a hydrogen atom and G² is a -NH₂ group) by reaction with ammonium acetate or ammonium hydroxide in the presence of a reducing agent. The reaction may be carried out in a variety of solvents such as tetrahydrofuran, alcohols as methanol, ethanol or isopropyl alcohol, as well as a mixture of solvents such as methanol/tetrahydrofuran or ethanol/tetrahydrofuran. The temperature range may be between 5 °C and 100 °C but more specifically between 15 °C and 90 °C. The reducing agent may be a hydride such as sodium borohydride, sodium cyanoborohydride or sodium triacetoxyborohydride as well as hydrogen plus a hydrogenation catalyst such as Raney® Nickel.

In the special case of compounds of formula (IIIe2) (corresponding to compounds of general (III) wherein G² is a -NH₂ group, R^{3b} is hydrogen and R⁵ is -NHCONH₂) or the groups: 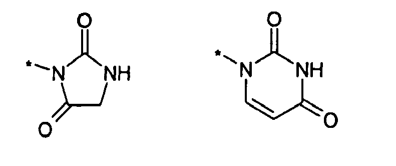 the path shown in Scheme 3 may be used. 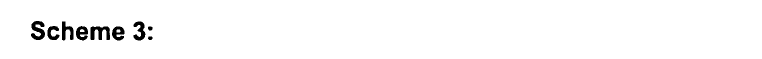 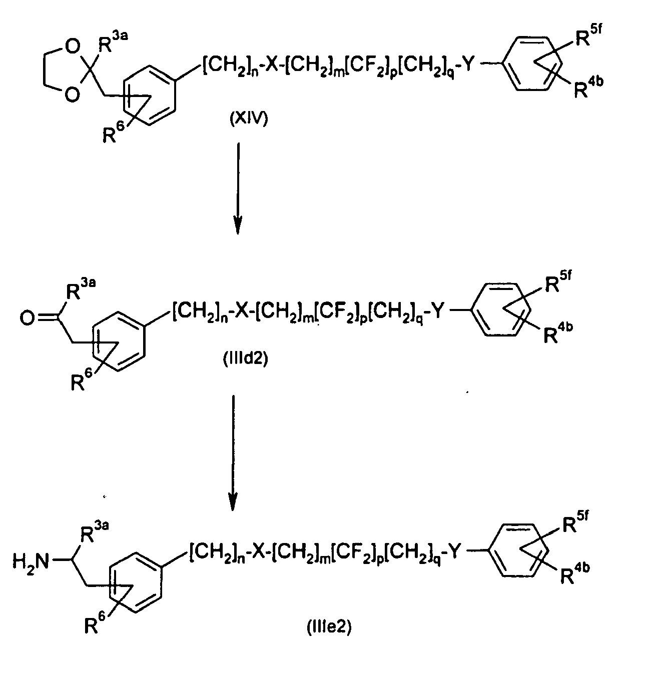 wherein:
R^{3a} is a C₁₋₄ alkyl
R^{4b} is selected from hydrogen or halogen atoms or groups selected from C₁₋₄ alkyl or C₁₋₄ alkoxy, -CONH₂, -SR⁷, -SOR⁷, -SO₂R⁷ and -SO₂NHR⁸,
R^{5f} is selected from -NHCONH₂ or the groups: 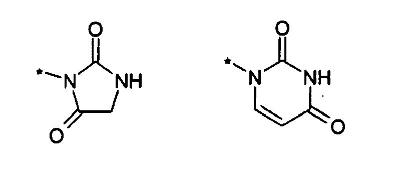
and R⁶, R⁷, n, m, p, q, X and Y are as defined above

Ketones of formula (IIId2) (corresponding to compounds of general formula III wherein R^{3b} together with G² form an =O (oxo) group) may be prepared by deprotection of the corresponding acetals of formula (XIV). Deprotection may be carried out by treatment with an acid such as hydrochloric acid or sulphuric acid in a solvent such as water, methanol, ethanol or tetrahydrofuran and a temperature from room temperature to the boiling point of the solvent.

Ketones of formula (IIId2) (corresponding to compounds of general formula III wherein R^{3b} together with G² form an =O (oxo) group) may be easily converted to the corresponding amines of formula (IIIe2) (corresponding to compounds of general formula III wherein R^{3b} is a hydrogen atom, R⁵ is -NHCONH₂ or a group of formula: 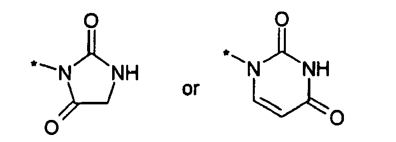 and G²:is a -NH₂ group) by reaction with ammonium acetate or ammonium hydroxide in the presence of a reducing agent. The reaction may be carried out in a variety of solvents such as tetrahydrofuran, alcohols as methanol, ethanol or isopropyl alcohol, as well as a mixture of solvents such as methanol/tetrahydrofuran or ethanol/tetrahydrofuran. The temperature range may be between 5 °C and 100°C but more specifically between 15°C and 90 °C. The reducing agent may be a hydride such as sodium borohydride, sodium cyanoborohydride or sodium triacetoxyborohydride as well as hydrogen plus a hydrogenation catalyst such as Raney® Nickel.

For the preparation of compounds of formula (IIIf) wherein G² is a -NH₂ group, R^{3b} is different from hydrogen and R⁴ and R⁵ are selected from the group consisting of hydrogen, halogen atoms, C₁₋₄ alkyl, C₁₋₄ alkoxy, -SR⁷ the path shown in Scheme 4 may be used. 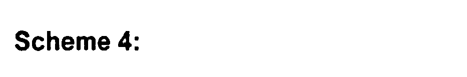 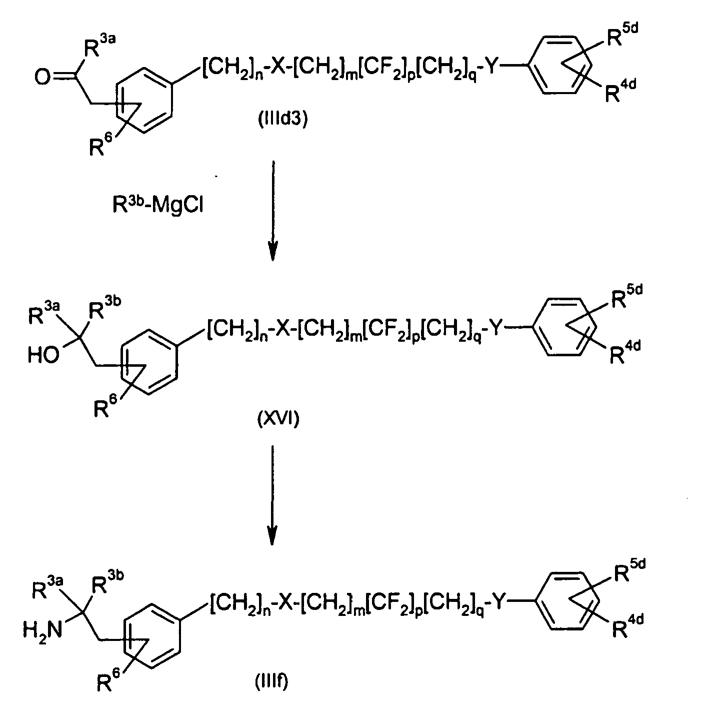 wherein:
R^{3a} and R^{3b} are C₁₋₄ alkyl
R^{4d} and R^{5d} are selected from hydrogen and halogen atoms or groups selected from C₁₋₄ alkyl, C₁₋₄ alkoxy and -SR⁷.
and R₆, R₇, n, m, p, q, X and Y are as defined above

Ketones of formula (IIId3) (corresponding to compounds of general formula III wherein R^{3b} together with G² form an =O (oxo) group) may react with Grignard reagents of formula R^{3b}MgCl in a solvent such as diethyl ether, tetrahydrofuran or dioxane and a temperature from -78 °C to 50 °C to give alcohols of formula (XVI).

Tertiary alcohols of formula (XVI) may be treated with an alkyl nitrile (such as acetonitrile or chloroacetonitrile) in the presence of an acid (such as sulphuric acid or acetic acid) to give an intermediate amide which is in turn cleaved by acidic hydrolysis to give the corresponding amines of formula (IIIf) (corresponding to compounds of general formula III wherein G² is a -NH₂ group). The cleavage of the intermediate amide may be carried out with acids such as acetic acid or hydrochloric acid, optionally in the presence of a solvent (such as water or ethanol) and a temperature from room temperature to the boiling point of the solvent.

The path shown in **Scheme 5** may be used to obtain the compounds of formula (IIIg) (corresponding to the compounds of formula (III) wherein G² is a -NH₂ group and both R^{3a} and R^{3b} are hydrogen atoms, 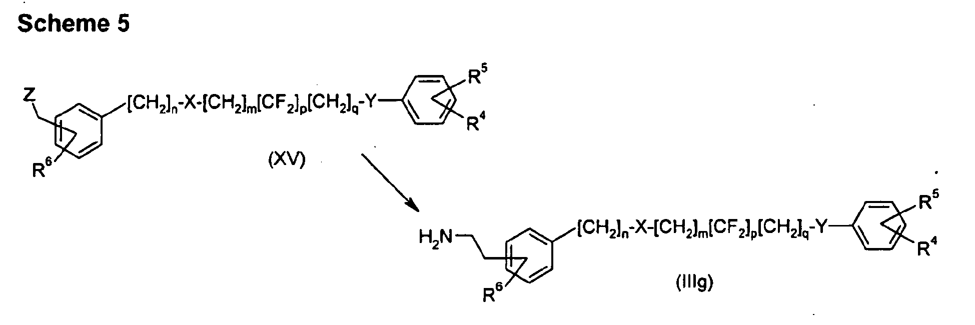 wherein R⁴, R⁵, R⁶, n, m, p, q, X and Y are as defined above and Z is selected from -CN and -CONH₂ groups.

When R⁴ and R⁵ are selected from hydrogen or halogen atoms or groups selected from C₁₋₄ alkyl or C₁₋₄ alkoxy or -SR⁷, the reducing agent may be a hydride such as lithium aluminium hydride, diborane or sodium borohydride in a solvent such as diethyl ether or tetrahydrofuran, in a neutral or acidic media and at a temperature from 0 °C to the boiling point of the solvent. When R⁴ and R⁵ are defined as described above, and Z is a -CN group, the reducing agent could be hydrogen plus a hydrogenation catalyst such as platinum dioxide or Raney® Nickel.

Hydrogenation may be carried out in a solvent such as methanol or ethanol in a neutral, acidic or basic media, at a temperature from 10 °C to 30 °C and at a pressure from 1 to 3 bar.

The compounds of formula (XIII), (XIV) and (XV) used as starting materials in the **Schemes 2 to 5** may be obtained by a variety of methods some of which are described in **Schemes 6 to 13.**

Compounds of general formula (XIIIa) (corresponding to compounds of formula (XIII) wherein R^{4d} and R^{5d} are selected from hydrogen or halogen atoms or groups selected from C₁₋₄ alkyl and C₁₋₄ alkoxy and -SR⁷ groups, R^{9a} is a halogen atom, a CH₂CN group or an alkyl or aryl sulfonate such as methylsulfonate, trifluoromethylsulfonate or p-toluensulfonate) may be prepared as shown in **Scheme 6.** 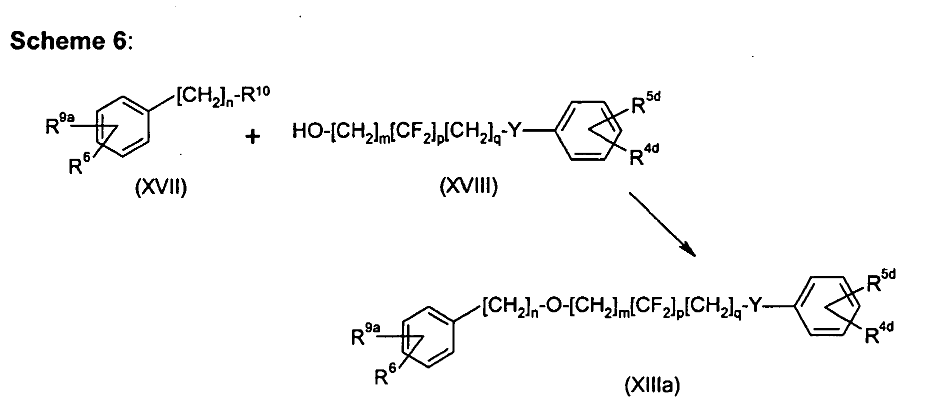 wherein R¹⁰ represents a halogen atom or an alkyl or aryl sulfonate such as methylsulfonate, trifluromethylsulfonate or p-toluensulfonate

Alcohols of formula (XVIII) may react with halides or sulfonates of formula (XVII) to give ethers of formula (XIIIa). The reaction may be carried out with a base such as sodium hydroxide, potassium hydroxide or sodium hydride, optionally in the presence of a base transfer catalyst such as tetrabutylammonium bromide, with a solvent such as water, dimethylformamide, diethylene glycol dimethyl ether or dimethylsulfoxide, and at a temperature from 20 °C to 100 °C.

Compounds of general formula (XIIIb) (corresponding to compounds of formula (XIII) wherein R^{4c} and R^{5c} are selected from hydrogen or halogen atoms or groups selected from C₁₋₄ alkyl, C₁₋₄ alkoxy, SR⁷, SOR⁷ and SO₂R⁷, R^{9a} is a halogen atom, a group CH₂CN or an alkyl or aryl sulfonate such as methylsulfonate, trifluromethylsulfonate or p-toluensulfonate) may be prepared as shown in **Scheme 7.**

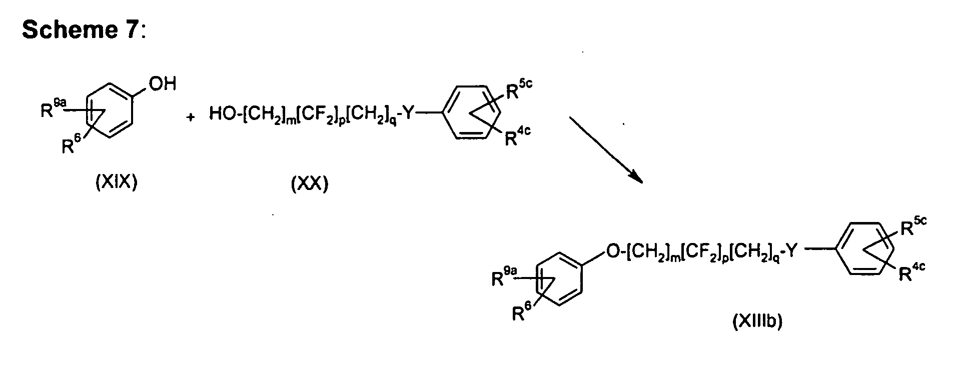

The reaction between the phenols of formula (XIX) and the alcohols of formula (XX) may be carried out with triphenylphosphine and diethyl azodicarboxylate (DEAD) in a solvent such as dichloromethane and tetrahydrofuran at a temperature from room temperature to the boiling point of the solvent.

Compounds of general formula (XIIIc) (corresponding to compounds of formula (XIII) wherein, Y stands for a single bond, R^{4d} and R^{5d} are selected from hydrogen or halogen atoms or groups selected from C₁₋₄ alkyl, C₁₋₄ alkoxy and SR⁷, R^{9a} is a halogen atom or an alkyl or aryl sulfonate such as methylsulfonate, trifluromethylsulfonate or p-toluensulfonate and R^{6a} is selected from hydrogen atom or groups selected from C₁₋₄ alkyl or C₁₋₄ alkoxy) may be prepared as shown in **Scheme 8.** 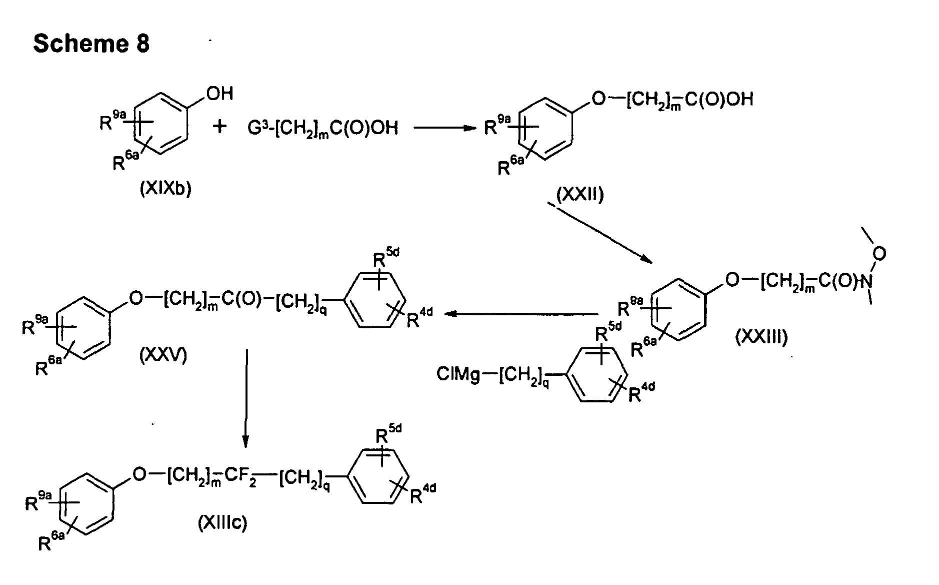 wherein G³ is a chlorine or bromine atom

Alkylation of phenols of formula (XIXb) with acids of formula (XXI) is carried out with a base such as sodium hydroxide, potassium hydroxide, sodium carbonate or potassium carbonate, in a solvent such as water, methanol, ethanol, tetrahydrofuran, acetonitrile or dimethylformamide at a temperature from room temperature to the boiling point of the solvent to give compounds of formula (XXII).

Acids of formula (XXII) may be easily converted to the corresponding Weinreb amides of formula (XXIII) by reaction with N-methyl-N-methoxyamine in the presence of isobutyl or ethyl chloroformate, 1-(3-dimethylaminopropyl)-3-ethylcarbodiimide hydrochloride (EDC) or 1-[bis(dimethylamino)methylene]-1H-benzotriazolium 3-oxide hexafluorophosphate (HBTU) and an amine such as triethylamine, diisopropylethylamine or dimethylaminopyridine, in a solvent such as dichloromethane, tetrahydrofuran or dimethylformamide and at a temperature from room temperature to the boiling point of the solvent.

The Weinreb amides of formula (XXIII) may react with Grignard derivatives of formula (XXIV) in a solvent such as ethyl ether, tetrahydrofuran or dioxane and at a temperature from -78°C to 50°C to give ketones of formula (XXV).

Ketones of formula (XXV) may be converted to fluorinated compounds of formula (XIIIc) by reaction with a fluorinated agent such as (diethylamino) sulfur trifluoride (DAST) or [di(methoxyethyl)amino] sulfur trifluoride (DEOXOFLUOR^{®}), optionally in the presence of a solvent such as methylene chloride, chloroform, methanol, ethanol or tetrahydrofuran, and at a temperature from room temperature to the boiling point of the solvent.

Compounds of general formula (XIIId) (corresponding to compounds of formula (XIII) wherein X is an oxygen atom, n is zero and both m, p and q are one) may be prepared as shown in **Scheme 9.**

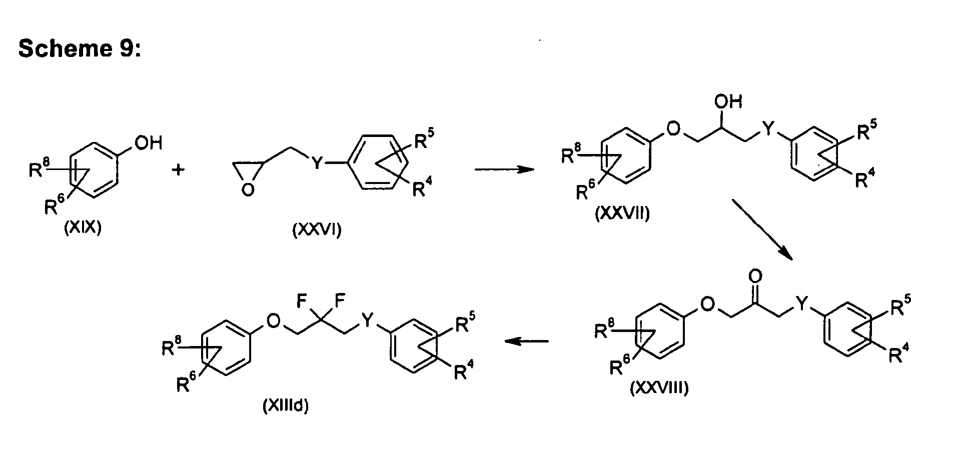

The synthesis of β-hydroxy ethers of formula (XXVII) consists of the ring opening of oxyranes (XXVI) with phenols (XIX) in the presence of amines or under alkaline conditions, such as 1,4-diazabicyclo[2.2.2]octane, cesium fluoride, potassium carbonate or sodium hydroxide, in a solvent such as dimethylformamide, dimethylacetamide, or ethanol, and at a temperature from 80 to 150°C.

β-Hydroxy ethers of formula (XXVII) may be converted to ketones of formula (XXVIII) by reaction with chromium trioxide, manganese dioxide, potassium dichromate, pyridinium chlorochromate, oxalyl chloride in dimethylsulfoxide or Dess-Martin reagent in a solvent such as pyridine, methylene chloride, chloroform, dimethylsulfoxide or acetonitrile, and at a temperature from -78° to 130°C.

Ketones of formula (XXVIII) may be converted to fluorinated compounds of formula (Xllld) by reaction with a fluorinated agent such as (diethylamino) sulfur trifluoride (DAST) or [di(methoxyethyl)amino] sulfur trifluoride (DEOXOFLUOR^{®}), optionally in the presence of a solvent such as methylene chloride, chloroform, methanol, ethanol or tetrahydrofuran, and at a temperature from room temperature to the boiling point of the solvent.

Compounds of general formula (XIIIe) (corresponding to compounds of formula (XIII) wherein X represents an oxygen atom, both n and q are zero, both m and p are one, Y stands for a single bond and R^{4g} and R^{5g} are selected from the group consisting of hydrogen or halogen atoms and groups C₁₋₄alkyl, C₁₋₄ alkoxy, - CONH₂,-SR⁷, -SOR⁷, -SO₂R⁷) may be prepared as shown in **Scheme 10.**

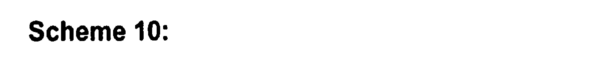

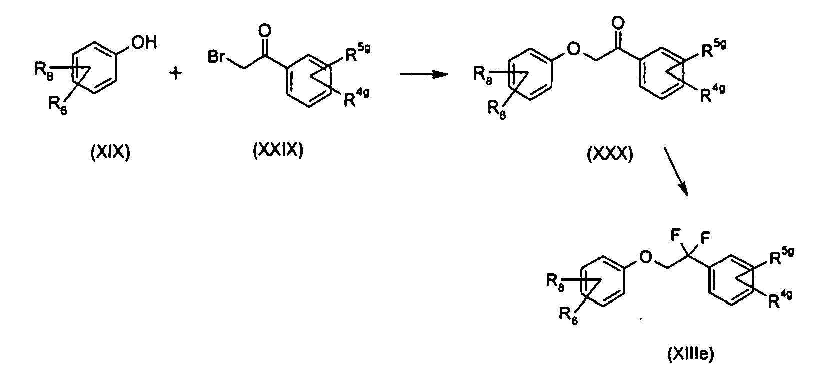

Alkylation of phenols of formula (XIX) with phenacyl halides of formula (XXIX) is carried out with a base such as sodium hydroxide, potassium hydroxide, sodium carbonate or potassium carbonate, in a solvent such as water, methanol, ethanol, tetrahydrofuran, acetonitrile or dimethylformamide at a temperature from room temperature to the boiling point of the solvent to give ketones of formula (XXX).

Ketones of formula (XXX) may be converted to fluorinated compounds of formula (XIIIe) by reaction with a fluorinated agent such as (diethylamino) sulfur trifluoride (DAST) or [di(methoxyethyl)amino] sulfur trifluoride (DEOXOFLUOR^{®}), optionally in the presence of a solvent such as methylene chloride, chloroform, methanol, ethanol or tetrahydrofuran, and at a temperature from room temperature to the boiling point of the solvent.

Compounds of formula (XVa) (corresponding to compounds of formula (XV) wherein R⁵ is -NHCONH₂ and Z is a -CN group) may be prepared as shown is **Scheme 11**

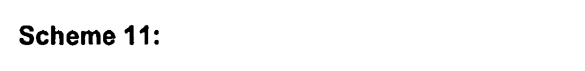

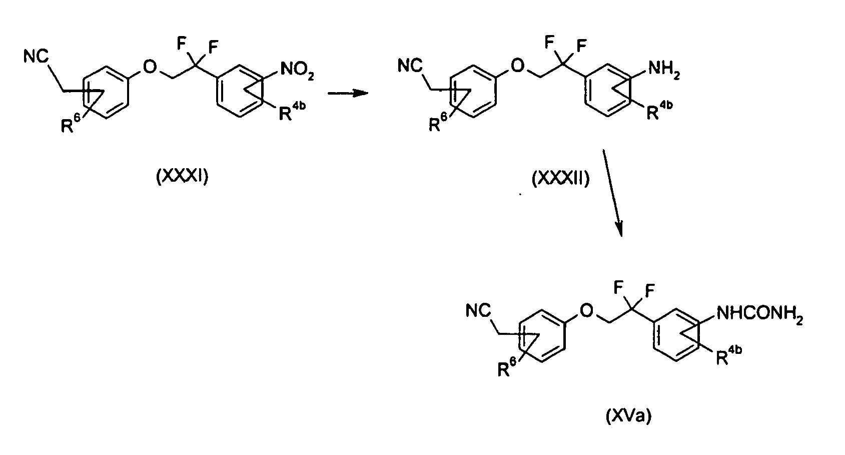

Anilines (XXXII) may be easily prepared by reduction of the corresponding nitro derivatives (XXXI). This step may be achieved in a variety of solvents such as dimethylformamide, ethyl acetate, methanol or ethanol, in a neutral or acidic media and at a temperature from room temperature to the boiling point of the solvent. The reducing agent may be tin dichloride as well as hydrogen plus a hydrogenation catalyst such as Raney® nickel or palladium on charcoal at a pressure from 1 to 3 bar.

Ureas of formula (XVa) may be prepared from anilines (XXXII) by reaction with potassium cyanate in the presence of an acid such as hydrochloric acid or acetic acid. The reaction may be carried out in a solvent such as water and at a temperature from 0 °C to 100 °C.

Compounds of formula (XIVa) wherein R^{3a} is a C₁₋₄ alkyl, R^{4a} is selected from the group consisting of hydrogen or fluorine atom or groups selected from C₁₋₄ alkyl, C₁₋₄ alkoxy, -CONH₂, SR⁷, SOR⁷, SO₂R⁷ or SO₂NHR⁸, R^{5f} is NHCONH₂, and R^{6a} is selected from hydrogen or groups selected from C₁₋₄ alkyl or C₁₋₄ alkoxy may be prepared as shown is **Scheme 12** 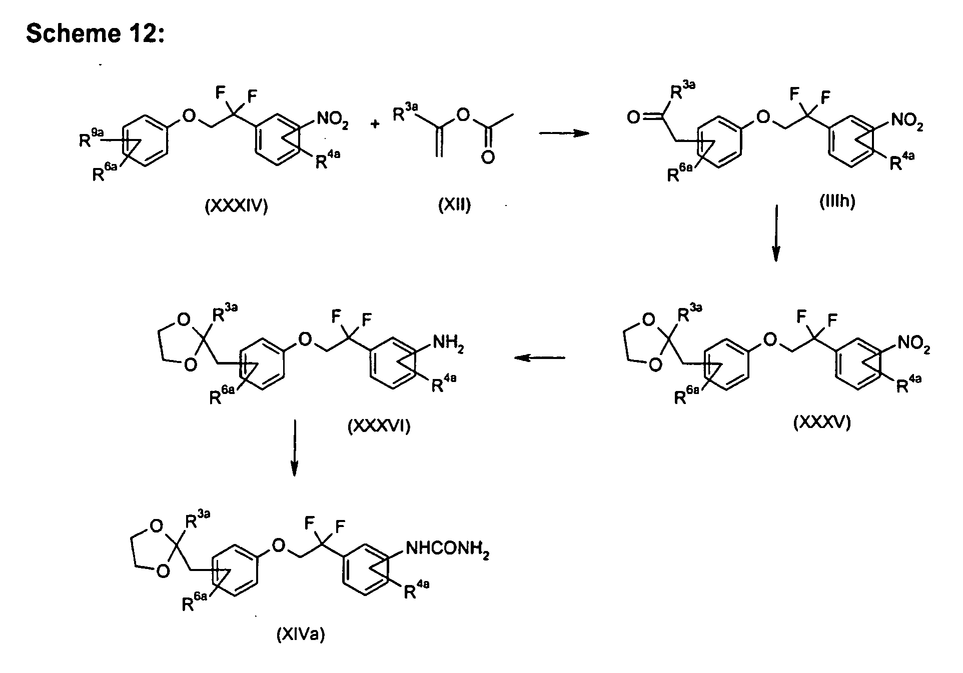 wherein R^{9a} is a halogen atom or an alkyl or aryl sulfonate such as methylsulfonate, trifluromethylsulfonate or p-toluensulfonate

Ketones of formula (IIIh) may be prepared by a palladium-mediated coupling of an aryl halide or sulfonate of formula (XXXIV). In a standard procedure, the compound of formula (XXXIV) is reacted with a tin enolate generated in-situ by treatment of a substituted vinylacetate of formula (XII) with tri-n-butyltin methoxide in the presence of a suitable palladium catalyst such as palladium acetate and a phospine such as tri-ortho-tolylphosphine in a non-polar solvent such as toluene. Preferably, the reaction is carried out at a temperature comprised between 80 °C and 110 °C.

Ketones of formula (IIIh) may be easily converted to the acetals of formula (XXXV) by reaction with ethylene glycol under acid catalysis. This step may be carried out in a solvent such as benzene, toluene or dichloromethane, at a temperature from room temperature to the boiling point of the solvent and with p-toluensulfonic acid as catalyst. When the reaction is carried out in benzene or toluene as solvents, a Dean-Stark system may be used in order to eliminate the water formed in the reaction and force the reaction to completion.

Anilines (XXXVI) may be easily prepared by reduction of the corresponding nitro derivatives of formula (XXXV). This step may be achieved in a variety of solvents such as dimethylformamide, ethyl acetate, methanol or ethanol, in a neutral or acidic media and at a temperature from room temperature to the boiling point of the solvent. The reducing agent may be tin dichloride as well as hydrogen plus a hydrogenation catalyst such as Raney® nickel or palladium on charcoal at a pressure from 1 to 3 bar.

Ureas of formula (XIVa) may be prepared from anilines of formula (XXXVI) by reaction with potassium cyanate in the presence of an acid such as acetic acid. The reaction may be carried out in a solvent such as water and at a temperature from 0 °C to 100 °C.

Compounds of formula (Xlllh) wherein R^{4g} and R^{5g} are independently selected from the group consisting of hydrogen or halogen atom or groups selected from C₁₋₄alkyl, C₁₋₄alkoxy, -CONH₂, SR⁷, SOR⁷ and SO₂R⁷ may be prepared as shown is **Scheme 13**

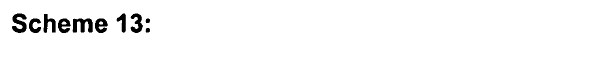

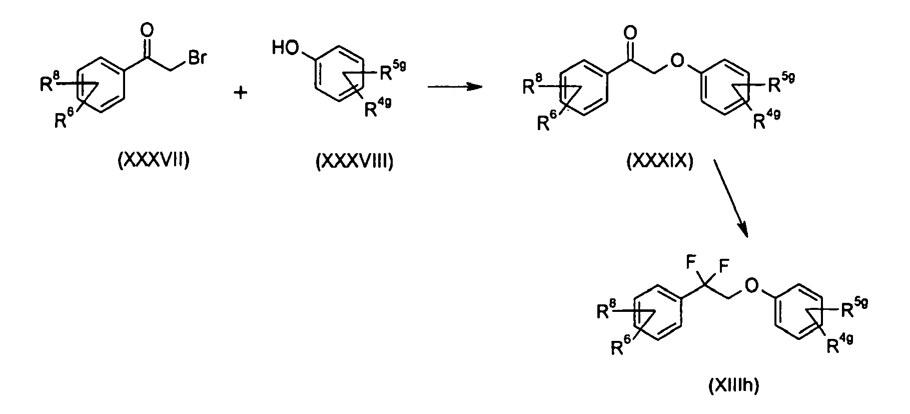

Alkylation of phenols of formula (XXXVIII) with phenacyl halides of formula (XXXVII) is carried out with a base such as sodium hydroxide, potassium hydroxide, sodium carbonate or potassium carbonate, in a solvent such as water, methanol, ethanol, tetrahydrofuran, acetonitrile or dimethylformamide at a temperature from room temperature to the boiling point of the solvent to give ketones of formula (XXXIX).

Ketones of formula (XXXIX) may be converted to fluorinated compounds of formula (XIIIh) by reaction with a fluorinated agent such as (diethylamino) sulfur trifluoride (DAST) or [di(methoxyethyl)amino] sulfur trifluoride (DEOXOFLUOR^{®}), optionally in the presence of a solvent such as methylene chloride, chloroform, methanol, ethanol or tetrahydrofuran, and at a temperature from room temperature to the boiling point of the solvent.

Compounds of formula (XVb) (corresponding to compounds of formula (XV) wherein n and m are zero, X is direct bond, p and q are 1 and Y is oxygen, R^{4e} and R^{5e} are selected from hydrogen or halogen atoms or groups selected from C₁₋₄ alkoxy, -CONH₂, SR⁷, SOR⁷ and SO₂R⁷ and R^{6b} is selected from hydrogen or halogen atoms or groups selected from C₁₋₄ alkoxy) may be prepared as shown in **Scheme 14:**

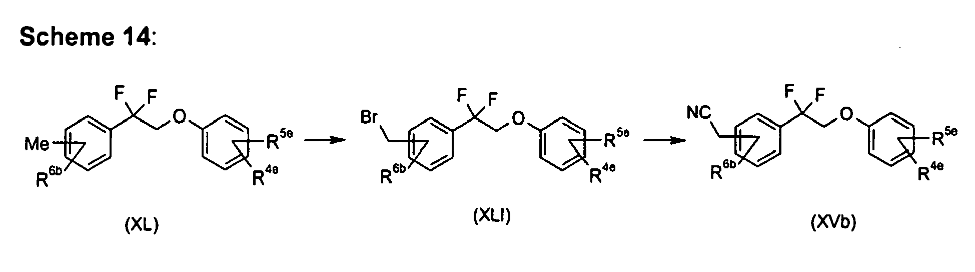

Compounds of formula (XL) may be converted to the corresponding benzylic bromides of formula (XLI) by reaction with N-bromosuccinimide in the presence of a radical initiator such as 2-2'-azobis(isoburyronitrile) (AIBN) or benzoyl peroxide. The reaction may be carried out in a variety of solvents such as carbon tetrachloride, chloroform, methylene chloride or ethyl acetate at a temperature from room temperature to the boiling point of the solvent.

Benzylic bromides of formula (XLI) may react with sodium cyanide or potassium cyanide to give the benzylic nitriles of formula (XVb). The reaction may be carried out in a variety of solvents such as acetonitrile, dimethylsulfoxide or ethanol as well as in a mixture of solvents such as dioxane/water or ethanol/water and at a temperature from room temperature to the boiling point of the solvent.

Alcohols of general formula (XX) (corresponding to compounds of formula XVIII wherein R^{4g} and R^{5g} are selected from hydrogen or halogen atoms or groups selected from C₁₋₄ alkyl, C₁₋₄ alkoxy, CONH₂, SR⁷, SOR⁷ and SO₂R⁷) and alcohols of general formula (XVIIIa) (corresponding to compounds of formula XVIII wherein R^{4d} and R^{5d} are selected from hydrogen or halogen atoms or groups selected from C₁₋₄ alkyl, C₁₋₄ alkoxy and SR⁷) may be prepared by a variety of methods some of which are described in **Schemes 15-17:**

The alcohols of formula (XXa) (corresponding to the compounds of formula (XX) wherein Y is a direct bond and both m and p are 1) may be prepared following **Scheme 15.** 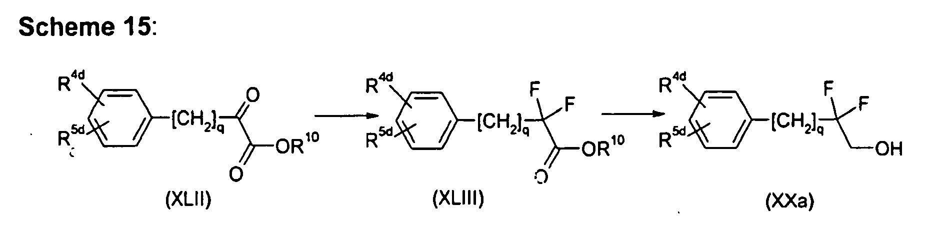 wherein R¹⁰ is a C₁₋₄alkyl group

Compounds of formula (XLII) may be transformed to compounds of formula (XLIII) by reaction with a fluorinated agent such as (diethylamino) sulfur trifluoride (DAST) or [di(methoxyethyl)amino] sulfur trifluoride (DEOXOFLUOR^{®}), optionally in the presence of a solvent such as methylene chloride, chloroform, methanol, ethanol or tetrahydrofuran, and at a temperature from room temperature to the boiling point of the solvent.

Alcohols of formula (XXa) may be obtained by treatment of esters of formula (XLIII) with a hydride such as lithium aluminum hydride, sodium borohydride or diisobutylaluminum hydride in a solvent such as ethyl ether, diisopropyl ether, tetrahydrofuran or methanol, and at a temperature from 0°C to the boiling point of the solvent.

The alcohols of formula (XXb) (corresponding to the compounds of formula (XX) wherein Y is a direct bond, q is zero, p is 1 and m is 2 or 3) may be prepared following **Scheme 16:** 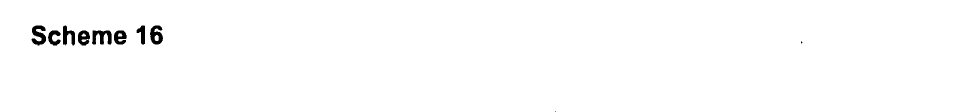 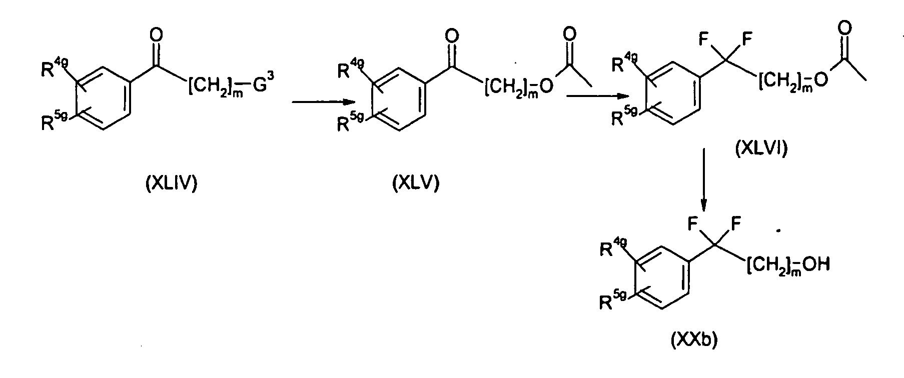 wherein G³ stands for a chlorine or bromine atom

Compounds of formula (XLIV) may react with sodium or potassium acetate, sodium or potassium iodide, in a solvent such as glacial acetic acid and at a temperature from room temperature to the boiling point of the solvent to give compounds of formula (XLV).

Esters of formula (XLV) may be converted to fluorinated compounds of formula (XLVI) by reaction with a fluorinated agent such as (diethylamino) sulfur trifluoride (DAST) or [di(methoxyethyl)amino] sulfur trifluoride (DEOXOFLUOR^{®)}, optionally in the presence of a solvent such as methylene chloride, chloroform, methanol, ethanol or tetrahydrofuran, and at a temperature from room temperature to the boiling point of the solvent.

Fluorinated alcohols of formula (XXb) may be prepared from fluorinated esters of formula (XLVI). The reaction may be carried out with an aqueous solution of sodium hydroxide, potassium hydroxide or sodium carbonate, optionally in the presence of a solvent such as ethanol, methanol or isopropyl alcohol, and at a temperature from room temperature to the boiling point of the solvent.

The alcohols of formula (XXc) (corresponding to the compounds of formula (XX) wherein Y is a direct bond, q is zero, p is 1 and m is 3) may be prepared following **Scheme 17:** 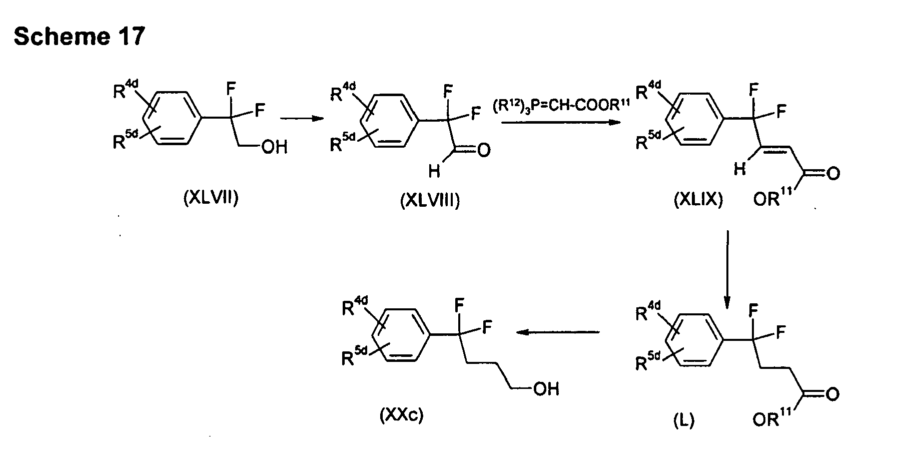 wherein R¹¹ stands for C₁₋₄ alkyl and R¹² stands for C₁₋₄ alkyl or phenyl group.

Alcohols of formula (XLVII) may be converted to aldehydes of formula (XLVIII) by reaction with chromium trioxide, manganese dioxide, potassium dichromate, pyridinium chlorochromate, oxalyl chloride in dimethylsulfoxide or Dess-Martin reagent in a solvent such as pyridine, methylene chloride, chloroform, dimethylsulfoxide or acetonitrile, and at a temperature from -78° to 130°C.

Aldehydes of formula (XLVIII) may react with a phosphorane of formula (R¹²)₃P=CH-COOR¹¹ to give esters of formula (XLIX). The reaction may be carried out in a solvent such as methylene chloride, tetrahydrofuran, ethyl ether or toluene, and at a temperature from room temperature to the boiling point of the solvent.

Hydrogenation of compounds of formula (XLIX) gives esters of formula (L). The reaction may be carried out with a catalyst such as palladium on charcoal or platinum dioxide, in a solvent such as ethanol, methanol, ethyl acetate or dimethylformamide, at a temperature from room temperature to 70°C, and at a pressure from 1 to 3 bar.

Alcohols of formula (XXc) may be obtained by treatment of esters of formula (L) with a hydride such as lithium aluminum hydride, sodium borohydride or diisobutylaluminum hydride in a solvent such as ethyl ether, diisopropyl ether, tetrahydrofuran or methanol, and at a temperature from room temperature to the boiling point of the solvent.

### EXAMPLES

**General.** Reagents, starting materials, and solvents were purchased from commercial suppliers and used as received. Concentration refers to evaporation under vacuum using a Büchi rotatory evaporator. Reaction products were purified, when necessary, by flash chromatography on silica gel (40-63 µm) with the solvent system indicated. Spectroscopic data were recorded on a Varian Gemini 300 spectrometer and a Varian Inova 400 spectrometer. Melting points were recorded on a Büchi 535 apparatus. HPLC-MS were performed on a Gilson instrument equipped with a Gilson piston pump 321, a Gilson 864 vacuum degasser, a Gilson liquid handler 215, a Gilson 189 injection module, a Gilson Valvemate 7000, a 1/1000 splitter, a Gilson 307 make-up pump, a Gilson 170 diode array detector, and a Thermoquest Finnigan aQa detector. Semi-preparative purifications were carried out using a SunFire C18 reverse phase column (100 A, 5 µm, 19 x 100 mm, purchased from WATERS).

### Intermediate 1. 2-(4-Bromophenoxy)-1-phenylethanone

To a solution of 4-bromophenol (4.56 g, 26.38 mmol) and phenacyl bromide (5.00 g, 25.12 mmol) in acetonitrile (250 mL) was added potassium carbonate (3.86 g, 27.63 mmol). The resulting mixture was stirred at reflux overnight before the solvent was removed under reduced pressure. The residue was partitioned between ethyl acetate (100 mL) and water (100 mL). The organic layer was separated and washed with 2N sodium hydroxide (2 x 100 mL), water (2 x 100 mL) and brine (100 mL), dried (Na₂SO₄) and the solvent removed under reduced pressure. The residue was triturated with n-hexane and the precipitate was collected by filtration to obtain the title compound as a pale yellow solid (6.32 g, 86%).

### Intermediate 2. 1-Bromo-4-(2,2-difluoro-2-phenylethoxy)benzene

To a suspension of Intermediate 1 (5.65 g, 19.40 mmol) in methylene chloride (20 mL) was added DAST (7.8 mL, 59.1 mmol). The mixture was stirred at room temperature overnight. The crude reaction was diluted with methylene chloride (50 mL) and poured into a stirred mixture of water (100 mL) and ice (100 g). The organic layer was separated, washed with water (2 x 100 mL), saturated solution of sodium bicarbonate (2 x 100 mL) and brine (100 mL), and dried (Na₂SO₄). The solvent was removed under reduced pressure and the residue was purified by column chromatography with silica gel, eluting with n-hexane/ethyl acetate (from 10:1 to 1:1). The title compound was obtained as a yellow oil (4.86 g, 80%).

### Intermediate 3. 1-[4-(2,2-Difluoro-2-phenylethoxy)phenyl]acetone

A solution of Intermediate 2 (3.86 g, 12.33 mmol), isopropenyl acetate (2.04 mL, 18.49 mmol), tri-n-butyltin methoxide (4.26 mL, 14.79 mmol), palladium (II) acetate (0.14 g, 0.62 mmol) and tri-o-tolylphosphine (0.38 g, 1.23 mmol) in toluene (200 mL) was degassed and then heated at 100 °C under argon overnight. The reaction mixture was cooled to room temperature, diluted with ethyl acetate (20 mL) and a solution of potassium fluoride (9.1 g, 156.74 mmol) in water (40 mL) was added. The resulting mixture was stirred at room temperature for 2 hours and filtered through a pad of Celite®, washing the precipitate with ethyl acetate (100 mL). The organic phase of the filtrate was separated, washed with water (50 mL) and brine (50 mL), dried (Na₂SO₄) and the solvents removed under reduced pressure. The residue was purified by column chromatography with silica gel, eluting with n-hexane/ethyl acetate (from 10:1 to 7:3) to give the title compound as a yellow oil (1.37 g, 38%).

### Intermediate 4. 8-(Benzyloxy)-5-[(1R,S)-2-({(1R,S)-2-[4-(2,2-difluoro-2-phenylethoxy) phenyl]-1-methylethyl}amino)-1-hydroxyethyl]quinolin-2(1H)-one

A solution of Intermediate 3 (0.32 g, 1.1 mmol) and (*R*,*S*)-5-(2-amino-1-hydroxyethyl)-8-(benzyloxy)quinolin-2(1H)-one (0.34 g, 1.1 mmol) in a mixture of tetrahydrofuran (4 mL) and ethanol (4 mL) was heated at reflux for 5 hours. The reaction mixture was cooled to room temperature and diluted with tetrahydrofuran (2 mL) and ethanol (2 mL). Sodium borohydride (0.13 g, 3.3 mmol) was added at 0 °C and the resulting mixture was stirred at room temperature overnight. The solvents were removed under reduced pressure and the crude was partitioned between methylene chloride (50 mL) and saturated solution of sodium bicarbonate (50 mL). The organic layer was separated, washed with saturated solution of sodium bicarbonate (2 x 25 mL) and brine (50 mL), dried (MgSO₄) and the solvent removed under reduced pressure. The residue was purified by column chromatography with silica gel, eluting with methylene chloride/methanol (from 98:2 to 8:2) to give the title compound as a yellow oil (0.25 g, 40%).

### EXAMPLE 1. 5-[(1R,S)-2-({(1R,S)-2-[4-(2,2-difluoro-2-phenylethoxy)phenyl]-1-methyl ethyl}amino)-1-hydroxyethyl]-8-hydroxyquinolin-2(1H)-one

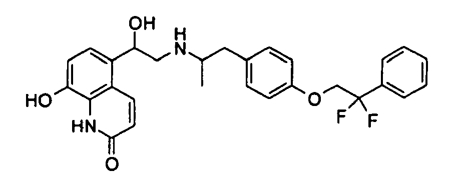

To a solution of Intermediate 4 (0.30 g, 0.51 mmol) in methanol (14 mL) were added 7 drops of a saturated hydrochloric acid solution in ethanol and palladium on charcoal (10%, 32 mg). The mixture was hydrogenated at 30 psi overnight. The catalyst was filtered through Celite® and the solvent removed under reduced pressure. The crude was dissolved in an 80:20:2 mixture of methylene chloride/methanol/ammonium hydroxide (20 mL) and the solvents were removed under reduced pressure. The crude oil obtained was purified by column chromatography eluting with methylene chloride/methanol/ammonium hydroxide (from 90:10:1 to 80:20:2) to give the title compound (0.19 g, 76%) as a foam.
¹H-NMR (300 MHz, dimethylsulfoxide-D⁶): 0.86 (d, *J*=6.0 Hz, 6H); 2.30-2.50 (m, 2H); 2.50-2.80 (m, 8H); 4.54 (t, *J*_{F-H} =13.5 Hz, 2H (one diastereoisomer)); 4.56 (t, *J*_{F-H} =13.5 Hz, 2H (other diastereoisomer)); 4.90-5.00 (m, 2H); 6.50 (d, *J*=9.0 Hz, 2H); 6.80-6.93 (m, 6H); 6.95-7.08 (m, 6H); 7.45-7.55 (m, 6H), 7.60-7.68 (m, 4H); 8.14 (d, *J*=9.0 Hz, 1H (one diastereoisomer)); 8.15 (d, *J*=9.0 Hz, 1H (other diastereoisomer)). MS (M+): 495.

### Intermediate 5. (1R,S)-2-({(1R,S)-2-[4-(2,2-Difluoro-2-phenylethoxy)phenyl]-1-methyl ethyl}amino)-1-(2,2-dimethyl-4H-1,3-benzodioxin-6-yl)ethanol

Obtained from Intermediate 3 (0.40 g, 1.38 mmol) and (*R*,*S*)-2-amino-1-(2,2-dimethyl-4*H*-1,3-benzodioxin-6-yl)ethanol (0.31 g, 1.38 mmol) by the procedure described in Intermediate 4. Purification by column chromatography with silica gel and methylene chloride/methanol/ammonium hydroxide (97:3:0.3) as eluent yielded the title compound (0.34 g, 49%) as an oil.

### EXAMPLE 2. 4-[(1R,S)-2-({(1R,S)-2-[4-(2,2-Difluoro-2-phenylethoxy)phenyl]-1-methyl ethyl}amino)-1-hydroxyethyl]-2-(hydroxymethyl)phenol acetate

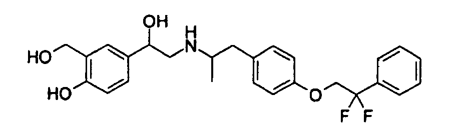

A solution of Intermediate 5 (0.33 g, 0.66 mmol) in a mixture of acetic acid (1.3 mL) and water (0.7 mL) was heated at 80°C for 30 minutes. The solvent was removed under reduced pressure to give the title compound (0.31 g, 91 %) as a solid.
¹H-NMR (300 MHz, dimethylsulfoxide-D⁶): 0.87 (d, *J*=6.0 Hz, 6H); 2.33-2.56 (m, 4H); 2.48 (s, 6H); 2.58-2.70 (m, 4H); 2.73-2.83 (m, 2H); 4.42-4.47 (m, 2H); 4.44 (s, 4H); 4.54 (t, *J*_{F-H} =13.5 Hz, 2H (one diastereoisomer)); 4.55 (t, *J*_{F-H} =13.5 Hz, 2H (other diastereoisomer)); 6.67 (d, *J*=9.0 Hz, 2H); 6.82-6.89 (m, 4H); 6.93-6.98 (m, 2H); 7.01-7.09 (m, 4H); 7.23 (s, 2H); 7.48-7.55 (m, 6H); 7.59-7.65 (m, 4H). MS (M+): 458.

### Intermediate 6. (2R,S)-1-[4-(2,2-Difluoro-2-phenylethoxy)phenyl]propan-2-amine

To a solution of Intermediate 3 (0.2 g, 0.69 mmol) in methanol (6.7 mL) were added ammonium acetate (0.53 g, 6.9 mmol) and sodium cyanoborohydride (0.17 g, 2.76 mmol) and the resulting mixture was heated at reflux under argon for 90 minutes. The solvent was removed under reduced pressure and the crude was successively treated at 0 °C with water (3 mL), 2N hydrochloric acid solution (3 mL) and 5N hydrochloric acid solution (4 mL). The resulting solution was stirred at room temperature for 30 minutes and washed with methylene chloride (20 mL). The aqueous phase was then basified until pH = 8-9 with solid potassium carbonate and extracted with ethyl acetate (3 x 50 mL). The combined organic layers were washed with water (20 mL) and brine (20 mL), dried (MgSO₄) and the solvent removed under reduced pressure. The residue was purified by column chromatography with silica gel, eluting with methylene chloride/methanol/ammonium hydroxide (80:20:2) to give the title compound as an oil (0.14 g, 70%).

### Intermediate 7. {2-(Benzyloxy)-5-[(1R,S)-1-{[tert-butyl(dimethyl)silyl]oxy}-2-({(1R,S)-2-[4-(2,2-difluoro-2-phenylethoxy)phenyl]-1-methylethyl}amino)ethyl]phenyl} formamide

To a solution of (*R*,*S*)-[2-(benzyloxy)-5-(2-bromo-1-{[tert-butyl(dimethyl)silyl]oxy}-ethyl) phenyl]formamide (0.30 g, 0.65 mmol) and Intermediate 6 (0.19 g, 0.65 mmol) in dimethylsulfoxide (0.8 mL) were added potassium carbonate (0.36 g, 2.59 mmol) and sodium iodide (0.1 g, 0.75 mmol). The mixture was heated at 125 °C for 1 hour. After cooling, the reaction was diluted with water (15 mL) and extracted with ethyl acetate (2 x 30 mL). The combined organic extracts were washed with water (2 x 20 mL) and brine (20 mL), dried (Na₂SO₄), and the solvent removed under reduced pressure. The residue was purified by column chromatography with silica gel, eluting with methylene chloride/methanol (95:5) to give the title compound (0.31 g, 70%).

### Intermediate 8. {2-(Benzyloxy)-5-[(1R,S)-2-({(1R,S)-2-[4-(2,2-difluoro-2-phenylethoxy) phenyl]-1-methylethyl}amino)-1-hydroxyethyl]phenyl}formamide

To a solution of Intermediate 7 (0.18 g, 0.27 mmol) in tetrahydrofuran (1.6 mL) was added tetra-n-butyl ammonium fluoride trihydrate (0.14 g, 0.43 mmol). The mixture was stirred at 45 °C for 3 hours. The solvent was removed under reduced pressure and the residue was partitioned between water (20 mL) and methylene chloride (20 mL). The organic layer was separated, washed with water (2 x 20 mL), dried (MgSO₄) and the solvent removed under reduced pressure. The residue was triturated with n-hexane and the precipitate was collected by filtration to obtain the title compound as a pale yellow solid (0.12 g, 81%).

### EXAMPLE 3. Formic acid - {5-[(1R,S)-2-({(1R,S)-2-[4-(2,2-Difluoro-2-phenylethoxy) phenyl]-1-methylethyl}amino)-1-hydroxyethyl]-2-hydroxyphenyl} formamide (1:1)

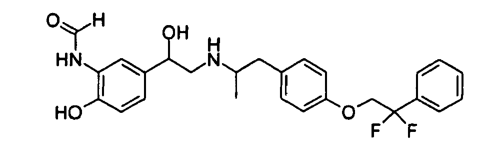

To a solution of Intermediate 8 (0.27 g, 0.483 mmol) in ethanol (17 mL) were added 5 drops of a saturated solution of hydrochloric acid in ethanol and palladium on charcoal (10%, 41 mg). The mixture was hydrogenated at 2.76 bar overnight. The catalyst was filtered through Cetite® and the solvent removed under reduced pressure. The resulting oil was purified by semi-preparative HPLC eluting with water/acetonitrile/ammonium formiate (from 100/0/0.1 to 50/50/0.1) to give the title compound (0.09 g, 37%) as a yellow solid.
¹H-NMR (400 MHz, CD₃OD): 1.19 (d, *J*=6.0 Hz, 3H (one stereoisomer)); 1.20 (d, *J*=6.0 Hz, 3H (other stereoisomer)); 2.72-2.61 (m, 2H); 3.22-3.05 (m, 6H); 3.47-3.35 (m, 2H); 4.44 (t, *J*_{F-H}=12 Hz, 4H); 4.90-4.76 (m, 2H); 6.91-6.84 (m, 6H); 7.11-7.00 (m, 2H), 7.16-7.13 (m, 4H); 7.49-7.44 (m, 6H); 7.61-7.57 (m, 4H); 8.13 (bs, 2H); 8.29 (bs, 2H); 8.52 (bs, 2H). MS (M+): 471.

### Intermediate 9. 2-(3-Bromophenoxy)-1-phenylethanone

Obtained from 3-bromophenol (5.0 g, 28.9 mmol), phenacyl bromide (5.48 g, 27.52 mmol) and potassium carbonate (4.39 g, 31.79 mmol) by the procedure described for the Intermediate 1. The title compound was obtained (7.62 g, 95%) as a yellow solid.

### Intermediate 10. 1-Bromo-3-(2,2-difluoro-2-phenylethoxy)benzene

Obtained from Intermediate 9 (6.53 g, 22.42 mmol) and DAST (8.81 mL, 67.26 mmol) by the procedure described for the Intermediate 2. Purification by column chromatography with silica gel and n-hexane/ethyl acetate (from 10:1 to 4:1) as eluent gave the title compound (5.62 g, 80%) as a yellow oil.

### Intermediate 11. 1-[3-(2,2-Difluoro-2-phenylethoxy)phenyl]acetone

Obtained from Intermediate 10 (0.80 g, 2.56 mmol), isopropenyl acetate (0.42 mL, 3.84 mmol), tri-n-butyltin methoxide (0.88 mL, 3.07 mmol), palladium (II) acetate (0.03 g, 0.13 mmol) and tri-o-tolylphosphine (0.08 g, 0.26 mmol) by the procedure described for the Intermediate 3. Purification by column chromatography with silica gel and n-hexane/ethyl acetate (10:1) as eluent gave the title compound (0.47 g, 63%) as a yellow oil.

### Intermediate 12. (2R,S)-1-[3-(2,2-Difluoro-2-phenylethoxy)phenyl]propan-2-amine

Obtained from Intermediate 11 (1.00 g, 3.44 mmol), ammonium acetate (2.65 g, 34.4 mmol), sodium cyanoborohydride (0.87 g, 13.8 mmol) and two drops of acetic acid by the procedure described for the Intermediate 6. Purification by column chromatography with silica gel and methylene chloride/methanol/ammonium hydroxide (80:20:2) as eluent yielded the title compound (0.73 g, 73%) as a yellow oil.

### Intermediate 13. 8-(Benzyloxy)-5-((1R)-1-{[tert-butyl(dimethyl)silyl]oxy}-2-({(1R,S)-2-[3-(2,2-difluoro-2-phenylethoxy)phenyl]-1-methylethyl}amino)-ethyl]quinolin-2(1H)-one

To a solution of 8-(benzyloxy)-5-((1*R*)-2-bromo-1-{[tert-butyl(dimethyl)silyl]oxy} ethyl)quinolin-2(1*H*)-one (0.84 g, 1.72 mmol) and Intermediate 12 (0.5 g, 1.72 mmol) in dimethylsulfoxide (2.3 mL) was added potassium carbonate (0.94 g, 6.82 mmol) and sodium iodide (0.28 g, 1.89 mmol). The mixture was heated at 125 °C for 90 minutes. After cooling, the reaction was diluted with water (45 mL) and extracted with ethyl acetate (2 x 30 mL). The combined organic extracts were washed with water (2 x 20 mL) and brine (20 mL), dried (MgSO₄), and the solvent removed under reduced pressure. The residue was purified by column chromatography with silica gel, eluting with methylene chloride/methanol (97:3) to give the title compound as an oil (0.58 g, 48%).

### Intermediate 14. 8-(Benzyloxy)-5-((1R)-2-({(1R,S)-2-[3-(2,2-difluoro-2-phenylethoxy) phenyl]-1-methylethyl}amino)-1-hydroxyethyl]quinolin-2(1H)-one

Obtained from Intermediate 13 (0.57 g, 0.82 mmol) and tetra-n-butyl ammonium fluoride trihydrate (0.41 g, 1.31 mmol) by the procedure described for the Intermediate 8. Purification by column chromatography with silica gel and methylene chloride/methanol (from 95:5 to 90:10) as eluent yielded the title compound (0.35 g, 72%).

### Example 4. 5-((1R)-2-({(1R,S)-2-[3-(2,2-difluoro-2-phenylethoxy)phenyl]-1-methylethyl}amino)-1-hydroxyethyl]-8-hydroxyquinolin-2(1H)-one

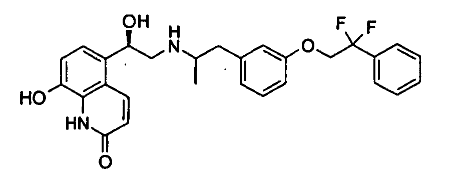

Obtained from Intermediate 14 (0.35 g, 0.59 mmol) and palladium on charcoal (10%, 0.05 g) by the procedure described in Example 1. Purification by column chromatography with silica gel and methylene chloride/methanol/ammonium hydroxide (90:10:1) as eluent gave the title compound (0.23 g, 78%) as a solid.
¹H-NMR (300 MHz, CD₃OD): 1.06 (d, *J*=6.0 Hz, 3H (one diastereoisomer)); 1.08 (d, *J*=6.0 Hz, 3H (other diastereoisomer)); 2.56-2.69 (m, 4H); 2.71-2.82 (m, 2H); 2.87-3.03 (m, 4H); 4.41 (t, *J*_{F-H} =13.5 Hz, 2H (one diastereoisomer)); 4.44 (t, *J*_{F-H} =1 3.5 Hz, 2H (other diastereoisomer)); 5.06-5.15 (m, 2H); 6.57-6.81 (m, 8H); 6.86-6.94 (m, 2H); 7.03-7.18 (m, 4H), 7.42-7.48 (m, 6H); 7.53-7.62 (m, 4H); 8.28 (d, *J*=9.0 Hz, 2H). MS (M+): 495.

### Intermediate 15. (1R)-2-({(1R,S)-2-[3-(2,2-difluoro-2-phenylethoxy)phenyl]-1-methylethyl}amino)-1-(2,2-dimethyl-4H-1,3-benzodioxin-6-yl)ethanol

Obtained from Intermediate 11 (0.37 g, 1.26 mmol), (*R*)-2-amino-1-(2,2-dimethyl-4*H*-1,3-benzodioxin-6-yl)ethanol (0.28 g, 1.26 mmol) and 2 drops of acetic acid by the procedure described in Intermediate 4. Purification by column chromatography with silica gel and methylene chloride/methanol/ammonium hydroxide (97:3:0.3) as eluent yielded the title compound (0.34 g, 54%) as an oil.

### Example 5. 4-[(1 R)-2-({(1R,S)-2-[3-(2,2-difluoro-2-phenylethoxy)phenyl]-1-methylethyl}amino)-1-hydroxyethyl]-2-(hydroxymethyl)phenol

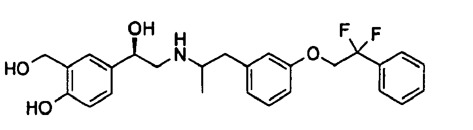

Obtained from Intermediate 15 (0.34 g, 0.67 mmol), acetic acid (1.33 mL) and water (0.66 mL) by the procedure described in Example 2. Purification by column chromatography with silica gel and methylene chloride/methanol/ammonium hydroxide (80:20:2) as eluent yielded the title compound (0.22 g, 72%) as a solid.
¹H-NMR (300 MHz, CD₃OD): 1.04 (d, *J*=6.0 Hz, 6H); 2.53-2.70 (m, 6H); 2.76-2.91 (m, 4H); 4.42 (t, *J*_{F-H}=12.0 Hz, 2H (one diastereoisomer)); 4.44 (t, *J*_{F-H} =12.0 Hz, 2H (other diastereoisomer)); 4.56-4.60 (m, 2H); 4.60 (s, 2H (one diastereoisomer)); 4.61 (s, 2H (other diastereoisomer)); 6.64-6.81 (m, 8H); 6.97-7.03 (m, 2H); 7.09-7.23 (m, 4H), 7.45-7.52 (m, 6H); 7.56-7.63 (m, 4H). MS (M+): 458.

### Intermediate 16. 1-Bromo-3-[(2,2-difluoro-2-phenylethoxy)methyl]benzene

To a solution of 2,2-difluoro-2-phenylethanol (0.30 g, 1.90 mmol) in dimethylformamide (2.2 mL) was added at 0 °C 60% sodium hydride (0.08 g, 2.09 mmol) and 1-bromo-3-(bromomethyl)benzene (0.52 g, 2.09 mmol). The mixture was stirred at room temperature for 2 hours. The crude was diluted with methylene chloride (50 mL) and washed with water (3 x 50 mL), dried (MgSO₄) and concentrated. The crude was purified by column chromatography with silica gel using n-hexane/ethyl acetate (10:1) as eluent. The title compound was obtained (0.50 g, 81%) as solid.

### Intermediate 17. 1-{3-[(2,2-Difluoro-2-phenylethoxy)methyl]phenyl}acetone

Obtained from Intermediate 16 (0.50 g, 1.54 mmol), isopropenyl acetate (0.25 mL, 2.31 mmol), tri-n-butyltin methoxide (0.53 mL, 1.84 mmol), palladium (II) acetate (0.02 g, 0.08 mmol) and tri-o-tolylphosphine (0.05 g, 0.15 mmol) by the procedure described for the Intermediate 3. Purification by column chromatography with silica gel and n-hexane/ethyl acetate (9:1) as eluent gave the title compound (0.28 g, 60%).

### Intermediate 18. (1R)-2[((1R,S)-2-{3-[(2,2-Difluoro-2-phenylethoxy)methyl]-phenyl}-1-methylethyl)amino]-1-(2,2-dimethyl-4H-1,3-benzodioxin-6-yl)ethanol

Obtained from Intermediate 17 (0.28 g, 0.92 mmol), (*R*)-2-amino-1-(2,2-dimethyl-4*H-*1,3-benzodioxin-6-yl)ethanol (0.21 g, 0.92 mmol) and 2 drops of acetic acid by the procedure described in Intermediate 4. Purification by column chromatography with silica gel and methylene chloride/methanol/ammonium hydroxide (97:3:0.3) as eluent yielded the title compound (0.15 g, 31%) as a yellow solid.

### Example 6. 4-{(1R)-2-[((1R,S)-2-{3-[(2,2-Difluoro-2-phenylethoxy)methyl]-phenyl}-1-methylethyl)amino]-1-hydroxyethyl}-2-(hydroxymethyl)phenol acetate

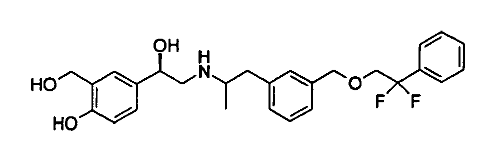

Obtained from Intermediate 18 (0.15 g, 0.29 mmol), acetic acid (0.6 mL) and water (0.3 mL) by the procedure described in Example 2. The title compound was obtained (0.15 g, 95%) as a yellow solid.
¹H-NMR (300 MHz, CD₃OD) 1.16 (d, *J*=6.0 Hz, 3H (one diastereoisomer)); 1.17 (d, *J*=6.0 Hz, 3H (other diastereoisomer)); 1.91 (s, 6H); 2.63-2.73 (m, 2H); 3.00-3.15 (m, 6H); 3.30-3.40 (m, 2H); 3.90 (t, *J*_{F-H} =13.5 Hz, 2H (one diastereoisomer)); 3.91 (t, *J*_{F-H} =13.5 Hz, 2H (other diastereoisomer)); 4.54 (s, 4H); 4.64 (s, 4H); 4.75-4.85 (m, 2H); 6.72-6.78 (m, 2H); 7.09-7.16 (m, 8H); 7.23-7.36 (m, 4H); 7.40-7.45 (m, 6H); 7.48-7.55 (m, 4H).
MS (M+): 472.

### Intermediate 19. [4-(2-Oxo-2-phenylethoxy)phenyl]acetonitrile

Obtained from (4-hydroxyphenyl)acetonitrile (12.8 g, 64.3 mmol), phenacyl bromide (9.00 g, 67.59 mmol) and potassium carbonate (9.8 g, 71.01 mmol) by the procedure described for the Intermediate 1. The title compound was obtained (15.50 g, 96%) as a yellow solid.

### Intermediate 20. [4-(2,2-Difluoro-2-phenylethoxy)phenyl]acetonitrile

Obtained from Intermediate 19 (15.50 g, 56.71 mmol) and DAST (40.5 mL, 309 mmol) by the procedure described for the Intermediate 2. Purification by column chromatography with silica gel and methylene chloride/n-hexane (from 5:1 to 9:1) as eluent gave the title compound (13.16 g, 78%) as a yellow oil.

### Intermediate 21. 2-[4-(2,2-Difluoro-2-phenylethoxy)phenyl]ethanamine

To a solution of Intermediate 20 (11.10 g, 40.54 mmol) in ethanol (29 mL) were added a solution of sodium hydroxide (3.73 g, 93.25 mmol) in ethanol (110 mL) and Raney Nickel® (10 g of a 50% slurry in water). The mixture was hydrogenated at 2.76 bar for 3 hours. The catalyst was filtered through Celite® and the solvent removed under reduced pressure. The crude was partitioned between water (200 mL) and ethyl acetate (350 mL). The aqueous layer was separated and washed with ethyl acetate (2 x 150 mL). The combined organic extracts were washed with brine (20 mL), dried (MgSO₄) and the solvent removed under reduced pressure. The residue was purified by column chromatography with silica gel, eluting with methylene chloride/methanol/ammonium hydroxide (from 100:0:0 to 90:10:1) to give the title compound (11.01 g, 98%) as an orange oil.

### Intermediate 22. 8-(benzyloxy)-5-[(1R,S)-2-({2-[4-(2,2-difluoro-2-phenylethoxy)phenyl]ethyl}amino)-1-hydroxyethyl]quinolin-2(1H)-one

A solution of Intermediate 21 (0.57 g, 2.07 mmol) and 8-(benzyloxy)-5-(dihydroxyacetyl)quinolin-2(1*H*)-one (0.57 g, 1.76 mmol) in dimethylsulfoxide (5.9 mL) was stirred at room temperature for 3.5 hours. After this reaction time, methanol (5.9 mL) and sodium borohydride (0.2 g, 5.31 mmol) were successively added and the reaction mixture was stirred at room temperature overnight. The reaction crude was partitioned between ethyl acetate (90 mL) and a saturated solution of sodium bicarbonate (60 mL). The organic layer was separated, washed with water (2 x 30 mL), dried (MgSO₄) and the solvents removed under reduced pressure. The residue was purified by column chromatography with silica gel, eluting with methylene chloride/methanol (from 75:1 to 9:1) to give the title compound as an orange oil (0.85 g, 84%).

### Example 7. 5-[2-({2-[(1R,S)-4-(2,2-difluoro-2-phenylethoxy)phenyl]ethyl}-amino)-1-hydroxyethyl]-8-hydroxyquinolin-2(1H)-one

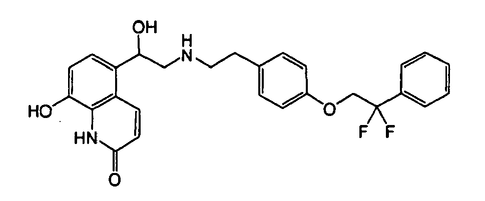

Obtained from Intermediate 22 (0.85 g, 1.49 mmol) and palladium on charcoal (0.085 g, 10%) by the same procedure described in Example 1 (reaction time: 48 hours). The crude obtained was purified by column chromatography with silica gel, eluting with mehylene chloride/methanol (from 75:1 to 10:1) to obtain the title compound as a yellow solid (0.48 g, 66%).
¹H-NMR (300 MHz, dimethylsulfoxide-D⁶): 2.76-2.80 (m, 2H); 2.89-2.96 (m, 4H); 4.58 (t, *J*_{F-H} =13.46 Hz, 2H); 5.22 (bs, 1H); 6.54 (d, *J*=9.89 Hz, 1H); 6.91-6.97 (m, 3H); 7.10-7.15 (m, 3H); 7.52-7.56 (m, 3H); 7.63-7.66 (m, 2H); 8.19 (d, *J*=9.89 Hz, 1H).
MS (M+): 481.

### Intermediate 23. 8-(Benzyloxy)-5-[(1R)-1-{[tert-butyl(dimethyl)silyl]oxy}-2-({2-[4-(2,2-difluoro-2-phenylethoxy)phenyl]ethyl}amino)ethyl]quinolin-2(1H)-one

Obtained from 8-(benzyloxy)-5-((1*R*)-2-bromo-1-{[*tert*-butyl(dimethyl)silyl]oxy}ethyl) quinolin-2(1*H*)-one (2.01 g, 4.11 mmol), Intermediate 21 (1.72 g, 6.21 mmol), potassium carbonate (1.72 g, 12.47 mmol) and potassium iodide (0.76 g, 4.58 mmol) by the procedure described for the Intermediate 13. Purification by column chromatography with silica gel and ethyl acetate/methanol (from 100:0 to 15:1) as eluent gave the title compound (1.36 g, 48%) as an oil.

### Intermediate 24. 8-(Benzyloxy)-5-[(1R)-2-({2-[4-(2,2-difluoro-2-phenylethoxy)-phenyl] ethyl}amino)-1-hydroxyethyl]quinolin-2(1H)-one

Obtained from Intermediate 23 (1.70 g, 2.48 mmol) and tetra-n-butyl ammonium fluoride trihydrate (1.25 g, 3.96 mmol) by the procedure described for the Intermediate 8. Purification by column chromatography with silica gel and methylene chloride/methanol/ammonium hydroxide (from 100:0:0 to 90:10:1) as eluent yielded the title compound (1.40 g, 99%) as an oil.

### Example 8. 5-[(1R)-2-({2-[4-(2,2-Difluoro-2-phenylethoxy)phenyl]ethyl}amino)-1-hydroxyethyl]-8-hydroxyquinolin-2(1H)-one

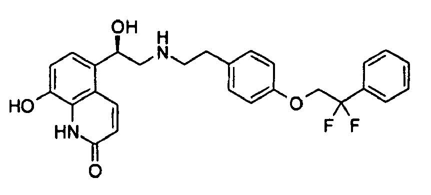

Obtained from Intermediate 24 (1.40 g, 2.45 mmol) and palladium on charcoal (10%, 0.14 g) by the procedure described in Example 1. Purification by column chromatography with silica gel and methylene chloride/methanol/ammonium hydroxide (80:20:2) as eluent gave the title compound (1.05 g, 89%) as a yellow solid.
¹H-NMR (300 MHz, dimethylsulfoxide-D6): 2.69-2.77 (m, 2H); 2.80-2.93 (m, 4H); 4.56 (t, *J*_{F-H} =13.5 Hz, 2H); 5.16 (t, *J*=6.0 Hz, 1H); 6.52 (d, *J*=9.0 Hz, 1H); 6.88-6.97 (m, 3H); 7.07-7.12 (m, 3H); 7.50-7.55 (m, 3H); 7.61-7.64 (m, 2H); 8.17 (d, *J*=9.0 Hz, 1H).
MS (M+): 481.

### Intermediate 25. (1R,S)-1-[4-(Benzyloxy)-3-(hydroxymethyl)phenyl]-2-({2-[4-(2,2-difluoro-2-phenylethoxy)phenyl]ethyl}amino)ethanol

Obtained from Intermediate 21 (0.33 g, 1.19 mmol) and [4-(benzyloxy)-3-(hydroxymethyl)phenyl](oxo)acetaldehyde (0.32 g, 1.19 mmol) by the procedure described in Intermediate 4. Purification by column chromatography with silica gel and methylene chloride/methanol/ammonium hydroxide (90:10:1) as eluent yielded the title compound (0.27 g, 43%) as an oil.

### Example 9. 4-[(1R,S)-2-({2-[4-(2,2-Difluoro-2-phenylethoxy)phenyl] ethyl}amino)-1-hydroxyethyl]-2-(hydroxymethyl)phenol

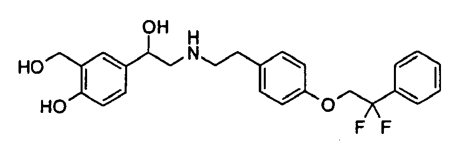

To a solution of Intermediate 25 (0.27 g, 0.51 mmol) in methanol (10 mL) was added palladium on charcoal (10%, 27 mg). The mixture was hydrogenated at 20 psi for 6 hours. The catalyst was filtered through Celite® and the solvent removed under reduced pressure. The resulting oil was purified by column chromatography eluting with methylene chloride/methanol/ammonium hydroxide (from 100:0:0 to 80:20:2) to give the title compound (0.14 g, 63%).
¹H-NMR (400 MHz, dimethylsulfoxide-D⁶): 2.54-2.63 (m, 4H); 2.65-2.74 (m, 2H); 4.44 (s, 2H); 4.44-4.49 (m, 1H); 4.55 (t, *J*_{F-H} =14.0 Hz, 2H); 4.90-4.95 (bs, 1H); 5.02-5.07 (bs, 1H); 6.66 (d, *J*=8.0 Hz, 1H); 6.87 (d, *J*=8.0 Hz, 2H); 6.95 (d, *J*=8.0 Hz, 1H); 7.09 (d, *J*=8.0 Hz, 2H); 7.23 (s, 1H); 7.49-7.55 (m, 3H); 7.61-7.65 (m, 2H); 9.12-9.23 (bs, 1H).
MS (M+): 444.

### Intermediate 26. {2-(Benzyloxy)-5-[(1R,S)-1-{[tert-butyl(dimethyl)silyl]oxy}-2-({2-[4-(2,2-difluoro-2-phenylethoxy)phenyl]ethyl}amino)ethyl]phenyl} formamide

Obtained from (*R*,*S*)-[2-(benzyloxy)-5-(2-bromo-1-{[*tert*-butyl(dimethyl)silyl]oxy} ethyl)phenyl]formamide (0.56 g, 1.21 mmol), Intermediate 21 (0.35 g, 1.27 mmol), potassium carbonate (0.66 g, 3.63 mmol) and sodium iodide (0.20 g, 1.33 mmol) by the procedure described in Intermediate 7. Purification by column chromatography with silica gel and methylene chloride/methanol (95:5) as eluent yielded the title compound (0.37 g, 46%).

### Intermediate 27. {2-(Benzyloxy)-5-[(1R,S)-2-({2-[4-(2,2-difluoro-2-phenylethoxy) phenyl]ethyl}amino)-1-hydroxyethyl]phenyl}formamide

Obtained from Intermediate 26 (0.37 g, 0.55 mmol) and tetra-n-butyl ammonium fluoride trihydrate (0.28 g, 0.89 mmol) by the procedure described for the Intermediate 8. The residue was triturated with n-hexane and the precipitate was collected by filtration to yield the title compound as a pale yellow solid (0.27 g, 88%).

### Example 10. {5-[(1R,S)-2-({2-[4-(2,2-Difluoro-2-phenylethoxy)phenyl]-1-methylethyl} amino)-1-hydroxyethyl]-2-hydroxyphenyl}formamide formate

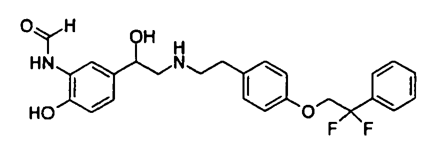

To a solution of Intermediate 27 (0.27 g, 0.49 mmol) in ethanol (17 mL) was added palladium on charcoal (10%, 41 mg). The mixture was hydrogenated at 2.76 bar overnight. The catalyst was filtered through Celite® and the solvent removed under reduced pressure. The resulting oil was purified by semi-preparative HPLC eluting with water/acetonitrile/ammonium formiate (from 100/0/0.1 to 50/50/0.1) to give the title compound (83 mg, 34%) as a yellow solid.
¹H-NMR (400 MHz, dimethylsulfoxide-D⁶): 2.67-2.75 (m, 4H); 2.81-2.90 (m, 2H); 4.55-4.60 (m, 1H); 4.56 (t, *J*_{F-H}=12.0 Hz, 2H); 6.79-6.91 (m, 4H); 7.11 (d, *J*=8.0 Hz, 2H); 7.50-7.54 (m, 3H); 7.61-7.64 (m, 2H); 8.05 (s, 1H); 8.25 (s, 1H); 8.30 (s, 1H); 9.58 (s, 1H).
MS (M+): 457.

### Intermediate 28. (3-Hydroxyphenyl)acetonitrile

To a solution of (3-methoxyphenyl)acetonitrile (1.89 mL, 13.59 mmol) in methylene chloride (100 mL) was added dropwise at 0 °C a 1 M solution of boron tribromide in methylene chloride (65.22 mL, 65.22 mmol) under nitrogen. The resulting mixture was stirred at room temperature for 3 hours, quenched by slow addition of ethanol (100 mL) at 0 °C and poured into an excess of saturated sodium bicarbonate solution. The organic layer was separated and washed with ethyl acetate (3 x 100 mL). The combined organic layers were washed with water (2 x 100 mL), dried (MgSO₄) and the solvents removed under reduced pressure. The title compound was obtained (1.78 g, 98%) as a brown oil and was used in the next step without further purification.

### Intermediate 29. [3-(2-Oxo-2-phenylethoxy)phenyl]acetonitrile

Obtained from Intermediate 28 (2.0 g, 15.02 mmol), phenacyl bromide (2.85 g, 14.30 mmol) and potassium carbonate (2.28 g, 16.52 mmol) by the procedure described for the Intermediate 1. Purification by column chromatography with silica gel and methylene chloride/n-hexane (from 1:1 to 100:0) as eluent yielded the title compound (1.70 g, 45%) as a pale orange solid.

### Intermediate 29. [3-(2,2-Difluoro-2-phenylethoxy)phenyl]acetonitrile

Obtained from Intermediate 28 (1.70 g, 6.76 mmol) and DAST (4.43 mL, 33.8 mmol) by the procedure described for the Intermediate 2. Purification by column chromatography with silica gel and n-hexane/ethyl acetate (from 10:1 to 10:4) as eluent yielded the title compound (1.55 g, 84%) as a brown oil.

### Intermediate 30. [3-(2,2-Difluoro-2-phenylethoxy)phenyl]acetonitrile

Obtained from Intermediate 29 (1.70 g, 6.76 mmol) and DAST (4.43 mL, 33.8 mmol) by the procedure described for the Intermediate 2. Purification by column chromatography with silica gel and n-hexane/ethyl acetate (from 10:1 1 to 10:4) as eluent yielded the title compound (1.55 g, 84%) as a brown oil.

### Intermediate 31. 2-[3-(2,2-Difluoro-2-phenylethoxy)phenyl]ethanamine

To a solution of Intermediate 30 (1.55 g, 5.67 mmol) in methanol (39 mL) were added a solution of concentrated hydrochloric acid (37%, 1.12 mL) and platinum (IV) oxide (0.13 g, 0.57 mmol) and the resulting mixture was hydrogenated overnight. The catalyst was filtered through Celite® and the solvent removed under reduced pressure. The crude was partitioned between saturated solution of potassium carbonate (100 mL) and ethyl acetate (100 mL). The aqueous layer was separated and washed with ethyl acetate (2 x 50 mL). The combined organic extracts were washed with brine (50 mL), dried (MgSO₄) and the solvent removed under reduced pressure. The title compound was obtained (0.90 g, 57%) and was used in the next step without further purification.

### Intermediate 32. 5-Acetyl-8-[(4-methoxybenzyl)oxy]quinolin-2(1H)-one

To a solution of 5-acetyl-8-hydroxyquinolin-2(1*H*)-one (14.40 g, 71 mmol) in dimethylformamide (360 mL) were added sodium bicarbonate (9.9 g, 117.85 mmol) and sodium iodide (0.1 g, 0.67 mmol). The resulting suspension was then heated at 40 °C and a solution of 1-(chloromethyl)-4-methoxybenzene (10.7 mL, 79.25 mmol) in dimethylformamide (47 mL) was slowly added during 4 hours. The reaction mixture was stirred at 40 °C overnight. After this reaction time, sodium bicarbonate (3.30 g, 39 mmol) was added to the reaction mixture followed by the slow addition at 40 °C of a solution of 1-(chloromethyl)-4-methoxybenzene (5.35 mL, 39.63 mmol) in dimethylformamide (23.5 mL) in a period of 4 hours. The stirring was continued overnight at the same temperature before the solvent was removed under reduced pressure. The residue was successively triturated with water and ethyl acetate. The resulting solid was dissolved in methylene chloride, washed with water, dried (MgSO₄) and the solvent removed under reduced pressure. The residue was triturated with diethyl ether and the precipitate was collected by filtration to obtain the title compound as a pale yellow solid (18.5 g, 81%).

### Intermediate 33. 5-(Dihydroxyacetyl)-8-[(4-methoxybenzyl)oxy]quinolin-2(1H)-one

To a solution of Intermediate 32 (2.5 g, 7.73 mmol) in dioxane (39 mL) and water (1.7 mL) was added selenium dioxide (1.28 g, 11.60 mmol). The resulting mixture was stirred at reflux overnight before being filtered through a pad of Celite®. This first filtrate was discarded. Celite® was then washed several times with an excess of boiling dioxane. The filtrates were combined and the solvent removed under reduced pressure to yield the title compound (2.01 g, 73%) as a yellow solid.

### Intermediate 34. 5-[(1R,S)-2-({2-[3-(2,2-Difluoro-2-phenylethoxy)phenyl]ethyl}amino)-1-hydroxyethyl]-8-[(4-methoxybenzyl)oxy]quinolin-2(1H)-one

A solution of Intermediate 31 (0.18 g, 0.65 mmol) and Intermediate 33 (0.30 g, 0.84 mmol) in dimethylsulfoxide (3 mL) was stirred at room temperature for 3 hours. After this reaction time, methanol (3 mL) and sodium borohydride (0.10 g, 2.60 mmol) were successively added and the reaction mixture was stirred at room temperature for 2 hours before being partitioned between ethyl acetate (25 mL) and a saturated solution of sodium bicarbonate (25 mL). The organic layer was separated, washed with water (2 x 20 mL), dried (MgSO₄) and the solvents removed under reduced pressure. The residue was purified by column chromatography with silica gel, eluting with methylene chloride/methanol/aqueous ammonia (from 75:1:0 to 80:20:2) to give the title compound as an oil (0.21 g, 54%).

### Example 11. 5-[(1R,S)-2-({2-[3-(2,2-Difluoro-2-phenylethoxy)phenyl]ethyl}amino)-1-hydroxyethyl]-8-hydroxyquinolin-2(1H)-one

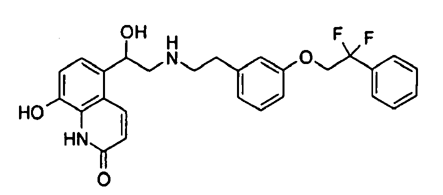

To a solution of Intermediate 34 (0.15 g, 0.25 mmol) in methylene chloride (1.5 mL) was added trifluoroacetic acid (0.19 mL, 2.5 mmol). The resulting mixture was stirred at room temperature for 3 hours before the solvent was removed under reduced pressure. The crude was dissolved in an 80:20:2 mixture of methylene chloride/methanol/aqueous ammonia (10 mL) and the solvents were removed under reduced pressure. The crude oil obtained was purified by column chromatography eluting with methylene chloride/methanol/aqueous ammonia (from 90:10:1 to 80:20:2) to give the title compound (0.08 g, 67%) as a yellow solid.
¹H-NMR (400 MHz, dimethylsulfoxide-D6): 2.65-2.73 (m, 2H); 2.76-2.79 (m, 4H); 2.82-2.90 (m, 2H); 4.57 (t, *J*_{F-H} =14.0 Hz, 2H); 5.05-5.08 (m, 1H); 6.50 (d, *J*=8.0 Hz, 1H); 6.80-6.83 (m, 3H); 6.91 (d, *J*=8.0 Hz, 1H); 7.06 (d, *J*=8.0 Hz, 1H); 7.18 (t, *J*=8.0 Hz, 1H); 7.50-7.54 (m, 3H); 7.61-7.64 (m, 2H); 8.14 (d, *J*=8.0 Hz, 1H).
MS (M+): 481.

### Intermediate 35. 8-(benzy)oxy)-5-[(1R)-1-{[tert-butyl(dimethyl)silyl]oxy}-2-({2-[3-(2,2-difluoro-2-phenylethoxy)phenyl]ethyl}amino)ethyl]quinolin-2(1H)-one

To a solution of 8-(benzyloxy)-5-((1*R*)-2-bromo-1-{[*tert*-butyl(dimethyl)silyl]oxy} ethyl)quinolin-2(1*H*)-one (0.64 g, 1.31 mmol) and Intermediate 31 (0.4 g, 1.36 mmol) in dimethylsulfoxide (2 mL) was added sodium hydrogen carbonate (0.13 g, 1.57 mmol) and sodium iodide (0.18 g, 0.12 mmol). The mixture was heated at 140°C for 1 hour. After cooling, the reaction was diluted with water (26 mL) and extracted with ethyl acetate (2 x 30 mL). The combined organic extracts were washed with water (2x10 mL) and brine (10 mL), dried (MgSO₄), and the solvent removed under reduced pressure. The residue was purified by column chromatography with silica gel, eluting with hexane/ethyl acetate (from 4:1 to 1:8) to give the title compound as a yellow oil (0.65 g, 70%).

### Intermediate 36. 8-(benzyloxy)-5-[(1R)-2-({2-[3-(2,2-difluoro-2-phenylethoxy) phenyl]ethyl}amino)-1-hydroxyethyl]quinolin-2(1H)-one

Obtained from a solution of Intermediate 35 (0.65 g, 0.93 mmol) in tetrahydrofuran and tetrabutylammonium fluoride trihydrate (0.47 g, 1.49 mmol) by the procedure described for the Intermediate 8. Purification by column chromatography with chloroform/methanol (from 75:1 to 10: 1) as eluent yielded the title compound (0.35 g, 67%) as an oil.

### Example 12. 5-[(1R)-2-({2-[3-(2,2-difluoro-2-phenylethoxy)phenyl]ethyl} amino)-1-hydroxyethyl]-8-hydroxyquinolin-2(1H)-one

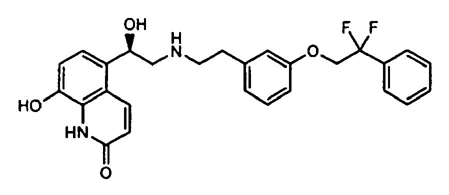

To a solution of Intermediate 36 (0.350 g, 0.61 mmol) in methanol (12 mL) was added palladium on charcoal (10%, 35 mg). The mixture was hydrogenated at 2.76 bar for 20 hours. The catalyst was filtered through Celite® and the solvent removed under reduced pressure. The crude oil obtained was purified by column chromatography with silica gel, eluting with methylene chloride/methanol/aqueous ammonia (from 90:5:0.5 to 80:20:2) to give the title compound (0.21 g, 70%) as a foam.
¹H-NMR (300 MHz, dimethylsulfoxide-D6): 2.80-2.85 (m, 2H); 2.90 (d, *J*=6.31 Hz, 2H); 2.97 (bs, 2H); 4.63 (t, *J*_{F-H} =13.4 Hz, 2H); 5.21 (t, *J*=5.50 Hz, 1H); 6.56 (d, *J*=9.89 Hz, 1H); 6.80-6.88 (m, 3H); 6.99 (d, *J*=7.97 Hz, 1H); 7.14 (d, *J*=7.97 Hz, 1H); 7.25 (t, *J*=7.41 Hz, 3H); 7.50-7.58 (m, 3H); 7.65-7.70 (m, 2H); 8.22 (d, *J*=9.89 Hz, 1H).
MS (M+): 481.

### Intermediate 37. (1R,S)-1-[4-(Benzyloxy)-3-(hydroxymethyl)phenyl]-2-({2-[3-(2,2-difluoro-2-phenylethoxy)phenyl]ethyl}amino)ethanol

Obtained from Intermediate 31 (0.30 g, 1.08 mmol) and [4-(benzyloxy)-3-(hydroxymethyl)phenyl](oxo)acetaldehyde (0.30 g, 1.11 mmol) by the procedure described in Intermediate 4. Purification by column chromatography with silica gel and methylene chloride/methanol/aqueous ammonia (from 15:1:0 to 90:10:1) as eluent yielded the title compound (0.26 g, 45%) as an oil.

### Example 13. 4-[(1R,S)-2-({2-[3-(2,2-Difluoro-2-phenylethoxy)phenyl]ethyl}amino)-1-hydroxyethyl]-2-(hydroxymethyl)phenol

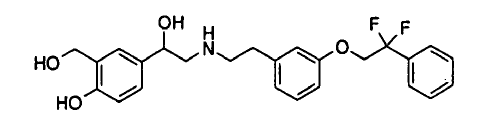

Obtained from Intermediate 37 (0.26 g, 0.49 mmol) and palladium on charcoal (10%, 0.03 g) by the procedure described in Example 8. Purification by column chromatography with silica gel and methylene chloride/methanol/aqueous ammonia (from 90:10:1 to 80:20:2) as eluent yielded the title compound (0.042 g, 22%) as a solid.
¹H-NMR (400 MHz, dimethylsulfoxide-D6): 2.83-2.99 (m, 4H); 3.05-3.12 (m, 2H); 4.46 (s, 2H); 4.59 (t, *J*_{F-H} = 14.0 Hz, 2H); 4.75-4.80 (m, 1H); 4.99-5.03 (m, 1H); 5.80-5.90 (bs, 1H); 6.74 (d, *J*=8.0 Hz, 1H); 6.82-6.89 (m, 3H); 7.02 (d, *J*=8.0 Hz, 1H); 7.20-7.25 (m, 1H); 7.30 (s, 1H); 7.49-7.56 (m, 3H); 7.61-7.65 (m, 2H); 9.38-9.46 (bs, 1H).
MS (M+): 444.

### Intermediate 38. {2-(Benzyloxy)-5-[(1R,S)-1-{[tert-butyl(dimethyl)silyl]oxy}-2-({2-[3-(2,2-difluoro-2-phenylethoxy)phenyl]ethyl}amino)ethyl]phenyl}formamide

Obtained from (*R*,*S*)-[2-(benzyloxy)-5-(2-bromo-1-{[*tert*-butyl(dimethyl)silyl]oxy}ethyl) phenyl]formamide (0.45 g, 0.97 mmol), Intermediate 31 (0.30 g, 1.08 mmol), potassium carbonate (0.45 g, 3.25 mmol) and sodium iodide (0.18 g, 1.19 mmol) by the procedure described in Intermediate 7. Purification by column chromatography with silica gel and methylene chloride/methanol (10:1) as eluent yielded the title compound (0.43 g, 61%) as a yellow oil.

### Intermediate 39. {2-(Benzyloxy)-5-[(1R,S)-2-({2-[3-(2,2-difluoro-2-phenylethoxy) phenyl]ethyl}amino)-1-hydroxyethyl]phenyl}formamide

Obtained from Intermediate 38 (0.43 g, 0.65 mmol) and tetra-n-butyl ammonium fluoride trihydrate (0.33 g, 1.04 mmol) by the procedure described for the Intermediate 8. The title compound was obtained (0.26 g, 75%) as an oil and was used in the next step without further purification.

### Example 14. {5-[(1R,S)-2-({2-[3-(2,2-Difluoro-2-phenylethoxy)phenyl]ethyl}amino)-1-hydroxyethyl]-2-hydroxyphenyl}formamide

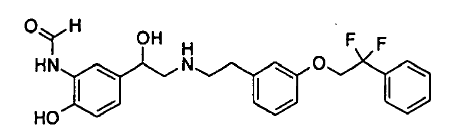

Obtained from Intermediate 39 (0.26 g, 0.48 mmol) and palladium on charcoal (10%, 0.03 g) by the procedure described in Example 10. Purification by column chromatography with silica gel and ethyl acetate/methanol (from 100:0 to 100:20) as eluent yielded the title compound (0.09 g, 41%) as a solid.
¹H-NMR (400 MHz, dimethylsulfoxide-D6): 2.70-2.76 (m, 4H); 2.85-2.93 (m, 2H); 4.55-4.58 (m, 1H); 4.58 (t, *J*_{F-H}= 14.0 Hz, 2H); 5.40-5.60 (bs, 1H); 6.78-6.89 (m, 6H); 7.19 (t, *J*=8.0 Hz, 1H); 7.50-7.55 (m, 3H); 7.61-7.65 (m, 2H); 8.06 (s, 1H); 8.25 (s, 1H); 9.56 (s, 1H).
MS (M+): 457.

### Intermediate 40. {4-[2-(2-Methoxyphenyl)-2-oxoethoxy]phenyl}acetonitrile

Obtained from (4-hydroxyphenyl)acetonitrile (3.05 g, 22.92 mmol), 2-bromo-1-(2-methoxyphenyl)ethanone (5.00 g, 21.87 mmol) and potassium carbonate (3.32 g, 24.00 mmol) by the procedure described for the Intermediate 1. The title compound was obtained (6.12 g, 99%) as an orange solid.

### Intermediate 41. {4-[2,2-Difluoro-2-(2-methoxyphenyl)ethoxy]phenyl}acetonitrile

To a solution of Intermediate 40 (6.12 g, 21.83 mmol) in methylene chloride (22 mL) was added DAST (8.58 mL, 65.48 mmol). The reaction mixture was stirred 12 hours at rt. and 5 hours at reflux. After this reaction time, DAST (4 mL, 30.52 mmol) was added and the reaction mixture was heated at reflux overnight. The reaction mixture was then diluted with methylene chloride (20 mL) and poured into a stirred mixture of a saturated solution of potassium carbonate (100 mL) and ice (100 g). The aqueous layer was separated and washed with methylene chloride (2 x 50 mL). The combined organic layers were washed with brine (100 mL), dried (Na₂SO₄) and the solvent was removed under reduced pressure. The residue was purified by column chromatography with silica gel, eluting with n-hexane/ethyl acetate (from 10:1 to 1:1) to give the title compound (4.71 g, 71 %) as a yellow solid.

### Intermediate 42. 2-{4-[2,2-Difluoro-2-(2-methoxyphenyl)ethoxy]phenyl}ethanamine

To a solution of Intermediate 41 (1.00 g, 3.29 mmol) in methanol (22 mL) and tetrahydrofuran (8 mL) were added a solution of concentrated hydrochloric acid (37%, 0.61 mL) and platinum (IV) oxide (0.07 g, 0.30 mmol). The resulting mixture was hydrogenated for 4 hours. The catalyst was filtered through Celite® and the solvents removed under reduced pressure. The crude was partitioned between saturated solution of potassium carbonate (50 mL) and methylene chloride (50 mL). The aqueous layer was separated and washed with methylene chloride (2 x 25 mL). The combined organic extracts were washed with brine (50 mL), dried (MgSO₄) and the solvent removed under reduced pressure. The residue was purified by column chromatography with silica gel, eluting with methylene chloride/methanol/aqueous ammonia (from 95:5:0 to 80:20:2) to give the title compound (0.51 g, 51 %).

### Intermediate 43.8-(Benzyloxy)-5-{(1R,S)-2-[(2-{4-[2,2-difluoro-2-(2-methoxyphenyl) ethoxy]phenyl}ethyl)amino]-1-hydroxyethyl}quinolin-2(1H)-one

Obtained from Intermediate 42 (0.50 g, 1.62 mmol), 8-(benzyloxy)-5-(dihydroxyacetyl)quinolin-2(1*H*)-one (0.53 g, 1.62 mmol) and sodium borohydride (0.18 g, 4.85 mmol) by the procedure described in Intermediate 34. Purification by column chromatography with silica gel and methylene chloride/methanol (from 100:0 to 9:1) as eluent yielded the title compound (0.44 g, 46%).

### Example 15. 5-{(1R,S)-2-[(2-{4-[2,2-Difluoro-2-(2-methoxyphenyl)ethoxy]phenyl} ethyl)amino]-1-hydroxyethyl}-8-hydroxyquinolin-2(1H)-one

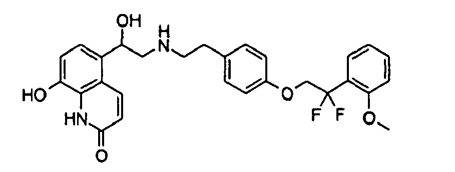

Obtained from Intermediate 43 (0.44 g, 0.74 mmol) and palladium on charcoal (10%, 0.04 g) by the procedure described in Example 1. Purification by column chromatography with silica gel and methylene chloride/methanol/aqueous ammonia (from 90:10:0.5 to 80:20:2) as eluent gave the title compound (0.16 g, 42%) as a yellow solid.
¹H-NMR (300 MHz, dimethylsulfoxide-D6): 2.60-2.70 (m, 2H); 2.71-2.85 (m, 4H); 3.81 (s, 3H); 4.59 (t, *J*_{F-H}=13.5 Hz, 2H); 5.02-5.08 (m, 1H); 6.50 (d, *J*=9.0 Hz, 1H); 6.80-6.95 (m, 3H); 7.01-7.18 (m, 5H); 7.45-7.56 (m, 2H); 8.15 (d, *J*=9.0 Hz, 1H). MS (M+): 511.

### Intermediate 44. 1-(4-Bromophenoxy)-3-phenylacetone

To a solution of 2-(4-bromophenoxy)acetic acid (8.0 g, 34.6 mmol) in anhydrous tetrahydrofuran (120 mL) was added 4-methylmorpholine (11.4 mL, 0.104 mol) and 2-chloro-4,6-dimethoxy-1,3,5-triazine (7.3 g, 41.6 mmol). The mixture was stirred at room temperature for 1 hour and then, N,O-dimethylhydroxylamine hydrochloride (3.38 g, 34.6 mmol) was slowly added. The reaction was stirred at room temperature overnight. The precipitated solid was filtered and the resulting solution was cooled to -40°C. Then, a 2M solution of benzylmagnesium chloride in anhydrous tetrahydrofuran (17.6 mL, 35.3 mmol) was slowly added under nitrogen. The mixture was warmed to room temperature for 2 hours and a saturated solution of ammonium chloride (200 mL) was added. The solvent was removed under reduced pressure and the aqueous residue was extracted with ethyl acetate (2 x 75 mL). The organic layer was washed with 1 N hydrochloric acid (2 x 50 mL), water (2 x 50 mL), and brine (50 mL), dried (Na₂SO₄) and the solvent was removed under reduced pressure. The title compound was obtained (3.7 g, 35%) as an oil.

### Intermediate 45. 1-Bromo-4-(2,2-difluoro-3-phenylpropoxy)benzene

A solution of Intermediate 44 (8.0 g, 30 mmol) in DAST (17.2 mL, 130 mmol) was stirred at 45°C in a sealed tube overnight. After cooling, the mixture was diluted with methylene chloride (100 mL) and a cooled solution of saturated solution of sodium bicarbonate was slowly added until the mixture reached pH=6. The organic phase was separated and washed with saturated solution of sodium bicarbonate (2 x 100 mL) and brine (75 mL), dried (Na₂SO₄), and the solvent removed under reduced pressure. The resulting oil was purified by column chromatography with silica gel, eluting with n-hexane/ethyl acetate (from pure n-hexane to 90:1) to give the title compound (5 g, 58%) as an oil.

### Intermediate 46. 1-[4-(2,2-Difluoro-3-phenylpropoxy)phenyl]acetone

Obtained from Intermediate 45 (5.0 g, 15.3 mmol), isoprenyl acetate (2.52 mL, 22.9 mmol), tri-n-butyltin methoxide (5.28 mL, 18.9 mmol), palladium (II) acetate (170mg), and tri-o-tolylphosphine (470 mg) by the procedure described in Intermediate 3. Purification by column chromatography with silica gel and n-hexane/ethyl acetate (from 6:1 to 4:1) as eluent gave 1-[4-(2,2-difluoro-3-phenylpropoxy)-phenyl]acetone (1.90 g, 41%) as an oil.

### Intermediate 47. (2R,S)-1-[4-(2,2-Difluoro-3-phenylpropoxy)phenyl]propan-2-amine

Obtained from Intermediate 46 (1.90 g, 6.2 mmol), ammonium acetate (4.81 g, 62.4 mmol), and sodium cyanoborohydride (1.60 g, 25.0 mmol) by the procedure described in Intermediate 6. The title compound was obtained (0.88 g, 42%) as an oil.

### Intermediate 48. 8-(Benzyloxy)-5-[(1R)-1-{[tert-butyl(dimethyl)silyl]oxy}-2-({(1R,S)-2-[4-(2,2-difluoro-3-phenylpropoxy)phenyl]-1-methylethyl}amino)ethyl]quinolin-2(1H)-one

Obtained from Intermediate 47 (0.88 g, 2.88 mmol), (*R*)-8-(benzyloxy)-5-(2-bromo-1-(*tert-*butyldimethylsilyloxy)ethyl)quinolin-2(1*H*)-one (0.94 g, 1.92 mmol), potassium carbonate (0.80 g, 3.01 mmol), and sodium iodide (0.37 g, 1.16 mmol) by the procedure described in Intermediate 7 (reaction time: 3 hours). Purification by column chromatography with silica gel, eluting with methylene chloride/methanol (from 100:1 to 30:1) gave 8-(benzyloxy)-5-[(1*R*)-1-{[*tert*-butyl(dimethyl)silyl]oxy}-2-({(1*R*,*S*)-2-[4-(2,2-difluoro-3-phenylpropoxy)phenyl]-1-methylethyl}amino)ethyl]quinolin-2(1*H*)-one (0.32 g, 23%).

### Intermediate 49. 8-(Benzyloxy)-5-[(1R)-2-({(1R,S)-2-[4-(2,2-difluoro-3-phenyl-propoxy)phenyl]-1-methylethyl}amino)ethyl]quinolin-2(1H)-one

Obtained from Intermediate 48 (0.32 g, 0.45 mmol) and tetra-n-butyl ammonium fluoride trihydrate (0.23 g, 0.73 mmol) by the procedure described in Intermediate 8. The title compound was obtained (0.26 g, 92%) as an oil and was used in the next step without further purification.

### Example 16. 5-[(1R)-2-({(1R,S)-2-[4-(2,2-difluoro-3-phenylpropoxy)phenyl]-1-methyl-ethyl}amino)-1-hydroxyethyl)-8-hydroxyquinolin-2(1H)-one

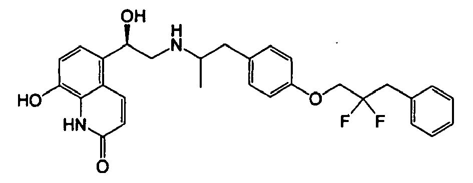

To a solution of Intermediate 49 (0.26 g, 0.43 mmol) in methanol (15 mL) was added palladium on charcoal (10%, 40 mg). The mixture was hydrogenated at 2.76 bar for 42 hours. The catalyst was filtered through Celite® and the solvent removed under reduced pressure. The crude oil obtained was purified by column chromatography eluting with methylene chloride/methanol/aqueous ammonia (from 150:40:1 to 40:8:1) to give the title compound (15 mg, 7%) as an off-white solid.
¹H-NMR (300 MHz, Cl₃CD): 1.08 (t, *J*=5.2 Hz, 3H); 2.58-2.99 (m, 5H); 3.34 (t, *J*=16.5 Hz, 2H); 3.93 (t, *J*=11.3 Hz, 2H); 4.81 (bs, 1H); 5.37 (bs, 2 H) 6.59-6.61 (m, 1H); 6.80 (d, *J*=8.2 Hz, 2H); 6.86-6.89 (m, 1H); 7.06 (d, *J*=8.0 Hz, 2H); 7.26-7.33 (m, 7H); 8.42 (bs, 1H). MS (M+): 509.

### Intermediate 50. 3-Oxo-3-phenylpropyl acetate

To a solution of 3-chloro-1-phenylpropan-1-one (30.0 g, 0.18 mol) in acetic acid (240 mL) was added sodium acetate (73 g, 0.89 mol) and potassium iodide (3.0 g, 20 mmol). The mixture was distributed into three sealed tubes and heated at 130°C overnight. After cooling, the combined reaction mixtures were diluted with water (200 mL) and extracted with methylene chloride (3 x 100 mL). The combined organic extracts were washed with water (2 x 100 mL), saturated solution of sodium bicarbonate (2 x 100 mL) and brine (75 mL), dried (Na₂SO₄) and the solvent removed under reduced pressure. The title compound was obtained (28.0 g, 82%) as an orange solid.

### Intermediate 51. 3,3-Difluoro-3-phenylpropyl acetate

Obtained from Intermediate 47 (14.0 g, 70.0 mmol) and DAST (95 mL, 0.73 mol) by the procedure described in Intermediate 45. Purification by column chromatography with silica gel, eluting with n-hexane/ethyl acetate (from pure n-hexane to 4:1) gave the title compound (4 g, 26%) as an oil.

### Intermediate 52. 3,3-Difluoro-3-phenylpropan-1-ol

To a suspension of Intermediate 51 (9.4 g, 43.9 mol) in ethanol (125 mL) was added 35% sodium hydroxide (30 mL). The mixture was stirred at room temperature for 2 hours. The crude reaction was diluted with methylene chloride (150 mL), washed with water (1 x 50 mL) and 1 N hydrochloric acid (2 x 50 mL), dried (Na₂SO₄) and the solvent removed under reduced pressure. The title compound was obtained (6.5 g, 86%) as an oil, and was used in the next step without further purification.

### Intermediate 53. [4-(3,3-Difluoro-3-phenylpropoxy)phenyl]acetonitrile

To a solution of Intermediate 52 (0.80 g, 4.65 mmol) in anhydrous tetrahydrofuran (25 mL) were added 2-(4-hydroxyphenyl)acetonitrile (0.62 g, 4.66 mmol), triphenylphosphine (1.80 g, 6.98 mmol), and diethyl azodicarboxylate (1.30 ml, 6.98 mmol). The mixture was refluxed under nitrogen for 48 hours. After cooling, the solvent was removed under reduced pressure. The residue was dissolved in methylene chloride (50 mL), washed with saturated solution of sodium bicarbonate (2 x 25 mL) and water (50 mL), dried (Na₂SO₄), and the solvent removed under reduced pressure. The resulting oil was purified by column chromatography with silica gel, eluting with n-hexane/ethyl acetate (from 15:1 to 10: 1), to yield [4-(3,3-difluoro-3-phenylpropoxy)phenyl]acetonitrile (0.42 g, 31 %) as an oil.

### Intermediate 54. {2-[4-(3,3-difluoro-3-phenylpropoxy)phenyl]ethyl}amine

Obtained from Intermediate 53 (0.54 g, 1.9 mmol), platinum (IV) oxide (43 mg), and concentrated hydrochloric acid (0.3 mL) by the procedure described in Intermediate 31 (reaction time: 3 hours). The title compound was obtained (0.40 g, 73%) as an oil and was used in the next step without further purification.

### Intermediate 55. 8-(Benzyloxy)-5-[(1R,S)-[2-({2-[4-(3,3-difluoro-3-phenylpropoxy)-phenyl]ethyl}amino)-1-hydroxyethyl]quinolin-2(1H)-one

A solution of Intermediate 54 (0.38 g, 124 mmol) and 8-(benzyloxy)-5-(dihydroxyacetyl)quinolin-2(1*H*)-one (0.40 g, 1.37 mmol) in a mixture of tetrahydrofuran (20 mL) and ethanol (20 mL) was stirred at room temperature for 30 min. The reaction mixture was cooled to 0°C. Sodium borohydride (0.1 g, 3.4 mmol) was added at the same temperature and the resulting mixture was stirred at room temperature for 3 hours. The solvents were removed under reduced pressure and the crude was partitioned between ethyl acetate (50 mL) and water (50 mL). The organic layer was separated, washed with water (25 mL) and brine (50 mL), dried (MgSO₄) and the solvent removed under reduced pressure. The residue was purified by column chromatography with silica gel, eluting with methylene chloride/methanol (from 40:1 to 20:1) to give the title compound as an oil (0.42 g, 58%).

### Example 17. 5-[(1R,S)-2-{[4-(3,3-Difluoro-3-phenylpropoxy)benzyl]amino}-1-hydroxyethyl)]-8-hydroxyquinolin-2(1H)-one

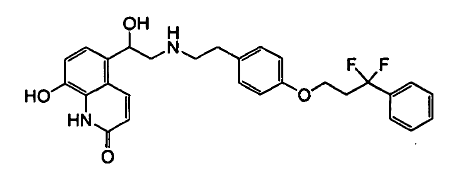

Obtained from Intermediate 55 (0.42 g, 0.72 mmol) and palladium on charcoal (10%, 80 mg) by the procedure described in Example 16 (reaction time: 5 hours). Purification by column chromatography with silica gel, eluting with methylene chloride/methanol 9:1 gave 5-[(1*R*,*S*)-[2-{[4-(3,3-difluoro-3-phenylpropoxy)benzyl]amino}-1-hydroxyethyl)]-8-hydroxyquinolin-2(1H)-one (95 mg, 27%) as an off-white solid.
¹H-NMR (300 MHz, dimethylsulfoxide-D6): 2.74-2.96 (m, 6H); 3.52-3.61 (m, 2H); 4.12 (t, *J*=6.3 Hz, 2H); 4.85 (bs, 1H); 5.08 (t, *J*=5.7 Hz, 1H); 5.15 (t, *J*=5.7 Hz, 1H); 5.40 (s, 1H); 6.56-6.65 (m, 2H); 6.81-6.87 (m, 1H); 6.94 (bs, 1H); 6.69-7.05 (m, 2H); 7.14-7.19 (m, 3H); 7.57-7.66 (m, 3H); 8.22-8.32 (m, 2H).
MS (M+): 495.

### Intermediate 56. [4-(2-Hydroxy-3-phenoxypropoxy)phenyl]acetonitrile

To a solution of (4-hydroxyphenyl)acetonitrile (5.03 g, 37.8 mmol) in anhydrous dimethylformamide (100 mL) were added 2-(phenoxymethyl)oxirane (5.0 mL, 33.3 mmol) and 1,4-diazabicyclo[2.2.2]octane (0.42 g, 3.7 mmol). The mixture was stirred at 130°C under nitrogen for 6 hours. More 1,4-diazabicyclo[2.2.2]octane (0.40 g, 3.6 mmol) was added and the mixture was stirred for further 2 hours at the same conditions. After cooling, the mixture was diluted with ethyl acetate (100 mL) and poured into cooled 2N hydrochloric acid (200 mL). The organic phase was separated, washed with 2N hydrochloric acid (3 x 50 mL), water (2 x 50 mL), and brine (100 mL), dried (Na₂SO₄), and the solvent removed under reduced pressure. The resulting oil was purified by column chromatography with silica gel, eluting with n-hexane/ethyl acetate 4:1 to yield the title compound (4.28 g, 45 %) as an oil.

### Intermediate 57. [4-(2-Oxo-3-phenoxypropoxy)phenyl]acetonitrile

To a solution of Intermediate 56 (5.55 g, 19.6 mmol) in anhydrous methylene chloride (60 mL) was added Dess-Martin reagent (12.5 g, 29.4 mmol). The mixture was stirred at room temperature under nitrogen for 1,5 hours. The reaction mixture was washed with saturated solution of sodium bicarbonate (2 x 30 mL), water (2 x 30 mL), and brine (30 mL). A solid was separated by filtration. The organic phase was dried (Na₂SO₄) and the solvent removed under reduced pressure. The resulting oil was purified by column chromatography with silica gel, eluting with methylene chloride to give [4-(2-oxo-3-phenoxypropoxy)phenyl]acetonitrile (2.77 g, 50 %) as an oil.

### Intermediate 58. [4-(2,2-Difluoro-3-phenoxypropoxy)phenyl]acetonitrile

Obtained from Intermediate 57 (2.77 g, 9.85 mmol) and DAST (6.5 mL, 49.6 mmol) by the procedure described in Intermediate 45. The title compound was obtained (3.0 g, 100%) as an oil and was used in the next step without further purification.

### Intermediate 59. {2-[4-(2,2-Difluoro-3-phenoxypropoxy)phenyl]ethyl}amine

To a solution of Intermediate 58 (3.07 g, 10.1 mmol) in methanol (100 mL) were added concentrated hydrochloric acid (1.3 mL) and platinum (IV) oxide (240 mg). The mixture was hydrogenated at 2.76 bar overnight. The catalyst was filtered through Celite® and the solvent removed under reduced pressure. The resulting oil was dissolved in methylene chloride (100 mL), washed with saturated solution of sodium bicarbonate (3 x 100 mL), water (2 x 50 mL), and brine (50 mL), dried (Na₂SO₄), and the solvent removed under reduced pressure. The resulting oil was purified by column chromatography with silica gel, eluting with methylene chloride/ethanol/aqueous ammonia (100:8:1) to give the title compound (1.11 g of 75% purity, 27 % yield) as an oil.

### Example 18. 5-[(1R,S)-[2-({2-[4-(2,2-difluoro-3-phenoxypropoxy)phenyl]ethyl}-amino]-1-hydroxyethyl]-8-hydroxyquinolin-2(1H)-one

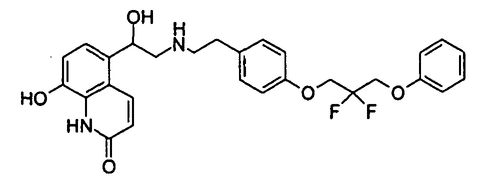

A solution of Intermediate 59 (0.80 g of 75% purity, 1.95 mmol) and 8-(benzyloxy)-5-(dihydroxyacetyl)quinolin-2(1H)-one (0.60 g, 1.95 mmol) in dimethylsulfoxide (7 mL) was stirred at room temperature for 4 hours. After this reaction time, methanol (7 mL) and sodium borohydride (0.22 g, 5.87 mmol) were successively added and the reaction mixture was stirred at room temperature overnight before being partitioned between ethyl acetate (50 mL) and a saturated solution of sodium bicarbonate (50 mL). The organic layer was separated, washed with water (2 x 20 mL), dried (Na2SO4) and the solvents removed under reduced pressure. The residue was partially purified by column chromatography with silica gel, eluting with methylene chloride/ethanol/aqueous ammonia (100:8:1). The resulting oil (0.85 g of 54% purity) was dissolved in methanol (20 mL) before adding 10 drops of 1.25 M hydrochloric acid solution in methanol and palladium on charcoal (10%, 72 mg). The mixture was hydrogenated at 2.76 bar oveernight. The catalyst was filtered through Celite® and the solvent removed under reduced pressure. The crude was dissolved in a 40:8:1 mixture of methylene chloride/methanol/aqueous ammonia (40 mL). The resulting solid was filtered and purified by column chromatography with C18 reverse phase, eluting with water (20 mM CH3COONH4, pH = 7) and acetonitrile/methanol (20mM CH3COONH4, pH = 7) (from 100:30 to 100:80) to give the title compound (0.175 g, 18% overall yield) as a yellow solid.
¹H-NMR (300 MHz, dimethylsulfoxide-D⁶): 2.54-2.84 (m, 6H); 4.32-4.69 (m, 4H); 5.02 (bs, 1H); 6.50 (d, *J*=9.61 Hz, 1H); 6.78-7.20 (m, 9H); 7.32 (t, *J*=7.28 Hz, 2H); 8.17 (d, *J*=9.89 Hz, 1H)
EM (M+): 511

### Intermediate 60. [3-Methoxy-4-(2-oxo-2-phenylethoxy)phenyl]acetonitrile

To a solution of 2-(4-hydroxy-3-methoxyphenyl)acetonitrile (2.27 g, 13.9 mmol) and potassium carbonate (2.90 g, 21.27 mmol) in water (100 mL) was added tetrabutylammonium bromide (250 mg, 0.74 mmol) and a solution 2-bromo-1-phenylethanone (2.80 g, 14.1 mmol) in methylene chloride (100 mL). The resulting mixture was refluxed overnight. The organic layer was separated, washed with water (2 x 50 mL) and brine (100 mL), dried (Na₂SO₄) and the solvent removed under reduced pressure. The residue was triturated with n-hexane and the precipitate was collected by filtration to obtain the title compound as a pale yellow solid (3.60 g, 92%).

### Intermediate 61. [4-(2,2-Difluoro-2-phenylethoxy)-3-methoxyphenyl]acetonitrile

Obtained from Intermediate 60 (3.60 g, 12.8 mmol) and DAST (8.40 mL, 64.1 mmol) by the procedure described in Intermediate 45. Purification by column chromatography with silica gel, eluting with methylene chloride gave [4-(2,2-difluoro-2-phenylethoxy)-3-methoxyphenyl]acetonitrile (3.0 g, 77%) as an oil.

### Intermediate 62. {2-[4-(2,2-Difluoro-2-phenylethoxy)-3-methoxyphenyl]ethyl}amine

Obtained from Intermediate 61 (3.21 g, 10.58 mmol), platinum (IV) oxide (240 mg) and concentrated hydrochloric acid (1.3 mL) by the procedure described in Intermediate 59 (reaction time: 2.5 hours). Purification by column chromatography with silica gel, eluting with methylene chloride/ethanol/aqueous ammonia (100:8:1) gave the title compound (1.3 g, 40%) as an oil.

### Intermediate 63. 8-(Benzyloxy)-5-[(1R,S)-[2-({2-[4-(2,2-difluoro-2-phenylethoxy)-3-methoxyphenyl]ethyl}amino)-1-hydroxyethyl]quinolin-2(1H)-one

Obtained from Intermediate 62 (0.65 g, 2.11 mmol), 8-(benzyloxy)-5-(dihydroxyacetyl)quinolin-2(1*H*)-one (0.65 g, 2.12 mmol) and sodium borohydride (0.24 g, 6.34 mmol) by the procedure described in Intermediate 34. Purification by column chromatography with silica gel, eluting with methylene chloride/ethanol/aqueous ammonia (100:8:1) gave the title compound (0.76 g, 60%) as an oil.

### Example 19. 5-[(1R,S)-[[2-({2-[4-(2,2-difluoro-2-phenylethoxy)-3-methoxyphenyl]-ethyl}amino)-1-hydroxyethyl]-8-hydroxyquinolin-2(1H)-one, formiate

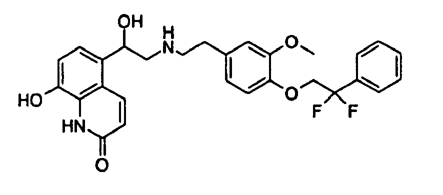

To a solution of Intermediate 63 (0.76 g, 1.27 mmol) in methanol (25 mL) were added 10 drops of 1.25 M hydrochloric acid solution in methanol and palladium on charcoal (10%, 72 mg). The mixture was hydrogenated at 2.76 bar for 3.5 hours. The catalyst was filtered through Celite® and the solvent removed under reduced pressure. The crude was dissolved in a 40:8:1 mixture of methylene chloride/methanol/aqueous ammonia (40 mL). The resulting solid was filtered and purified by column chromatography with C₁₈ reverse phase, eluting with water/acetonitrile/methanol (HCOONH4, pH=3) (from 100/0/0 to 0/50/50) to give the title compound (0.22 g, 4031%) as an off-white solid.
¹H-NMR (400 MHz, CD₃OD): 2.93-2.99 (m, 2H); 3.20-3.18 (m, 4H); 3.79 (s, 3H); 4.41 (t, *J*=12.5 Hz, 2H); 5.35-5.39 (m, 1H); 6.68 (d, *J*=9.8 Hz, 1H); 6.75-6.78 (m, 1H); 6.85-6.89 (m, 2H); 7.01 (d, *J*=8.2 Hz, 1H); 7.27 (d, *J*=8.2 Hz, 1H); 7.45-7.49 (m, 2H); 7.59-7.62 (m, 2H); 8.35 (d, *J*=9.8 Hz, 1H); 8.53 (bs, 1H).
MS (M+): 511

### Intermediate 64. [4-(2-Hydroxy-3-phenylpropoxy)phenyl]acetonitrile

Obtained from (4-hydroxyphenyl)acetonitrile (4.96 g, 37.3 mmol), 2-benzyloxirane (5.00 g, 37.3 mmol), and 1,4-diazabicyclo[2.2.2]octane (0.8 g, 7.5 mmol) by the procedure described in Intermediate 56. The resulting solid was triturated with n-hexane and the precipitate was collected by filtration to obtain the title compound as an off-white solid (7.44 g, 75%).

### Intermediate 65. [4-(2-Oxo-3-phenylpropoxy)phenyl]acetonitrile

Obtained from Intermediate 64 (7.00 g, 26.2 mmol) and Dess-Martin reagent (16.6 g, 39.3 mmol) by the procedure described in Intermediate 57. The residue was purified by column chromatography with silica gel, eluting with n-hexane/ethyl acetate (from 4:1 to 1:1) to yield [4-(2-oxo-3-phenylpropoxy)phenyl]acetonitrile (4.54 g, 65%).

### Intermediate 66. [4-(2,2-Difluoro-3-phenylpropoxy)phenyl]acetonitrile

Obtained from Intermediate 65 (4.54 g, 17.1 mmol) and DAST (11.2 mL, 85.5 mmol) by the procedure described in Intermediate 45. Purification by column chromatography with silica gel, eluting with n-hexane/methylene chloride (from 2:1 to pure methylene chloride) gave the title compound (3.45 g, 70%).

### Intermediate 67. {2-[4-(2,2-Difluoro-3-phenylpropoxy)phenyl]ethyl}amine, hydrochloride

Obtained from Intermediate 66 (2.10 g, 7.31 mmol), concentrated hydrochloric acid (1.1 mL), and platinum (IV) oxide (200 mg) by the procedure described in Intermediate 59 (reaction time: 1 hour). The resulting solid was triturated with n-hexane and the precipitate was collected by filtration to obtain the title compound as an off-white solid (1.73 g, 72%).

### Intermediate 68. 8-(Benzyloxy)-5-[(1R,S)-[2-({2-[4-(2,2-difluoro-3-phenylpropoxy)-phenyl]ethyl}amino)-1-hydroxyethyl]quinolin-2(1H)-one, formiate

Obtained from Intermediate 67 (1.10 g, 3.78 mmol), 8-(benzyloxy)-5-(dihydroxyacetyl)quinolin-2(1H)-one (1.16 g, 3.77 mmol) and sodium borohydride (0.4 g, 11.4 mmol) by the procedure described in Intermediate 34. Purification by column chromatography with C18 reverse phase, eluting with water/acetonitrile/methanol (HCOONH4, pH=3) (from 100/0/0 to 0/50/50) gave the title compound (0.83 g, 36%) as an off-white solid.

### Example 20. 5-[(1R,S)-2-({2-[4-(2,2-difluoro-3-phenylpropoxy)phenyl]ethyl}amino)-1-hydroxyethyl]-8-hydroxyquinolin-2(1H)-one, formiate

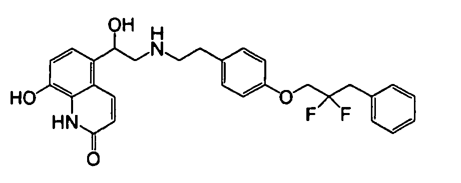

Obtained from Intermediate 68 (0.83 g, 1.32 mmol) and palladium on charcoal (10%, 85 mg) by the procedure described in Example 16 (reaction time: 12 hours). The residue was triturated with ethyl ether/n-hexane and the precipitate was collected by filtration to obtain the title compound as an off-white solid (0.52 g, 72%).
¹H-NMR (300 MHz, dimethylsulfoxide-D⁶): 2.65-2.95 (m, 6H); 3.39 (t, *J*=17.58 Hz, 2H); 4.18 (t, *J*=12.64 Hz, 2H); 5.12 (t, *J*=5.91 Hz, 1H); 6.52 (d, *J*=9.89 Hz, 1H); 6.87-6.98 (m, 3H); 7.03-7.19 (m, 3H); 7.25-7.39 (m, 5H); 8.17 (d, *J*=9.89 Hz, 1H); 8.29 (s, 1H)
EM (M+): 495

### Intermediate 69. 2-(4-bromophenoxy)-N-methoxy-N-methylacetamide

To a solution of 2-(4-bromophenoxy)acetic acid (8 g, 34.6 mmol) in tetrahydrofuran (120 mL) was added 4-methylmorpholine (11.4 g, 103.8 mmol) and 2-Chloro-4,6-dimethoxy-1,3,5-triazine (7.3 g, 41.5 mmol). The reaction mixture was stirred at room temperature for 1 hour. N,O-dimethylhydroxylamine (3.38 g, 34.6 mmol) was slowly added into the reaction mixture and stirred at room temperature overnight. The precipitate was filtered and the solution was used as a title compound in the next reaction without further purification.

### Intermediate 70. 1-(4-bromophenoxy)-4-phenylbutan-2-one

To a solution of Intermediate 69 (4.4 g, 16.09 mmol) in anhydrous tetrahydrofuran was slowly added phenethylmagnesium chloride (16 mL, 16 mmol) at -78°C under nitrogen. The reaction mixture was stirred at room temperature overnight. Ammonium chloride was added into the reaction mixture and solvents were removed under reduced pressure. The crude was partitioned between ethyl acetate and water and the organic layer was washed with water, diluted hydrochloric acid, brine and dried (Mg*S*O₄). The solvent was removed under reduced pressure and the residue was purified by column chromatography with silica gel, eluting by methylene chloride to give the title compound as solid (4 g, 78%).

### Intermediate 71. 1-bromo-4-(2,2-difluoro-4-phenylbutoxy)benzene

Obtained from Intermediate 70 (5.4 g, 17.17 mmol) and DAST (11.25 mL, 85.85 mmol) by the procedure described in Intermediate 45. Purification by column chromatography with silica gel, eluting with methylene chloride gave the title compound (4.3 g, 73%) as an oil.

### Intermediate 72. 1-[4-(2,2-difluoro-4-phenylbutoxy)phenyl]acetone

Obtained from Intermediate 71 (4.27 g, 12.52 mmol), isoprenyl acetate (2.07 mL, 18.77 mmol), tri-n-butyltin methoxide (4.3 mL, 15.02 mmol), palladium (II) acetate (0.1 g, 0.63 mmol), and tri-o-tolylphosphine (400 mg, 1.25 mmol) by the procedure described in Intermediate 3. Purification by column chromatography with silica gel and n-hexane/ethyl acetate (from 20:1 to 8:1) as eluent gave the title compound as an oil (0.68 g, 17%).

### Intermediate 73. (2R,S)-{2-[4-(2,2-difluoro-4-phenylbutoxy)phenyl]-1-methylethyl}amine

Obtained from Intermediate 72 (0.68 g, 2.14 mmol), ammonium acetate (1.6 g, 21.41 mmol), and sodium cyanoborohydride (0.53 g, 8.53 mmol) by the procedure described in Intermediate 6. Purification by column chromatography with silica gel, eluting by methylene chloride/ethanol/aqueous ammonia (100:8:1) gave the title compound (0.68 g, 67%) as an oil.

### Intermediate 74. 8-(benzyloxy)-5-[(1R,S)-2-((1R,S)-{2-[4-(2,2-difluoro-4-phenylbutoxy)phenyl]-1-methylethyl}amino)-1-hydroxyethyl]quinolin-2(1H)-one

Obtained from Intermediate 73 (0.46 g, 1.43 mmol), 8-(benzyloxy)-5-(dihydroxyacetyl)quinolin-2(1H)-one (0.44 g, 1.43 mmol) and sodium borohydride (0.16 g, 4.31 mmol) by the procedure described in Intermediate 43. Purification by column chromatography with silica gel, eluting with methylene chloride/ethanol/aqueous ammonia (100/8/1) gave the title compound (0.87 g, 85%) as an oil.

### EXAMPLE 21. 5-[(1R,S)-2-({2-[4-(2,2-diftuoro-4-phenylbutoxy)phenyl]-1-methylethyl}amino)-1-hydroxyethyl]-8-hydroxyquinolin-2(1H)-one

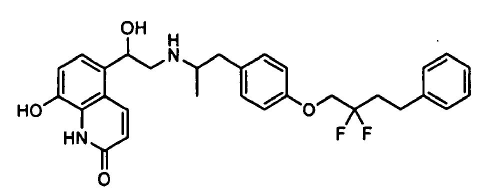

To a solution of Intermediate 74 (0.75 g, 1.22 mmol) in methanol (14 mL) were added 7 drops of a saturated hydrochloric acid solution in ethanol and palladium on charcoal (10%, 32 mg). The mixture was hydrogenated at 2.76 bar for 2 days. The catalyst was filtered through Celite® and the solvent removed under reduced pressure. The crude oil obtained was purified by column chromatography eluting with methylene chloride/methanol/aqueous ammonia (from 80:8:1 to 40:8:1) to give the title compound (0.23 g, 61 %) as an oil.

¹H-NMR (300 MHz, dimethylsulfoxide-D6): 0.94 (d, *J*=6.04 Hz, 3H); 2.31-2.51 (m, 4H); 2.64-2.89 (m, 7H); 4.36 (t, *J*_{F-H}=13.19 Hz, 2H); 5.03 (bs, 1H); 6.56 (d, *J*=9.89 Hz, 1H); 6.93-6.98 (m, 3H); 7.08-7.16 (m, 3H); 7.24-7.38 (m, 5H); 8.22 (d, *J*=9.90 Hz, 1H).
MS (M+): 523.

### Intermediate 75. 1-(4-bromophenyl)-2-phenoxyethanone

Obtained from 2-bromo-1-(4-bromophenyl)ethanone (10.3 g, 37.06 mmol), phenol (3.66 g, 38.89 mmol) and potassium carbonate (5.63 g, 40.74 mmol) by the same procedure described in Intermediate 1. The title compound was obtained as a brown solid (10.68 g, 89%) and used in the next step without further purification.

### Intermediate 76. 1-bromo-4-(1,1-difluoro-2-phenoxyethyl)benzene

Obtained from Intermediate 75 (5 g, 17.17 mmol) and DAST (6.75 mL, 51.51 mmol) by the same procedure described in Intermediate 2. The crude was purified by column chromatography with silica gel, eluting with n-hexane/ethyl acetate (from 10:1 to 3: 1) to give the title compound as a solid (4.5 g, 83%).

### Intermediate 77. 1-[4-(1,1-difluoro-2-phenoxyethyl)phenyl]acetone

Obtained from Intermediate 76 (2 g, 6.39 mmol), isoprenyl acetate (1.06 mL, 9.62 mmol), tri-n-butyltin methoxide (2.21 mL, 7.67 mmol), palladium (II) acetate (0.07 g), and tri-o-tolylphosphine (190 mg) by the procedure described in Intermediate 3. Purification by column chromatography with silica gel and n-hexane/ethyl acetate (10: 1) as eluent yielded the title compound as an oil (1.02 g, 55%).

### Intermediate 78. (2R,S)-{2-[4-(1,1-difluoro-2-phenoxyethyl)phenyl]-1-methylethyl}amine

Obtained from Intermediate 77 (0.76 g, 2.64 mmol), ammonium acetate (2.03 g, 26.35 mmol), and sodium cyanoborohydride (0.66 g, 10.53 mmol) by the procedure described in Intermediate 6. The crude was purified by column chromatography with silica gel, eluting by methylene chloride/ethanol/aqueous ammonia (100:8:1) to give the title compound (0.59 g, 76%) as an oil.

### Intermediate 79. 8-(benzyloxy)-5-[(1R)-1-{[tert-butyl(dimethyl)silyl]oxy}-2-({(1R,S)-2-[4-(1,1-difluoro-2-phenoxyethyl)phenyl]-1-methylethyl}amino)ethyl]quinolin-2(1H)-one

Obtained from Intermediate 78 (0.25 g, 0.88 mmol), (*R*)-8-(benzyloxy)-5-(2-bromo-1-(*tert-*butyldimethylsilyloxy)ethyl)quinolin-2(1*H*)-one (0.38 g, 0.8 mmol), sodium hydrogen carbonate (0.1 g, 1.19 mmol), and sodium iodide (0.01 g, 0.08 mmol) by the procedure described in Intermediate 13 (reaction time: overnight). The title compound was obtained without further purification as a solid (0.45 g, 80%).

### Intermediate 80. 8-(benzyloxy)-5-[(1R)-2-({(1R,S)-2-[4-(1,1-difluoro-2-phenoxyethyl)phenyl]-1-methylethyl}amino)-1-hydroxyethyl]quinolin-2(1H)-one

Obtained from Intermediate 79 (0.4 g, 0.58 mmol) and tetrabutylammonium trifluoride trihydrate (0.29 g, 0.93 mmol) by the same procedure described in Intermediate 8. The title compound was obtained as a solid (0.29 g, 88%) and used in the next step without further purification.

### EXAMPLE 22. 5-[(1R)-2-({(1R,S)-2-[4-(1,1-difluoro-2-phenoxyethyl)phenyl]-1-methylethyl}amino)-1-hydroxyethyl]-8-hydroxyquinolin-2(1H)-one

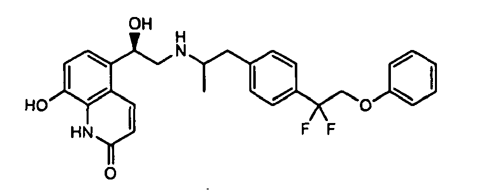

This compound is obtained from Intermediate 80 (0.3 g, 0.51 mmol) and palladium on charcoal (10%, 0.05 g) by the same procedure described in Intermediate 1. The crude was purified by column chromatography with silica gel, eluting with chloroform/methanol/aqueous ammonia (90:10:1) to give the title compound as a solid (0.21 g, 86%).
¹H-NMR (300 MHz, CD₃OD) 1.08 (bs, 3H); 2.60-3.06 (m, 5H); 4.36 (t, *J*_{F-H}=12.08 Hz, 2H); 5.17 (bs, 1H); 6.64 (d, *J*=9.89 Hz, 1H); 6.88-6.96 (m, 3H); 7.16 (d, *J*=8.24 Hz, 1H); 7.22-7.33 (m, 3H); 7.52 (t, *J*=7.5 Hz, 2H); 8.34 (d, *J*=9.88 Hz, 1H).
MS (M+): 495.

### Intermediate 81.8-(benzyloxy)-5-[(1R)-1-{[tert-butyl(dimethyl)silyl]oxy}-2-({2-[4-(3,3-difluoro-3-phenylpropoxy)phenyl]ethyl}amino)ethyl]quinolin-2(1H)-one

To a solution of Intermediate 55 (1.4 g, 4.81 mmol) and (*R*)-8-(benzyloxy)-5-(2-bromo-1-(*tert*-butyldimethylsilyloxy)ethyl)quinolin-2(1H)-one (1.95 g, 3.99 mmol) in toluene (20 mL) was added diisopropylethyl amine (0.8 mL, 4.81 mmol). The reaction mixture was stirred at 120°C for 3 hours. The solvent was removed under reduced pressure and the crude was partitioned between ethyl acetate and water. The organic layer was washed with water, bicarbonate and dried (MgSO₄). The solvent was removed under reduced pressure and the residue was purified by column chromatography with silica gel, eluting with methylene chloride/methanol/aqueous ammonia (150:2.5:0.1) to give the title compound as an oil (0.64 g, 23%).

### Intermediate 82. 8-(benzyloxy)-5-[(1R)-2-({2-[4-(3,3-difluoro-3-phenylpropoxy)phenyl]ethyl}amino)-1-hydroxyethyl]quinolin-2(1H)-one

Obtained from Intermediate 81 (0.64 g, 0.92 mmol) and tetrabutylammonium fluoride trihydrate (0.36 g, 1.38 mmol) by the same procedure described in Intermediate 8 (reaction time: 1 hour). The title compound was obtained as an oil (0.51 g, 95%) and used in the next step without further purification.

### EXAMPLE 23. 5-[(1R)-2-({2-[4-(3,3-difluoro-3-phenylpropoxy)phenyl]ethyl}amino)-1-hydroxyethyl]-8-hydroxyquinolin-2(1H)-one

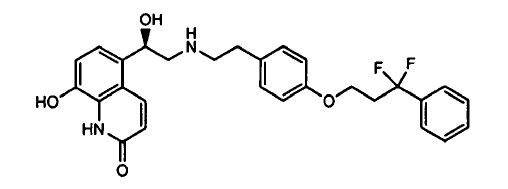

Obtained from Intermediate 82 (0.5 g, 0.86 mmol) and palladium on charcoal (10%, 0.1 g) by the same procedure described in Example 1 (reaction time: 5h). The crude was purified by column chromatography with silica gel, eluting by methylene chloride/methanol (from 98:1 to 9:1) to give the title compound as a solid (0.15 g, 34%).
¹H-NMR (300 MHz, CDCl₃): 2.70-3.17 (m, 10H); 4.10 (t, *J*_{F-H}=6.32 Hz, 2H); 5.48 (d, *J*=9.62 Hz, 1H); 6.61 (d, *J*=9.89 Hz, 1H); 6.85 (d, *J*=8.79 Hz, 2H); 7.06 (d, *J*=8.24 Hz, 2H); 7.17-7.22 (m, 3H); 7.54-7.62 (m, 5H); 8.3 (d, *J*=9.89 Hz, 1H).
MS (M+): 495.

### Intermediate 83. 4-oxo-4-phenylbutyl acetate

Obtained from 4-chloro-1-phenylbutan-1-one (25 g, 0.14 mol), sodium acetate (26.95 g, 0.33 mol) and potassium iodide (1.1 g, 0.01 mol) by the same procedure described in Intermediate 50. The crude was purified by column chromatography with silica gel, eluting with n-hexane/ethyl acetate (10:1) to give the title compound as an oil (11 g, 39%).

### Intermediate 84. 4,4-difluoro-4-phenylbutyl acetate

Obtained from Intermediate 83 (11 g, 0.05 mol) and DAST (69.9 mL, 0.53 mol) by the same procedure described in Intermediate 45. The crude was purified by column chromatography with silica gel, eluting with n-hexane/ethyl acetate (from 45:1 to 2:1) to give the title compound as an oil (3.19 g, 26%).

### Intermediate 85. 4,4-difluoro-4-phenylbutan-1-ol

Obtained from Intermediate 84 (3.16 g, 13.85 mmol) and sodium hydroxide (37%, 10 mL) by the same procedure described in Intermediate 52. The title compound was obtained as an oil (1.43 g, 55%) and used in the next step without further purification.

### Intermediate 86. [4-(4,4-difluoro-4-phenylbutoxy)phenyl]acetonitrile

Obtained from Intermediate 85 (1.1 g, 5.91 mmol), 2-(4-hydroxyphenyl)acetonitrile (0.76 g, 5.71 mmol), triphenylphosphine (2.4 g, 9.07 mmol), and diethyl azodicarboxylate (1.6 ml, 9.3 mmol) by the same procedure described in Intermediate 53 (reaction time: 54h). The crude obtained was purified by column chromatography with silica gel, eluting by n-hexane/ethyl acetate (from 15:1 to 3:1) to give the title compound as an oil (0.72 g, 42%).

### Intermediate 87. {2-[4-(4,4-difluoro-4-phenylbutoxy)phenyl]ethyl}amine

Obtained from Intermediate 86 (0.7 g, 2.32 mmol), platinum (IV) oxide (0.2 g, 0.88 mmol) and concentrated hydrochloric acid (37%, 0.5 mL) by the same procedure described in Intermediate 31 (reaction time: 4 hours). The title compound was obtained as an oil (0.5 g, 71%) and used in the next step without further purification.

### Intermediate 88. 8-(benzyloxy)-5-[(1R,S)-2-({2-[4-(4,4-difluoro-4-phenylbutoxy) phenyl]ethyl}amino)-1-hydroxyethyl]quinolin-2(1H)-one

Obtained from Intermediate 87 (0.5 g, 1.64 mmol), 8-(benzyloxy)-5-(dihydroxyacetyl)quinolin-2(1H)-one (0.50 g, 1.63 mmol) and sodium borohydride (0.2 g, 4.76 mmol) by the same procedure described in Intermediate 43. Purification by column chromatography with C18 reverse phase, eluting with water (20 mM CH3COONH4, pH = 7) and acetonitrile/methanol (20mM CH3COONH4, pH = 7) (from 100:30 to 100:80) gave the title compound as a solid (0.26 g, 27%).

### EXAMPLE 24. 5-[(1R,S)-2-({2-[4-(4,4-difluoro-4-phenylbutoxy)phenyl]ethyl}amino)-1-hydroxyethyl]-8-hydroxyquinolin-2(1H)-one

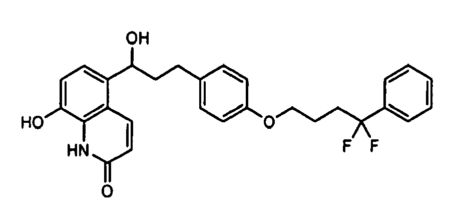

Obtained from Intermediate 88 (0.27 g, 0.44 mmol) and palladium on charcoal (10%, 0.05 g) by the same procedure described in Example 12 (reaction time: 20 hours). The crude was purified by column chromatography with C18 reverse phase, eluting with water (20 mM CH3COONH4, pH = 7) and acetonitrile/methanol (20mM CH3COONH4, pH = 7) (from 100:30 to 100:80) to give the title compound as a formiate salt (0.06 g, 23%).
¹H-NMR (300 MHz, CDCl₃): 1.73-1.82 (m, 2H); 2.29-2.45 (m, 3H); 2.51 (bs, 2H); 2.70-2.89 (m, 4H); 3.95 (t, *J_{F-H}*=6.05 Hz, 2H); 5.15 (bs, 1H); 6.52 (d, *J*=9.89 Hz, 1H); 6.82 (d, *J*=8.78 Hz, 2H); 6.95 (d, *J*=7.96 Hz, 12H); 7.07-7.11 (m, 3H); 7.51-7.53 (m, 5H); 8.18 (d, *J*=9.89 Hz, 1H).
MS (M+): 555.

### Intermediate 89.1-[4-(benzyloxy)-3-methylphenyl]ethanone

A solution of 1-(4-hydroxy-3-methylphenyl)ethanone (15 g, 0.10 mol) in acetone (150 mL) and potassium carbonate (20.7 g, 0.15 mol) was stirred at room temperature for 20 minutes. Then, (bromomethyl)benzene (13.7 mL, 0.11 mol) was added into the solution and the reaction mixture was stirred at reflux for 4 hours. The solvent was removed under reduced pressure and the crude was partitioned between ethyl acetate and water. The organic layer was washed with diluted ammonia, water, brine and dried (MgSO₄). The organic extract was removed under reduced pressure and the residue was triturated with a mixture of hexane/ether obtaining a solid, which was separated by filtration and washed with ether. The title compound was obtained as a white solid (20 g, 83%) and used in the next step without further purification.

### Intermediate 90. [4-(benzyloxy)-3-methylphenyl]acetic acid

A solution of Intermediate 89 (16 g, 0.07 mol), morpholine (6.45 mL, 0.07 mol) and sulphur (2.1 g, 0.07 mol) was stirred at reflux for 18 hours. The crude obtained was partitioned between ethyl acetate/water and the organic layer was extracted and washed with water, hydrochloric acid 1 N, brine and dried (MgSO₄). The residue was purified by column chromatography with silica gel, eluting with ethylene chloride/methanol (from 1:2 to 98:2) to give an oil, which was treated with a solution of 250 mL of ethanol, 70 mL of water and 50 g of potassium hydroxide. The reaction mixture was stirred at reflux overnight and the residue obtained was treated with hydrochloric acid and extracted with methylene chloride. The solvent was removed under reduced pressure to give the title compound as a solid (8.1 g, 48%).

### Intermediate 91. 2-[4-(benzyloxy)-3-methylphenyl]acetamide

To a solution of Intermediate 90 (8.1 g, 31.60 mmol) in toluene (60 mL) was added thionyl chloride (3.46 mL, 47.41 mmol). The reaction mixture was stirred at 100°C for 3 hours. The solvent was removed under reduced pressure and the crude obtained was added into a solution of ammonia saturated in methanol. The precipitate was separated by filtration and the title compound was obtained as a solid (5.1 g, 63%) and used in the next step without further purification.

### Intermediate 92. 2-(4-hydroxy-3-methylphenyl)acetamide

To a solution of Intermediate 91 (5.1 g, 19.98 mmol) in acetic acid (100 mL) was added palladium on charcoal (10%, 0.5 g). The reaction mixture was hydrogenated at 2.76 bar for 4 days. The catalyst was filtered through Celite® and the solvent removed under reduced pressure. The residue was triturated with methylene chloride and the solid obtained was separated by filtration to give the title compound as a solid (2.12 g, 64%).

### Intermediate 93. 2-[3-methyl-4-(2-oxo-2-phenylethoxy)phenyl]acetamide

To a solution of Intermediate 92 (2.12 g, 12.83 mmol) in acetonitrile (70 mL) was added 2-bromo-1-phenylethanone (2.81 g, 14.12 mmol) and potassium carbonate (2 g, 14.11 mmol). The reaction mixture was stirred at reflux for 2 hours. The precipitate was filtered and the solvent was removed under reduced pressure and partitioned between ethyl acetate and water. The organic layer was washed with water, brine and dried (MgSO₄). The solvent was removed under reduced pressure and the residue obtained was triturated with hexane and ether to obtain a solid, which was collected by filtration giving the title compound as a solid (3 g, 82%).

### Intermediate 94. 2-[4-(2,2-difluoro-2-phenylethoxy)-3-methylphenyl]acetamide

Obtained from Intermediate 93 (3 g, 10.59 mmol) and DAST (6.94 g, 52.96 mmol) by the same procedure described in Intermediate 45. The crude was purified by column chromatography with silica gel, eluting with methylene chloride/methanol (from 10:1 to 5:1) to give the title compound as an oil (0.43 g, 13%).

### Intermediate 95. {2-[4-(2,2-difluoro-2-phenylethoxy)-3-methylphenyl]ethyl}amine

To a solution of Intermediate 94 (0.43 g, 1.41 mmol) in tetrahydrofuran (15 mL) was added under nitrogen borane-methyl sulphide complex (2.01 mL, 21.17 mmol). The reaction mixture was stirred at reflux for 1 hour. After cooling, hydrochloric acid 1 N (3 mL) was added into the reaction mixture and the solution was basified with sodium hydroxide 5N, concentrated and extracted with ethyl acetate. The crude obtained was purified by column chromatography with silica gel, eluting with methylene chloride/methanol (from 98:1 to 9: 1) to give the title compound as an oil (0.15 g, 37%).

### Intermediate 96. 8-(benzyloxy)-5-[(1R,S)-2-({2-[4-(2,2-difluoro-2-phenylethoxy)-3-methylphenyl]ethyl}amino)-1-hydroxyethyl]quinolin-2(1H)-one

Obtained from Intermediate 95 (0.15 g, 0.51 mmol), 8-(benzyloxy)-5-(dihydroxyacetyl) quinolin-2(1H)-one (0.16 g, 0.52 mmol) and sodium borohydride (0.06 g, 1.59 mmol) by the same procedure described in Intermediate 43 (reaction time: overnight). The crude was purified by column chromatography with silica gel, eluting by methylene chloride/methanol (from 98:2 to 9:1) to give the title compound (0.18 g, 52%).

### EXAMPLE 25. 5-[(1R,S)-2-({2-[4-(2,2-difluoro-2-phenylethoxy)-3-methylphenyl]ethyl}amino)-1-hydroxyethyl]-8-hydroxyquinolin-2(1H)-one

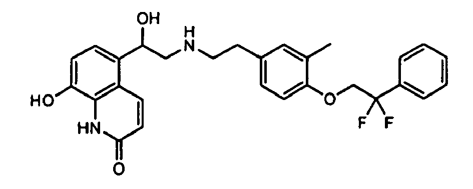

Obtained from Intermediate 96 (0.18 g, 0.3 mmol) and palladium on charcoal (10%, 0.03 g) by the same procedure described in Example 12 (reaction time: over-weekend). The residue was crystallized from ether and the solid obtained was separated by filtration giving the title compound as a solid (0.07 g, 50%).
¹H-NMR (300 MHz, CDCl₃): 2.05 (s, 3H); 2.56 (bs, 2H); 2.71-2.76 (m, 2H); 2.86-2.93 (m, 2H); 4.6 (t, *J*_{H-F}=12.9 Hz, 2H); 5.19 (bs, 1H); 6.58 (d, *J*=9.89 Hz, 1H); 6.95-7.01 (m, 4H); 7.14 (d, *J*=9.89 Hz, 1H); 7.55-7.61 (m, 3H); 7.68-7.71 (m, 3H); 8.23 (d, *J*=9.88 Hz, 1H).
MS (M+): 495.

### Intermediate 97. ethyl (3-fluoro-4-hydroxyphenyl)acetate

A solution of 2-(3-fluoro-4-hydroxyphenyl)acetic acid (5 g, 29.39 mmol) in 60 mL of hydrogen chloride saturated ethanol, was stirred in a sealed tub at 85°C for 3 hours. The solvent was removed under reduced pressure and the title compound was obtained as an oil (5.80 g, 99%) and used in the next step without further purification.

### Intermediate 98. ethyl [3-fluoro-4-(2-oxo-2-phenylethoxy)phenyl]acetate

To a solution of Intermediate 97 (5.8 g, 29.26 mmol) in acetonitrile (55 mL) was added 2-bromo-1-phenylethanone (6.12 g, 30.75 mmol) and potassium carbonate (4.5 g, 32.2 mmol). The reaction mixture was refluxed for 3 hours. Potassium carbonate was filtered off and the solvent was removed under reduced pressure. The crude was partitioned between ethyl acetate and water and the organic layer was washed with water, brine and dried (MgSO₄). The title compound was obtained as an oil (9.2 g, 99%) and used in the next step without further purification.

### Intermediate 99. ethyl [4-(2,2-difluoro-2-phenylethoxy)-3-fluorophenyl]acetate

Obtained from Intermediate 98 (4 g, 12.65 mmol) and DAST (7.46 mL, 56.93 mmol) by the same procedure described in Intermediate 41. The crude was purified by column chromatography with silica gel, eluting with methylene chloride/n-hexane (from 1:3 to 1:1) to give the title compound as an oil (3.5 g, 82%).

### Intermediate 100. [4-(2,2-difluoro-2-phenylethoxy)-3-fluorophenyl]acetic acid

To a solution of Intermediate 99 (3.5 g, 10.35 mmol) in ethanol (20 mL) was added sodium hydroxide (2N, 15.5 mL). The reaction mixture was stirred at room temperature for 2 hours. The solvent was removed under reduced pressure. The crude obtained was treated with hydrochloric acid, extracted with methylene chloride and the solvent was removed under reduced pressure obtaining the title compound as an oil which crystallizes (2.89 g, 90%).

### Intermediate 101. 2-[4-(2,2-difluoro-2-phenylethoxy)-3-fluorophenyl]acetamide

Obtained from Intermediate 100 (2.89 g, 9.31 mmol) and thionyl chloride (1.02 mL, 13.97 mmol) by the same procedure described in Intermediate 91. The title compound was obtained as a solid (2.05 g, 71%) and used in the next step without further purification.

### Intermediate 102. {2-[4-(2,2-difluoro-2-phenylethoxy)-3-fluorophenyl]ethyl}amine

Obtained from Intermediate 101 (1.3 g, 4.2 mmol) and borane-methyl sulphide complex (5.99 mL, 63.08 mmol) by the same procedure described in Intermediate 95. The crude was purified by column chromatography with silica gel, eluting by methylene chloride/methanol (from 100:0 to 90:10) to obtain the title compound as an oil (0.48 g, 39%).

### Intermediate 103. 8-(benzyloxy)-5-[2-({2-[4-(2,2-difluoro-2-phenylethoxy)-3-fluorophenyl]ethyl}amino)-1-hydroxyethyl]quinolin-2(1H)-one

Obtained from Intermediate 102 (0.46 g, 1.56 mmol), 8-(benzyloxy)-5-(dihydroxyacetyl)quinolin-2(1H)-one (0.48 g, 1.56 mmol) and sodium borohydride (0.2 g, 4.76 mmol) by the same procedure described in Intermediate 43 (reaction time: overnight). The crude was purified by column chromatography with silica gel, eluting by methylene chloride/methanol (from 98:2 to 90:10) to give the title compound as an oil (0.53 g, 58%).

### EXAMPLE 26. 5-[(1R,S)-2-({2-[4-(2,2-difluoro-2-phenylethoxy)-3-fluorophenyl]ethyl}amino)-1-hydroxyethyl]-8-hydroxyquinolin-2(1H)-one

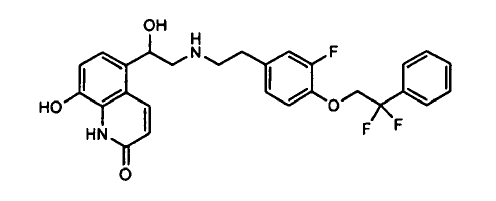

Obtained from Intermediate 103 (0.53 g, 0.9 mmol) and palladium on charcoal (10%, 0.07 g) by the same procedure described in Example 14 (reaction time: 24 hours). The residue obtained was triturated with ether to obtain the title compound as a solid (0.25 g, 78%).
¹H-NMR (300 MHz, CDCl₃): 2.59-2.77 (m, 6H); 4.65 (t, *J*_{H-F}=13.45 Hz, 2H); 4.98-5.02 (m, 1H); 6.49 (d, *J*=9.89 Hz, 1H); 6.89-6.93 (m, 2H); 7.04-7.15 (m, 3H); 7.51-7.56 (m, 2H); 7.62-7.65 (m, 2H); 8.15 (d, *J*=9.88 Hz, 1H).
MS (M+): 499.

### Intermediate 104. 3-(bromoacetyl)benzamide

Obtained from 3-acetylbenzamide (3.7 g, 22.77 mmol) and bromine (1.17 mL, 22.84 mmol) in acetic acid (342 mL) by the procedure described in Bioorg. Med. Chem. C.Y. Watson et al. 6 (1998) 721-734. The title compound was obtained (5.9 g, 83%) and used in the next step without further purification.

### Intermediate 105. 3-{[4-(cyanomethyl)phenoxy]acetyl}benzamide

Obtained from Intermediate 104 (4.47 g, 18.47 mmol), 2-(4-hydroxyphenyl)acetonitrile (2.58 g, 19.39 mmol) and potassium carbonate (2.8 g, 20.31 mmol) by the same procedure described in Intermediate 1. The precipitate obtained was collected by filtration to give the title compound as a solid (3.56 g, 66%).

### Intermediate 106. 3-{2-[4-(cyanomethyl)phenoxy]-1,1-difluoroethyl}benzamide

Obtained from Intermediate 105 (3.26 g, 11.08 mmol) and DAST (4.35 mL, 33.19 mmol) by the same procedure described in Intermediate 41. The crude was purified by column chromatography with silica gel, eluting with n-hexane/ethyl acetate (1:2) to give the title compound as a solid (1.8 g, 51%).

### Intermediate 107. 3-{2-[4-(2-aminoethyl)phenoxy]-1,1-difluoroethyl}benzamide

Obtained from Intermediate 106 (0.6 g, 1.9 mmol), Ni-Raney (0.06 g, 1.02 mmol) and sodium hydroxide (0.17 g, 4.38 mmol) by the same procedure described in Intermediate 21. The title compound was obtained as a white solid (0.41 g, 66%) and used in the next step without further purification.

### Intermediate 108. 3-(2-{4-[2-({(1R,S)-2-[8-(benzyloxy)-2-oxo-1,2-dihydroquinolin-5-yl]-2-hydroxyethyl}amino)ethyl]phenoxy}-1,1-difluoroethyl)benzamide

Obtained from Intermediate 107 (0.212 g, 0.66 mmol), 8-(benzyloxy)-5-(dihydroxyacetyl) quinolin-2(1H)-one (0.48 g, 1.56 mmol) and sodium borohydride (0.2 g, 4.76 mmol) by the same procedure described in Intermediate 43 (reaction time: overnight). The crude obtained was purified by column chromatography with silica gel, eluting with chloroform/methanol/aqueous ammonia (90:10:1) to give the title compound as a yellow foam (0.28 g, 65%).

### EXAMPLE 27. 3-{1,1-difluoro-2-[4-((1R,S)-2-{[2-hydroxy-2-(8-hydroxy-2-oxo-1,2-dihydroquinolin-5-yl)ethyl]amino}ethyl)phenoxy]ethyl}benzamide

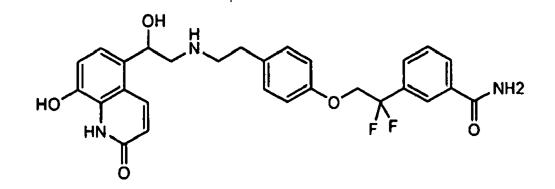

Obtained from Intermediate 108 (0.18 g, 0.3 mmol) and palladium on charcoal (10%, 0.03 g) by the same procedure described in Example 1. The crude obtained was purified by column chromatography with silica gel, eluting with chloroform/methanol/aqueous ammonia (from 85:15:1.5 to 80:20:2) to give the title compound as a yellow solid (0.115 g, 72%).
¹H-NMR (300 MHz, dimethylsulfoxide-D6): 2.74-2.78 (m, 2H); 2.83-2.97 (m, 4H); 4.48 (t, *J*_{H-F}=12.36 Hz, 2H); 5.17-5.21 (m, 1H); 6.63 (d, *J*=9.89 Hz, 1H); 6.81 (d, *J*=8.22 Hz, 2H); 6.94 (d, *J*=8.24 Hz, 1H); 7.07 (d, *J*=8.24 Hz, 2H); 7.16 (d, *J*=8.24 Hz, 1H); 7.6 (d, *J*=7.96 Hz, 1H); 7.8 (d, *J*=7.96 Hz, 1H); 8.01 (d, *J*=7.69 Hz, 1H); 8.15 (s, 1H); 8.33 (d, *J*=9.89 Hz, 1H).
MS (M+): 524.

### Intermediate 109. 2-(3-bromophenoxy)-1-(3-nitrophenyl)ethanone

Obtained from 2-bromo-1-(3-nitrophenyl)ethanone (10 g, 39.75 mmol), 3-bromophenol (7.44 g, 42.14 mmol) and potassium carbonate (6.3 g, 45.06 mmol) by the same procedure described in Intermediate 1. The precipitate obtained was collected by filtration and washed with ether and n-hexane to give the title compound as a brown solid (8.12 g, 61%).

### Intermediate 110. 3-bromophenyl 2,2-difluoro-2-(3-nitrophenyl)ethyl ether

Obtained from Intermediate 109 (6 g, 17.85 mmol) and DAST (7.02 mL, 53.57 mmol) by the same procedure described in Intermediate 2. Purification by column chromatography with silica gel, eluting with n-hexane/ethyl acetate (from 15:1 to 8:1) gave the title compound as a yellow oil (5.67 g, 86%).

### Intermediate 111. 1-{3-[2,2-difluoro-2-(3-nitrophenyl)ethoxy]phenyl}acetone

Obtained from Intermediate 110 (5.67 g, 15.36 mmol), isoprenyl acetate (2.62 mL, 23.58 mmol), tri-n-butyltin methoxide (5.5 mL, 18.51 mmol), palladium (II) acetate (0.18 g, 0.79 mmol) and tri-o-tolylphosphine (0.48 g, 1.54 mmol) by the same procedure described in Intermediate 3. The crude was purified by column chromatography with silica gel, eluting by n-hexane/ethyl acetate (from 10:1 to 1:1) giving the title compound (2.89 g, 54%).

### Intermediate 112. 2-{3-[2,2-difluoro-2-(3-nitrophenyl)ethoxy]benzyl}-2-methyl-1,3-dioxolane

A solution of Intermediate 111 (2.89 g, 8.62 mmol) in toluene (20 mL), ethane-1,2-diol (0.53 mL, 9.55 mmol) and p-toluenesulfonic acid monohydrate (0.1 g, 0.28 mmol) was stirred at reflux for 4 hours and at room temperature for overnight. Anhydrous magnesium sulphate was added into the solution and hexane (50 mL) was added dropwise. The mixture was stirred at room temperature for 20 minutes. Then the mixture was filtered and the filtrate was washed with sodium hydroxide (2x50 mL) and water (2x50 mL). The solvent was removed under reduced pressure to give the title compound as a yellow oil (2.86 g, 80%).

### Intermediate 113. [3-(1,1-difluoro-2-{3-[(2-methyl-1,3-dioxolan-2-yl)methyl]phenoxy}-ethyl)phenyl]amine.

To a solution of Intermediate 112 (1.8 g, 4.49 mmol) in anhydrous methanol (21.6 mL) was added palladium on charcoal (10%, 0.21 g). The reaction was hydrogenated at 30 psi for 2 hours. The catalyst was filtered through Celite® and the solvent removed under reduced pressure to give the title compound as a yellow oil (1.64 g, 92%), which was used in the next step without further purification.

### Intermediate 114. N-[3-(1,1-difluoro-2-{3-[(2-methyl-1,3-dioxolan-2-yl)methyl]-phenoxy}ethyl)phenyl]urea

To a solution of potassium Isocyanate (0.94 g, 11.12 mmol) in water (24.3 mL) was added at 0°C the Intermediate 113 (2.13 g, 5.41 mmol) in a mixture of acetic acid (24.3 mL) and water (12.2 mL). The resulting reaction mixture was stirred at 0°C for 1 hour and at room temperature for 1 hour. The precipitate obtained was collected by filtration and washed with water and hexane to give the title compound as a solid (2.48 g, 93%), which was used in the next step without further purification.

### Intermediate 115. N-(3-{1,1-difluoro-2-[3-(2-oxopropyl)phenoxy]ethyl}phenyl)urea

A solution of Intermediate 114 (1.83 g, 4.04 mmol) in a mixture of acetic acid (17.5 mL) and water (8.6 mL) was stirred at 80°C for 3 hours. Ethyl acetate was added into the solution and extracted. The organic layer was washed with water (2x50 mL), hydrochloric acid 1 N (2x50 mL), a solution of potassium carbonate, brine (50 mL) and dried (MgSO₄), and the solvent was removed under reduced pressure. The residue was purified by column chromatography with silica gel, eluting by n-hexane/ethyl acetate (from 20:1 to 1:5) to give the title compound as a white solid (1.3 g, 93%).

### Intermediate 116. N-(3-{2-[3-((2R,S)-2-aminopropyl)phenoxy]-1,1-difluoroethyl}-phenyl)urea

Obtained from Intermediate 115 (1.3 g, 3.78 mmol), ammonium acetate (2.9 g, 38.1 mmol) and sodium cyanoborohydride (1 g, 15.24 mmol) by the same procedure described in Intermediate 6 (reaction time: 1 hour). The crude was purified by column chromatography with silica gel, eluting with methylene chloride/methanol (from 95:1 to 90:10) giving the title compound as a foam (1.12 g, 77%).

### Intermediate 117. N-[(1R,S)-3-(2-{3-[2-(2R)-1-{[tert-butyl(dimethyl)silyl]oxy}-2-{[8-(benzyloxy)-2-oxo-1,2-dihydroquinolin-5-yl]-2-hydroxyethyl}amino)propyl]phenoxy}-1,1-difluoroethyl)phenyl]urea

Obtained from Intermediate 116 (0.92 g, 2.41 mmol), (*R*)-8-(benzyloxy)-5-(2-bromo-1-(*tert*-butyldimethylsilyloxy)ethyl)quinolin-2(1*H*)-one (1.14 g, 2.34 mmol), sodium hydrogen carbonate (0.23 g, 2.71 mmol) and sodium iodide (0.036 g, 0.24 mmol) by the same procedure described in Intermediate 35 (reaction time: 1 hour). The crude was purified by column chromatography with silica gel, eluting with n-hexane/ethyl acetate (from 1:2 to 9:1) giving the title compound as a solid (1.06 g, 39%).

### Intermediate 118. N-[(1R,S)-3-(2-{3-[2-({(2R)-2-[8-(benzyloxy)-2-oxo-1,2-dihydroquinolin-5-yl]-2-hydroxyethyl}amino)propyl]phenoxy}-1,1-difluoroethyl)phenyl]urea

Obtained from Intermediate 117 (0.333 g, 0.42 mmol) and tetrabutylammonium fluoride trihydrate (0.21 g, 0.68 mmol) by the same procedure described in Intermediate 8. The crude was purified by column chromatography with silica, gel eluting by chloroform/methanol (from 50:1 to 9:1) to give the title compound as a foam (0.25 g, 94%).

### EXAMPLE 28. N-((1R,S)-3-{1,1-difluoro-2-[3-(2-{[(2R)-2-hydroxy-2-(8-hydroxy-2-oxo-1,2-dihydroquinolin-5-yl)ethyl]amino}propyl)phenoxy]ethyl}phenyl)urea

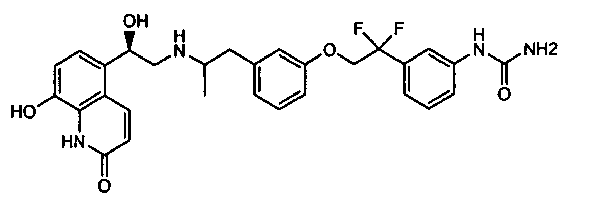

Obtained from Intermediate 118 (0.25 g, 0.39 mmol) and palladium on charcoal (10%, 0.022 g) by the same procedure described in Example 12 (reaction time: overnight). The crude was purified by column chromatography with silica gel, eluting with chloroform/methanol/aqueous ammonia (from 90:5:0.5 to 80:20:2) to give the title compound as a yellow foam (0.18 g, 86%).
¹H-NMR (300 MHz, dimethylsulfoxide-D6): 0.89 (d, *J*=6.04 Hz, 3H); 2.45 (q, *J*=6.04 Hz, 1H); 2.65-2.86 (m, 2H); 4.52 (t, *J*_{H-F}= 13.46 Hz, 2H); 4.97 (bs, 1H); 5.94 (s, 2H); 6.5 (d, *J*=9.89 Hz, 1H); 6.76-6.82 (m, 3H); 6.89 (d, *J*=8.24 Hz, 1H); 7.04 (d, *J*=8.24 Hz, 1H); 7.14-7.20 (m, 2H); 7.35 (t, *J*=7.69 Hz, 1H); 7.5 (d, *J*=8.24 Hz, 1H); 7.75 (s, 1H); 8.15 (d, *J*=9.88 Hz, 1H); 8.78 (s, 1H).
MS (M+): 553.

### Intermediate 119. 2-bromo-1-(3-fluorophenyl)ethanone

To a solution of 1-(3-fluorophenyl)ethanone (5 g, 36.2 mmol) in chloroform (34 mL) was added dropwise a solution of bromine (1.77 mL, 34.56 mmol) in chloroform (89 mL). The reaction mixture was stirred at room temperature overnight. The solvent was removed under reduced pressure and the residue was dissolved in methylene chloride (90 mL) and neutralized with sodium hydrogen carbonate (30 mL). The mixture was stirred for some minutes. The organic layer was extracted and washed with water, brine and dried (MgSO₄). The solvent was removed under reduced pressure to give the title compound (5.99 g, 79 %).

### Intermediate 120. {4-[2-(3-fluorophenyl)-2-oxoethoxy]phenyl}acetonitrile

Obtained from Intermediate 119 (5.99 g, 27.6 mmol), 2-(4-hydroxyphenyl)acetonitrile (3.86 g, 28.99 mmol) and potassium carbonate (4.2 g, 30.39 mmol) by the same procedure described in Intermediate 1. The crude was crystallized using a mixture of methylene chloride/ether/n-hexane to give the title compound as a solid (2.7 g, 36%).

### Intermediate 121. {4-[2,2-difluoro-2-(3-fluorophenyl)ethoxy]phenyl}acetonitrile

Obtained from Intermediate 120 (2.7 g, 10.03 mmol) and DAST (4 mL, 30.52 mmol) by the same procedure described in Intermediate 2. The crude was purified by column chromatography with silica gel, eluting with n-hexane/methylene chloride (from 1:3 to 1:9) to give the title compound (1.09 g, 37%).

### Intermediate 122. (2-{4-[2,2-difluoro-2-(3-fluorophenyl)ethoxy]phenyl}ethyl)amine

Obtained from Intermediate 121 (1.09 g, 3.74 mmol), sodium hydroxide (0.35 g, 8.75 mmol) in ethanol (10 mL) and Raney Nickel® (1 g of a 50% slurry in water) by the same procedure described in Intermediate 21. The crude was purified by column chromatography with silica gel, eluting with chloroform/methanol (from 50:1 to 10: 1) to give the title compound as a colourless oil (0.75 g, 68%).

### Intermediate 123. 8-(benzyloxy)-5-{(1R,S)-2-[(2-{4-[2,2-difluoro-2-(3-fluorophenyl)ethoxy]phenyl}ethyl)amino]-1-hydroxyethyl}quinolin-2(1H)-one

Obtained from Intermediate 122 (0.75 mg, 2.55 mmol), 8-(benzyloxy)-5-(dihydroxyacetyl)quinolin-2(1H)-one (0.83 g, 2.55 mmol) and sodium borohydride (0.25 g, 6.56 mmol) by the same procedure described in Intermediate 43 (reaction time: overnight). The crude was purified by column chromatography with silica gel, eluting with methylene chloride/methanol (from 75:1 to 9:1) to give the title compound as a foam (1.07 g, 69%).

### EXAMPLE 29. 5-{(1R,S)-2-[(2-{4-[2,2-difluoro-2-(3-fluorophenyl)ethoxy]phenyl}-ethyl)amino]-1-hydroxyethyl}-8-hydroxyquinolin-2(1H)-one

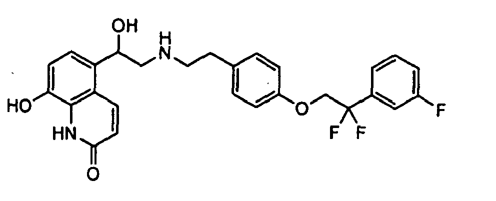

Obtained from Intermediate 123 (0.86 g, 1.46 mmol) and palladium on charcoal (10%, 0.08 g) by the same procedure described in Intermediate 10 (reaction time: 24 hours). The crude was purified by column chromatography with silica gel, eluting with chloroform/methanol (from 75:1 to 15:1) to give the title compound as a white solid (0.64 g, 88%).
¹H-NMR (300 MHz, dimethylsulfoxide-D6): 2.60-2.65 (m, 2H); 2.69-2.76 (m, 4H); 4.6 (t, *J*_{H-F}=13.18 Hz, 2H); 5.02 (bs, 1H); 6.5 (d, *J*=9.89 Hz, 1H); 6.89 (s, 1H); 6.89 (t, *J*=8.24 Hz, 2H); 7.06 (d, *J*=8.51 Hz, 1H); 7.1 (d, *J*=8.24 Hz, 2H); 7.42 (t, *J*=7.69 Hz, 2H); 7.51 (d, *J*=7.69 Hz, 2H); 7.56-7.64 (m, 1H); 8.16 (d, *J*=9.89 Hz, 1H).
MS (M+): 499.

### Intermediate 124.1-(3-mercaptophenyl)ethanone

To a solution of 1-(3-aminophenyl)ethanone (5 g, 36.99 mmol) in hydrochloric acid (6.5 mL) diluted with 12 g of ice was added a solution of sodium nitrite (2.4 g, 34.78 mmol) in water (3 mL). The reaction mixture was stirred at room temperature for 10 minutes. A solution of potassium ethyl xanthate (5.9 g, 36.81 mmol) in water (8 mL) was slowly added at 0°C into the reaction mixture, then the solution was temperate and stirred at 50°C for 2 hours. The crude was extracted with ether and the organic layer was washed with diluted sodium hydroxide, brine and dried (MgSO₄). The solvent was removed under reduced pressure to give an oil, which was treated with a solution of potassium hydroxide (10g) in ethanol (20 mL). The mixture was stirred at reflux for 1.5 hours. The solvent was removed under reduced pressure and the crude was partitioned between water and ether. The organic layer was washed with water, brine and dried (MgSO₄). The solvent was removed under reduced pressure to give the title compound as a brown solid (2.58 g, 54%) and used in the next step without further purification.

### Intermediate 125.1-(3-(cyclopentylthio)phenyl)ethanone

To a solution of sodium hydroxide (0.12 g, 3 mmol) in water (2.5 mL) was slowly added a solution of Intermediate 124 (0.5 g, 3.28 mmol) in dioxane (6 mL). A solution of bromocyclopentane (0.416 mL, 3.88 mmol) in dioxane (20 mL) was added dropwise and the reaction mixture was stirred at room temperature overnight. Ethyl acetate was poured into the reaction mixture and the organic layer was extracted and washed with water, brine and dried (MgSO₄). The solvent was removed under reduced pressure to give the title compound as an oil (0.23 g, 36%), which was used in the next step without further purification.

### Intermediate 126. 2-bromo-1-[3-(cyclopentylthio)phenyl]ethanone

To a solution of Intermediate 125(1.2 g, 5.78 mmol) in chloroform (10 mL) was added under nitrogen during 30 minutes a solution of bromine (0.29 mL, 5.78 mmol) in chloroform (4.3 mL). The reaction mixture was stirred at room temperature for 10 minutes. The solvent was removed under reduced pressure and the residue obtained was purified by column chromatography with silica gel, eluting with methylene chloride/n-hexane (1:1.5) giving the title compound (0.92 g, 53%).

### Intermediate 127. (4-{2-[3-(cyclopentylthio)phenyl]-2-oxoethoxy}phenyl)acetonitrile

Obtained from Intermediate 126 (0.92 g, 3.09 mmol), 2-(4-hydroxyphenyl)acetonitrile (0.43 g, 3.24 mmol) and potassium carbonate (0.47 g, 3.4 mmol) by the same procedure described in Intermediate 1 (reaction time: 3 hours). The title compound was obtained as a brown oil (1.03 g, 93%) and used in the next step without further purification.

### Intermediate 128. (4-{2-[3-(cyctopentylthio)phenyl]-2,2-difluoroethoxy}phenyl)-acetonitrile

Obtained from Intermediate 127 (1.04 g, 2.96 mmol) and DAST (1.16 mL, 8.87 mmol) by the same procedure described in Intermediate 2. The crude was purified by column chromatography with silica gel, eluting with n-hexane/ethyl acetate (1:2) to give the title compound (0.66 g, 59%).

### Intermediate 129. [2-(4-{2-[3-(cyclopentylthio)phenyl]-2,2-difluoroethoxy}phenyl)-ethyl]amine

To a solution of lithium aluminium hydride (0.15 g, 4.1 mmol) in anhydrous ether (4 mL) was slowly added at 0°C a solution of Intermediate 128 (0.41 g, 1.10 mmol) in anhydrous ether (3 mL). The reaction mixture was stirred at room temperature for 3 hours. The crude reaction was diluted at 0°C with water (5 mL), sodium hydroxide (4N, 5 mL) and water again (10 mL). The catalyst was filtered through Celite® and the solvent removed under reduced pressure. The title compound was obtained as an orange oil (0.213 g, 51%), which was used in the next step without further purification.

### Intermediate 130. 5-((1R,S)-2-{[2-(4-{2-[3-(cyclopentylthio)phenyl]-2,2-difluoro-ethoxy}phenyl)ethyl]amino}-1-hydroxyethyl)-8-[(4-methoxybenzyl)oxy]quinolin-2(1H)-one

Obtained from Intermediate 125 (0.34 g, 0.89 mmol), Intermediate 33 (0.32 g, 0.89 mmol) and sodium borohydride (0.13 g, 3.57 mmol) by the same procedure described in Intermediate 43. The crude was purified by column chromatography with silica gel eluting with chloroform/methanol (93:3) to give the title compound (0.23 g, 37%).

### EXAMPLE 30. 5-((1R,S)-2-{[2-(4-{2-[3-(cyclopentylthio)phenyl]-2,2-difluoroethoxy}-phenyl)ethyl]amino}-1-hydroxyethyl)-8-hydroxyquinolin-2(1H)-one

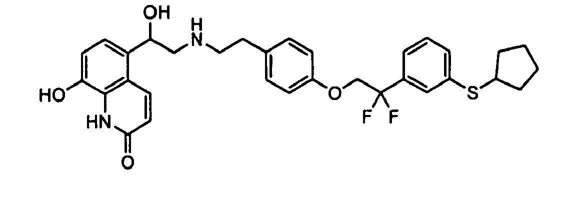

To a solution of Intermediate 130 (0.18 g, 0.26 mmol) in methylene chloride (3.5 mL) was added trifluoroacetic acid (0.2 mL, 2.6 mmol). The resulting mixture was stirred at room temperature for 3 hours before the solvent was removed under reduced pressure. The crude was dissolved in an 80:20:2 mixture of methylene chloride/methanol/aqueous ammonia (10 mL) and the solvents were removed under reduced pressure. The crude oil obtained was purified by column chromatography with silica gel, eluting with methylene chloride/methanol/aqueous ammonia (from 90:10:1 to 80:20:2) to give the title compound (0.12 g, 79%) as a yellow solid.
¹H-NMR (300 MHz, dimethylsulfoxide-D⁶): 1.44-1.73 (m, 6H); 2.00-2.09 (m, 2H); 2.60-2.76 (m, 6H); 3.74-3.82 (m, 1H); 4.59 (t, *J*_{H-F}=13.46 Hz, 2H); 5.01 (bs, 1H); 6.5 (d, *J*=9.89 Hz, 1H); 6.86-6.92 (m, 3H); 7.04-7.11 (m, 3H); 7.45-7.49 (m, 3H); 7.53 (s, 1H); 8.16 (d, *J*=9.89 Hz, 1H).
MS (M+): 581.

### Intermediate 131. tert-butyl [2-(4-hydroxyphenyl)ethyl]carbamate

To a solution of 4-(2-aminoethyl)phenol (9 g, 0.07 mol) in a mixture of dioxane (60 mL), water (60 mL) and tetrahydrofuran (120 mL) was added at 0°C potassium carbonate (9 g, 0.07 mol) and di-tert-butyl dicarbonate (14.3 g, 0.07 mol) in dioxane (35 mL). The reaction mixture was stirred at room temperature over-weekend. The organic layer was extracted and washed with water, sodium hydrogen carbonate (4%), brine and dried (MgSO₄). The solvent was removed under reduced pressure to give the title compound as an oil (15.45 g, 99%), which was used in the next step without further purification.

### Intermediate 132. tert-butyl [2-(4-{2-[3-(cyclopentylthio)phenyl]-2-oxoethoxy}-phenyl)ethyl]carbamate

Obtained from Intermediate 131 (5.7 g, 24.02 mmol), Intermediate 126 (7.19 g, 24.03 mmol) and potassium carbonate (3.3 g, 24.02 mmol) by the same procedure described in Intermediate 1 (reaction time: over-weekend). The title compound was obtained as an oil (10.5 g, 95%) and used in the next step without further purification.

### Intermediate 133. tert-butyl [2-(4-{2-[3-(cyclopentylsulfonyl)phenyl]-2-oxoethoxy}-phenyl)ethyl]carbamate

To a solution of Intermediate 132 (3.7 g, 8.12 mmol) in methylene chloride (70 mL) was slowly added at -78°C 3-chloroperoxybenzoic acid (4.5 g, 26.37 mmol). The reaction mixture was stirred at -78°C for 20 minutes and at room temperature overnight. The mixture was diluted with methylene chloride and washed with sodium bisulphite (10%), sodium bicarbonate (4%), brine and dried (MgSO₄). The solvent was removed under reduced pressure and the crude was purified by column chromatography with silica gel, eluting with n-hexane/ethyl acetate (from 4:1 to 3:1). The title compound was obtained as a solid (2.27 g, 59%).

### Intermediate 134. tert-butyl [2-(4-{2-[3-(cyclopenlylsulfonyl)phenyl]-2,2-difluoro-ethoxy}phenyl)ethyl]carbamate

Obtained from Intermediate 133 (2.27 g, 4.66 mmol) and DAST (3.05 mL, 23.27 mmol) by the same procedure described in Intermediate 45. The crude was purified by column chromatography with silica gel, eluting by n-hexane/ethyl acetate (from 4:1 to 3:1) to give the title compound as an oil (0.73 g, 31%).

### Intermediate 135. [2-(4-{2-[3-(cyclopentylsulfonyl)phenyl]-2,2-difluoroethoxy}-phenyl)ethyl]amine

A solution of Intermediate 133 (0.73 g, 1.43 mmol) in hydrogen chloride 1.25M in ethanol (10 mL) was stirred at room temperature for 5 hours. The solvent was removed under reduced pressure and the crude was crystallized from ether and some drops of methylene chloride giving the title compound as a solid (0.46 g, 77%).

### Intermediate 136. 8-(benzyloxy)-5-((1R,S)-2-{[2-(4-{2-[3-(cyclopentylsulfonyl)phenyl]-2,2-difluoroethoxy}phenyl)ethyl]amino}-1-hydroxyethyl)quinolin-2(1H)-one

Obtained from Intermediate 135 (0.61 g, 1.49 mmol), 8-(benzyloxy)-5-(dihydroxyacetyl)quinolin-2(1H)-one (0.46 g, 1.5 mmol) and sodium borohydride (0.17 g, 4.49 mmol) by the same procedure described in Intermediate 43 (reaction time: overnight). The crude obtained was purified by column chromatography with silica gel, eluting by methylene chloride/methanol (from 98:2 to 9:1) giving the title compound (0.095 g, 9%).

### EXAMPLE 31. 5-((1R,S)-(2-{[2-(4-{2-[3-(cyclopentylsulfonyl)phenyl]-2,2-difluoroethoxy}phenyl)ethyl]amino}-1-hydroxyethyl)-8-hydroxyquinolin-2(H)-one

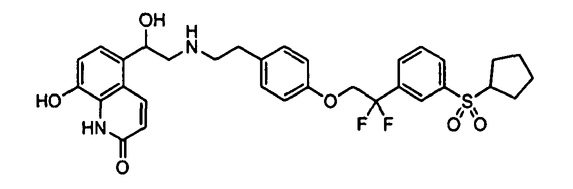

Obtained from Intermediate 136 (0.095 g, 0.14 mmol) and palladium on charcoal (10%, 0.01 g) by the same procedure described in Intermediate 10 (reaction time: 6 hours). The crude obtained was purified by column chromatography with silica gel, eluting by methylene chloride/methanol (from 98:2 to 9:1) to give the title compound as a solid (0.02 g, 35%).
¹H-NMR (300 MHz, CD₃OD): 1.60-1.99 (m, 8H); 2.96 (bs, 2H); 3.21 (bs, 4H); 3.67-3.74 (m, 1H); 4.55 (t, *J*_{H-F}=12.08 Hz, 2H); 5.38 (bs, 1H); 6.69 (d, *J*=9.61 Hz, 1H); 6.87-6.91 (m, 1H); 7.00-7.04 (m, 1H); 7.17-7.20 (m, 2H); 7.28 (d, *J*=7.97 Hz, 1H); 7.79 (t, *J*=7.69 Hz, 1H); 7.99 (d, *J*=7.69 Hz; 1H); 8.06 (d, *J*=7.69 Hz, 1H); 8.12 (bs, 1H); 8.37 (d, *J*=9.62 Hz, 1H).
MS (M+): 613.

### Intermediate 137.1-[4-(2,2-difluoro-2-phenylethoxy)phenyl]-2-methylpropan-2-ol

To a solution of Intermediate 3 (1.5 g, 5.17 mmol) in anhydrous ether (2mL) was added dropwise under argon at 5°C methylmagnesium bromide (3.79 mL, 11.37 mmol). The reaction mixture was stirred at 5°C for 5 minutes and at room temperature for 3 hours. The solvent was removed under reduced pressure and the residue obtained was purified by column chromatography with silica gel, eluting by n-hexane/ethyl acetate (4:1) giving the title compound as a yellow oil (1.04 g, 66%).

### Intermediate 138. 2-chloro-N-{2-[4-(2,2-difluoro-2-phenylethoxy)phenyl]-1,1-dimethylethyl}acetamide

To a solution of Intermediate 137 (0.83 g, 2.71 mmol) in acetic glacial acid (1.66 mL) was added chloroacetonitrile (0.343 mL, 5.42 mmol). The mixture was cooled at 0°C and concentrated sulphuric acid (1.66 mL) was slowly added maintaining the temperature under 10 °C. The reaction mixture was poured into a mixture of water and ice and basified with potassium carbonate. The organics were extracted with ethyl acetate and the organic layer was washed with water and dried (MgSO₄). The solvent was removed under reduced pressure and the crude was purified by column chromatography with silica gel, eluting by methylene chloride to give the title compound (0.45 g, 44%).

### Intermediate 139. {2-[4-(2,2-difluoro-2-phenylethoxy)phenyl]-1,1-dimethylethyl}-amine.

To a solution of Intermediate 137 (0.28 g, 0.75 mmol) in absolute ethanol (1.79 mL) was added thiourea (0.068 g, 0.90 mmol) and glacial acetic acid (0.36 mL). The reaction mixture was stirred at reflux for 2 hours. The precipitate was separated by filtration and the solvent was removed under reduced pressure. The residue obtained was dissolved with methylene chloride and basified with sodium hydroxide 1 M. The organic layer was extracted with methylene chloride, washed with brine and dried (MgSO₄). The solvent was removed under reduced pressure to give the title compound as a colourless oil (0.21 g, 92%), which was used in the next step without further purification.

### Intermediate 140. 8-(benzyloxy)-5-[(1R,S)2-({2-[4-(2,2-difluoro-2-phenylethoxy)-phenyl]-1,1-dimethylethyl}amino)-1-hydroxyethyl]quinolin-2(1H)-one

Obtained from Intermediate 139 (0.36 g, 1.19 mmol), 8-(benzyloxy)-5-(dihydroxyacetyl)quinolin-2(1H)-one (0.32 g, 1.19 mmol) and sodium borohydride (0.18 g, 4.76 mmol) by the same procedure described in Intermediate 43. The crude obtained was purified by column chromatography with silica gel, eluting by methylene chloride/methanol (from 98:2 to 9:1) to give the title compound (0.5 g, 69%).

### EXAMPLE 32. 5-[(1R,S)-2-({2-[4-(2,2-difluoro-2-phenylethoxy)phenyl]-1,1-dimethyl-ethyl}amino)-1-hydroxyethyl]-8-hydroxyquinolin-2(1H)-one

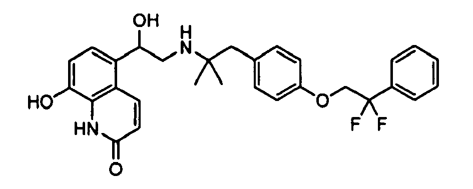

Obtained from Intermediate 140 (0.24 g, 0.4 mmol) and palladium on charcoal (10%, 0.04 g) by the same procedure described in Example 1 (reaction time: 24 hours). The crude was purified by column chromatography with silica gel, eluting by methylene chloride/methanol (from 95:1 to 8:1) to give the title compound (0.17 g, 82%).
¹H-NMR (300 MHz, CD₃OD) 1.06 (s, 6H); 2.66 (bs, 2H); 2.83-3.00 (m, 2H); 4.43 (t, *J*_{H}-_{F}=12.36 Hz, 2H); 5.13-5.18 (m, 1H); 6.66 (d, *J*=9.89 Hz, 1H); 6.78 (d, *J*=7.97 Hz, 2H); 6.96 (d, *J*=8.24 Hz, 1H); 7.02 (d, *J*=8.24 Hz, 2H); 7.21 (d, *J*=7.96 Hz, 1H); 7.48 (bs, 3H); 7.61 (bs, 2H); 8.39 (d, *J*=9.89 Hz, 1H).
MS (M+): 509.

### Pharmaceutical Compositions

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.
Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred.

Each capsule or cartridge may generally contain between 2µg and 150 µg of each therapeutically active ingredient. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring slides (e. g.US 5201308 and WO 97/00703) or measuring plungers with a local or circumferential recess to displace a certain volume of powder from the container to a delivery chamber or an air conduit e. g. EP 0505321, WO 92/04068 and WO 92/04928.

Reproducible dose measuring is one of the major concerns for multi dose inhaler devices.
The powder formulation has to exhibit good and stable flow properties, because filling of the dose measuring cups or cavities is mostly under the influence of the force of gravity.
For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi- dose devices, powder adhesion onto the inner walls of the air conduits and the de- agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with.
Such atomisers are described, for example, in PCT Patent Application No. WO 91/14468 and International Patent Application No. WO 97/12687, reference here being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants eg oleic acid or lecithin and cosolvens eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µ, preferably 2-5µ. Particles having a size above 20µ are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving a high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.
Pressurized aerosol compositions will generally be filled into canisters fitted with a valve, especially a metering valve. Canisters may optionally be coated with a plastics material e. g. a fluorocarbon polymer as described in WO96/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit contains suitably from 1 µg to 100 µg, and preferably from 5 µg to 50 µg of a β2-agonist according to the invention.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day.

The compositions of the invention can optionally comprise one or more additional active substances which are known to be useful in the treatment of respiratory disorders, such as PDE4 inhibitors, corticosteroids or glucocorticoids and/or anticholinergics.

Examples of suitable PDE4 inhibitors that can be combined with β2-agonists are denbufylline, rolipram, cipamfylline, arofylline, filaminast, piclamilast, mesopram, drotaverine hydrochloride, lirimilast, roflumilast, cilomilast, 6-[2-(3,4-Diethoxyphenyl)thiazol-4-yl]pyridine-2-carboxylic acid, (R)-(+)-4-[2-(3-Cyclopentyloxy-4-methoxyphenyl)-2-phenylethyl]pyridine, N-(3,5-Dichloro-4-pyridinyl)-2-[1-(4-fluorobenzyl)-5-hydroxy-1H-indol-3-yl]-2-oxoacetamide, 9-(2-Fluorobenzyl)-N6-methyl-2-(trifluoromethyl)adenine, N-(3,5-Dichloro-4-pyridinyl)-8-methoxyquinoline-5-carboxamide, N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride, 4-[6,7-Diethoxy-2,3-bis(hydroxymethyl)naphthalen-1-yl]-1-(2-methoxyethyl)pyridin-2(1H)-one, 2-carbomethoxy-4-cyano-4-(3-cyclopropylmethoxy-4-difluroromethoxyphenyl)cyclohexan1-one, *cis* [4-cyano-4-(3-cyclopropylmethoxy-4-difluoromethoxyphenyl)cyclohexan-1-ol, ONO-6126 (Eur Respir J 2003, 22(Suppl. 45): Abst 2557) and the compounds claimed in the PCT patent applications number WO03/097613 and PCT/EP03/14722 and in the Spanish patent application number P200302613.

Examples of suitable corticosteroids and glucocorticoids that can be combined with β2-agonists are prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, deprodone propionate, fluticasone propionate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable M3 antagonists (anticholinergics) that can be combined with β2-agonists are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts, 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

The combinations of the invention may be used in the treatment of respiratory diseases, wherein the use of bronchodilating agents is expected to have a beneficial effect, for example asthma, acute or chronic bronchitis, emphysema, or Chronic Obstructive Pulmonary Disease (COPD).

The active compounds in the combination, i.e. the β2-agonist of the invention and the PDE4 inhibitors, corticosteroids or glucocorticoids and/or anticholinergics may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers, however, any other form or parenteral or oral application is possible. Here, the application of inhaled compositions embodies the preferred application form, especially in the therapy of obstructive lung diseases or for the treatment of asthma.

Additional suitable carriers for formulations of the active compounds of the present invention can be found in Remington: The Science and Practice of Pharmacy, 20th Edition, Lippincott Williams & Wilkins, Philadelphia, Pa., 2000. The following nonlimiting examples illustrate representative pharmaceutical compositions of the invention.

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10 g |
| 1,1,1,2,3,3,3-heptafluoro-n-propane | q.s. to 200 ml |

### Biological Assays

The compounds of this invention, and their pharmaceutically-acceptable salts, exhibit biological activity and are useful for medical treatment. The ability of a compound to bind to the β adrenergic receptors, as well as its selectivity, agonist potency, and intrinsic activity can be demonstrated using Tests A to E below, or can be demonstrated using other tests that are known in the art.

### TEST A

### Human Adrenergic β1 and β2 Receptor Binding Assays

The study of binding to human adrenergic β1 and β2 receptors was performed using commercial membranes prepared from Sf9 cells where they are overexpressed (Perkin Elmer).

The membrane suspensions (16 µg/well for β1 and 5µg/well for β2) in assay buffer, 75mM Tris/HCl with 12.5mM MgCl₂ and 2mM EDTA pH=7.4, were incubated with 0.14nM ³H-CGP12177 (Amersham) and different concentrations of the test compounds, in a final volume of 250 µl, in GFC Multiscreen 96 well plates (Millipore) pretreated with + 0.3% PEI. Non specific binding was measured in the presence of 1µM propanolol. Incubation was for 60 minutes at room temperature and with gentle shaking. The binding reactions were terminated by filtration and washing with 2.5 volumes of Tris/HCl 50mM pH=7.4. The affinity of each test compound to the receptor was determined by using at least six different concentrations ran in duplicate. IC₅₀ values were obtained by non-linear regression using SAS.

Selected compounds of this invention were found to have IC₅₀ values less than 13 nM for β2 receptor and more than 68 nM for β1 receptor, with β1/β2 ratios from 30 to 200.

### TEST B

### Human Adrenergic β3 Receptor Binding Assay

Membranes prepared from Human SK-N-MC neurotumor cells from the American Type Culture Collection were used as the source of β3 receptor. The cells were grown, and the membranes prepared following the methods described in P.K. Curran and P.H. Fishman, Cell. Signal, 1996, 8 (5), 355-364.

The assay procedure as detailed in the mentioned publication can be summarized as follows: the SK-N-MC cell line expresses both β1 and β3 receptor and for that reason, for selective binding to β3, membranes were incubated with 1nM ¹²⁵I-CYP ((-)-3-[¹²⁵I]lodocyanopindolol (Amersham)) and 0.3µM CGP20712A (a β1 antagonist) in 50mM HEPES, 4mM MgCl₂ and 0.4% bovine serum albumin, pH=7.5 (assay buffer), and different concentrations of the test compounds. The final volume of the assay was 250µl Non specific binding was defined by 100µM alprenolol. The samples were incubated 90 minutes at 30°C with shaking.

The binding reactions were terminated by filtration through Whatman GF/C membranes, prewet in assay buffer at 4°C, using a BRANDEL M-24 harvester. The filters were washed three times with 4 ml each of 50 mM Tris/HCl and 4mM MgCl₂ pH 7.4, and the radioactivity, retained in the filters, measured.

The affinity of each test compound to the receptor was determined by using at least eight different concentrations ran in duplicate. IC₅₀ values were obtained by non-linear regression using SAS. Exemplified compounds of this invention were found to have IC₅₀ values more than 1200 nM for β3 receptor.

### TEST C

### Determination of agonist activity and offset of action on isolated guinea-pig tracheal rings (resting tone)

### Test compounds and products

The test compounds were dissolved in distilled water. Some of them needed to be dissolved using 10% polyethylene glycol 300 and a few drops of HCl 0.1 N. Isoprenaline hemisulfate (Sigma I 5752) and dissolved in distilled water. Stock solutions were then diluted in Krebs Henseleit solution (NaCl 118mM, KCI 4.7mM, CaCl₂ 2.52mM, MgSO₄ 1.66 mM, NaHCO₃ 24.9mM, KH₂PO₄ 1.18mM, glucose 5.55 mM, sodium pyruvate 2mM) to prepare different concentration ranges per each compound.

### Experimental procedure

The activity of compounds in tracheal ring was assessed according a previously described procedure (Cortijo et al., Eur J Pharmacol. 1991, 198, 171-176). Briefly, adult, male guinea pigs (400-500g) were sacrificed by a blow to the head with immediate exsanguinations (abdominal aorta). Tracheas were excised and placed into Krebs solution in a Petri dish. The adherent connective tissue was dissected away and the lumen gently flushed with Krebs solution. Each trachea was dissected into single rings. First, cotton thread was attached to the cartilage at both sides of the smooth muscle. The rings were opened by cutting through the cartilage on the side opposite to the smooth muscle band. Then, one end of the ring was attached to the strain gauge and the other end was attached to the organ-bath under a resting tension of 1g and changes in tension of the rings were measured using an isometric transducer. The bath contained Krebs solution gassed with 5% CO₂ in oxygen at 37°C. Tissues were then left for one hour to stabilize.

At the beginning of the experience, isoprenaline was administered at a concentration of 0.1 µM to test ring relaxation. Rings were then washed twice with Krebs solution and left to recover for 15-30 min. For each compound, a range of increasing and accumulative concentrations (0.01 nM to 0.1 µM) was administered with a maximum waiting time of 30 min between each administration. After the maximum concentration (achievement of complete relaxation), ring preparations were washed every 15 min during 1 hour. At the end of the experiment, 0.1 µM of isoprenaline was administered to each preparation to produce maximum relaxation back.

### Determination of agonist activity and offset of action

Agonist activity was determined by assaying accumulative increasing concentrations of test compounds prepared in the Krebs solution. The magnitude of each response was measured and expressed as a percentage *versus* the maximum relaxation induced by isoprenaline. Potency values for the test compounds were expressed in absolute terms (concentration required to induce a 50% relaxation, EC₅₀).
The time to 50% offset of action is defined as the time from the end of test compounds administration to attainment of 50% recovery. Recovery time was expressed as the percentage of recovery (loss of relaxation) reached 1h after test compounds administration. Selected compounds of this invention showed EC₅₀ values less than 5 nM with less than 1 % recovery at 60 min.

### TEST D

### Acetylcholine-induced bronchoconstriction in guinea pig

### Test compounds and products

The test compounds were dissolved in distilled water. Some of them need to be dissolved using a maximum of 10% polyethylene glycol 300. Acetylcholine HCl was supplied by Sigma (code A 6625) and dissolved in saline solution.

### Experimental procedure

Male guinea-pigs (450-600g) were supplied by Harlan (Netherlands), and maintained at a constant temperature of 22±2 °C, humidity 40-70% with 10 cycles of room air per hour. They were illuminated with artificial light in 12 hour cycles (from 7h am to 7h pm). A minimum of 5 days acclimatization period was left before animals were dosed with test compounds. The animals were fasted 18 hours before the experiment with water *ad libitum.*

Guinea pigs were exposed to an aerosol of a test compound or vehicle. These aerosols were generated from aqueous solutions using a Devilbiss nebuliser (Model Ultraneb 2000, Somerset, PA, SA). A mixture of gases (CO₂=5%, O₂=21%, N₂=74%) was flown through the nebuliser at 3 Uminute. This nebuliser was connected to a methacrylate box (17x17x25 cm) where the animals were placed one per session. Every guinea pig remained in the box for a total of 10 minutes. Aerosols were generated at 0 and 5 minutes during 60 seconds each one (approximately 5 mL of solution was nebulised).

Aerosol concentrations between 0.1 and 300 µg/ml of the compounds were administered. The bronchoprotective effects of test compounds were evaluated one hour or twenty four hours post-dose with a Mumed PR 800 system.

### Determination of bronchoprotective effect and calculations

The guinea pigs were anesthetized with an intramuscular injection of ketamine (43.75 mg/kg), xylazine (83.5 mg/kg), and acepromazine (1.05 mg/kg) at a volume of 1 ml/kg. After the surgical site was shaved, a 2-3 cm midline incision of the neck was made. The jugular vein was isolated and cannulated with a polyethylene catheter (Portex Ld.) to allow an intravenous bolus of acetylcoline (10 and 30 µg/kg iv) at 4-min intervals. The carotid artery was cannulated and the blood pressure was measured by a Bentley Tracer transducer. The trachea was dissected and cannulated with a teflon tube and connected at a pneumotachograph Fleisch for measuring the airflow. Animal was ventilated using an Ugo Basile pump, with a volume of 10 ml/kg at a rate of 60 breaths/min. The transpulmonary pressure was measured with an esophageal cannula (Venocath-14, Venisystems) connected to Celesco transducer. Once the cannulations were completed a Mumed pulmonary measurement computer program enabled the collection of pulmonary values. The baseline values were within the range of 0.3-0.9 mL/cm H₂O for compliance and within the range of 0.1-0.199 cm H₂O/mL per second for lung resistance (R_{L}).

The bronchocoprotective effect of inhaled compounds was determined with the concentration of the test compound causing a 50 % of inhibition of bronchoconstriction (EC₅₀) induced by acetylcholine at 30 µg/kg iv

### Determination of duration of action

Selected compounds of this invention show long duration of action.

## Claims

1. A compound of formula (I): 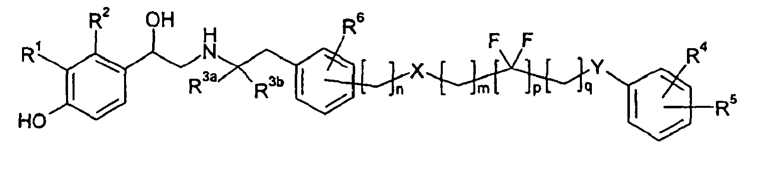 wherein:
• R¹ is a group selected from -CH₂OH,-NH(CO)H and
• R² is a hydrogen atom; or
• R¹ together with R² form the group -NH-C(O)-CH=CH-, wherein the nitrogen atom is bound to the carbon atom in the phenyl ring holding R¹ and the carbon atom is bound to the carbon atom in the phenyl ring holding R²
• R^{3a} and R^{3b} are independently selected from the group consisting of hydrogen atoms and C₁₋₄ alkyl groups
• X and Y are independently selected from the group consisting of direct bond and oxygen atom
• n, m and q each independently has a value selected from 0, 1, 2 and 3
• p has a value selected from 1, 2 and 3
• R⁴ and R⁵ are independently selected from hydrogen atoms, halogen atoms, C₁₋₄ alkyl, C₁₋₄ alkoxy, -CONH₂, -NHCONH₂, -SR⁷, -SOR⁷, -SO₂R⁷, -SO₂NHR⁸ and the groups 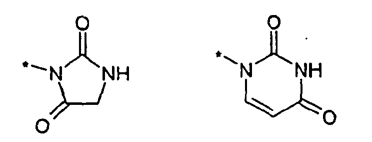 wherein R⁷ is selected from C₁₋₄ alkyl and C₃₋₈ cycloalkyl and R⁸ is selected from hydrogen atoms and C₁₋₄ alkyl groups
• R⁶ is selected from the group consisting of hydrogen atoms, halogen atoms, C₁₋₄ alkyl and C₁₋₄ alkoxy
or a pharmaceutically-acceptable salt or solvate or stereoisomer thereof

2. A compound according to claim 1 wherein p is 1, and/or n is 0, and/or X represents an oxygen atom, and/or Y represents a direct bond.

3. A compound according to any preceding claim wherein m has a value of 1 or 2, preferably 1.

4. A compound according to any preceding claim wherein q has a value of 0 or 1, preferably 0.

5. A compound according to any preceding claim wherein R^{3a} represents a hydrogen atom and R^{3b} is a hydrogen atom or methyl group, preferably a hydrogen atom.

6. A compound according to any preceding claim wherein R⁴ represents a hydrogen atom.

7. A compound according to any preceding claim wherein R⁴ represents a hydrogen atom and R⁵ is a hydrogen atom, halogen atom, -CONH₂, -NHCONH₂, - SR⁷, -SOR⁷ -SO₂R⁷ or-SO₂NHR⁸ group, preferably a hydrogen atom, -CONH₂ or -NHCONH₂ group.

8. A compound according to any preceding claim wherein R⁶ is a hydrogen atom or methoxy group, preferably a hydrogen atom.

9. A compound according to any preceding claim wherein R¹ together with R² form the group -NH-C(O)-CH=CH-, wherein the nitrogen atom is bound to the carbon atom in the phenyl ring holding R¹ and the carbon atom is bound to the carbon atom in the phenyl ring holding R², and wherein n and q preferably have a value of 0 and m and p preferably have a value of 1.

10. A compound according to any preceding claim wherein X represents an oxygen atom, Y represents a direct bond and R⁴, R⁵ and R⁶ independently represent hydrogen atoms.

11. A compound according to claim 1 which is selected from the group consisting of:
5-[(1*R*,*S*)-2-({(1*R,S*)-2-[4-(2,2-difluoro-2-phenylethoxy)phenyl]-1-methyl ethyl}amino)-1-hydroxyethyl]-8-hydroxyquinolin-2(1*H*)-one
4-[(1*R,S*)-2-({(1*R,S*)-2-[4-(2,2-Difluoro-2-phenylethoxy)phenyl]-1-methyl ethyl}amino)-1-hydroxyethyl]-2-(hydroxymethyl)phenol acetate
Formic acid - {5-[(1*R,S*)-2-({(1*R,S*)-2-[4-(2,2-Difluoro-2-phenylethoxy) phenyl]-1-methylethyl}amino)-1-hydroxyethyl]-2-hydroxyphenyl} formamide (1:1)
5-((1*R*)-2-({(1*R,S*)-2-[3-(2,2-difluoro-2-phenylethoxy)phenyl]-1-methylethyl}amino)-1-hydroxyethyl]-8-hydroxyquinolin-2(1*H*)-one
4-[(1*R*)-2-({(1*R,S*)-2-[3-(2,2-difluoro-2-phenylethoxy)phenyl]-1-methylethyl}amino)-1-hydroxyethyl]-2-(hydroxymethyl)phenol
4-{((1*R*)-2-[((1*R,S*)-2-{3-[(2,2-Difluoro-2-phenylethoxy)methyl]phenyl}-1-methylethyl)amino]-1-hydroxyethyl}-2-(hydroxymethyl)phenol acetate
5-[2-({2-[(1R,S)-4-(2,2-difluoro-2-phenylethoxy)phenyl]ethyl}amino)-1-hydroxyethyl]-8-hydroxyquinolin-2(1H)-one
5-[(1*R*)-2-({2-[4-(2,2-Difluoro-2-phenylethoxy)phenyl]ethyl}amino)-1-hydroxyethyl]-8-hydroxyquinolin-2(1*H*)-one
4-[(1*R,S*)-2-({2-[4-(2,2-Difluoro-2-phenylethoxy)phenyl]ethyl}amino)-1-hydroxyethyl]-2-(hydroxymethyl)phenol
{5-[(1*R,S*)-2-({2-[4-(2,2-Difluoro-2-phenylethoxy)phenyl]-1-methylethyl} amino)-1-hydroxyethyl]-2-hydroxyphenyl}formamide - formate
5-[(1*R,S*)-2-({2-[3-(2,2-Difluoro-2-phenylethoxy)phenyl]ethyl}amino)-1-hydroxyethyl]-8-hydroxyquinolin-2(1*H*)-one
5-[(1R)-2-({2-[3-(2,2-difluoro-2-phenylethoxy)phenyl]ethyl}amino)-1-hydroxyethyl]-8-hydroxyquinolin-2(1H)-one
4-[(1*R,S*)-2-({2-[3-(2,2-Difluoro-2-phenylethoxy)phenyl]ethyl}amino)-1-hydroxyethyl]-2-(hydroxymethyl)phenol
{5-[(1*R,S*)-2-({2-[3-(2,2-Difluoro-2-phenylethoxy)phenyl]ethyl}amino)-1-hydroxyethyl]-2-hydroxyphenyl}formamide
5-{(1*R,S*)-2-[(2-{4-[2,2-Difluoro-2-(2-methoxyphenyl)ethoxy]phenyl} ethyl)amino]-1-hydroxyethyl}-8-hydroxyquinolin-2(1*H*)-one
5-[(1*R*)-2-({(1*R,S*)-2-[4-(2,2-difluoro-3-phenylpropoxy)phenyl]-1-methylethyl}amino)-1-hydroxyethyl]-8-hydroxyquinolin-2(1*H*)-one
5-[(1*R,S*)-2-{[4-(3,3-Difluoro-3-phenylpropoxy)benzyl]amino}-1-hydroxyethyl)]-8-hydroxyquinolin-2(1H)-one
5-[(1*R,S*)-[2-({2-[4-(2,2-difluoro-3-phenoxypropoxy)phenyl]ethyl}-amino)-1-hydroxyethyl]-8-hydroxyquinolin-2(1*H*)-one
5-[(1*R,S*)-[[2-({2-[4-(2,2-difluoro-2-phenylethoxy)-3-methoxyphenyl]-ethyl}amino)-1-hydroxyethyl]-8-hydroxyquinolin-2(1*H*)-one, formate 5-[(1*R,S*)-2-({2-[4-(2,2-difluoro-3-phenylpropoxy)phenyl]ethyl}amino)-1-hydroxyethyl]-8-hydroxyquinolin-2(1*H*)-one, formate
5-[(1R,S)-2-({2-[4-(2,2-difluoro-4-phenylbutoxy)phenyl]-1-methylethyl}amino)-1-hydroxyethyl]-8-hydroxyquinolin-2(1H)-one,
5-[(1R)-2-({(1*R,S*)-2-[4-(1,1-difluoro-2-phenoxyethyl)phenyl]-1-methylethyl}amino)-1-hydroxyethyl]-8-hydroxyquinolin-2(1H)-one
5-[(1R)-2-({2-[4-(3,3-difluoro-3-phenylpropoxy)phenyl]ethyl}amino)-1-hydroxyethyl]-8-hydroxyquinolin-2(1H)-one
5-[(1*R,S*)-2-({2-[4-(4,4-difluoro-4-phenylbutoxy)phenyl]ethyl}amino)-1-hydroxyethyl]-8-hydroxyquinolin-2(1H)-one
5-[(1*R,S*)-2-({2-[4-(2,2-difluoro-2-phenylethoxy)-3-methylphenyl]ethyl}amino)-1-hydroxyethyl]-8-hydroxyquinolin-2(1H)-one
5-[(1*R,S*)-2-({2-[4-(2,2-difluoro-2-phenylethoxy)-3-fluorophenyl]ethyl}amino)-1-hydroxyethyl]-8-hydroxyquinolin-2(1H)-one
3-{1,1-difluoro-2-[4-((1*R,S*)-2-{[2-hydroxy-2-(8-hydroxy-2-oxo-1,2-dihydroquinolin-5-yl)ethyl]amino}ethyl)phenoxy]ethyl}benzamide
N-((1*R,S*)-3-{1,1-difluoro-2-[3-(2-{[(2R)-2-hydroxy-2-(8-hydroxy-2-oxo-1,2-dihydroquinolin-5-yl)ethyl]amino}propyl)phenoxy]ethyl}phenyl)urea
5-{(1*R,S*)-2-[(2-{4-[2,2-difluoro-2-(3-fluorophenyl)ethoxy]phenyl}ethyl)amino]-1-hydroxyethyl}-8-hydroxyquinolin-2(1H)-one
5-((1*R,S*)-(2-{[2-(4-{2-[3-(cyclopentylthio)phenyl]-2,2-difluoroethoxy}phenyl)ethyl]amino}-1-hydroxyethyl)-8-hydroxyquinolin-2(1H)-one
5-((1R,S)-(2-{[2-(4-{2-[3-(cyclopentylsulfonyl)phenyl]-2,2-difluoroethoxy}phenyl)ethyl]amino}-1-hydroxyethyl)-8-hydroxyquinolin-2(1H)-one
5-[(1*R,S*)-2-({2-[4-(2,2-difluoro-2-phenylethoxy)phenyl]-1,1-dimethylethyl}amino)-1-hydroxyethyl]-8-hydroxyquinolin-2(1H)-one
and pharmaceutically-acceptable salts and solvates thereof.

12. A pharmaceutical composition comprising a therapeutically effective amount of a compound according to anyone of claims 1 to 11 and a pharmaceutically acceptable carrier.

13. The pharmaceutical composition of claim 12, wherein the composition further comprises a therapeutically effective amount of one or more other therapeutic agents, wherein the other therapeutic agent is preferably a corticosteroid, an antichlolinergic agent, or a PDE4 inhibitor.

14. A compound as defined in any one of claims 1 to 11 or a pharmaceutical composition as defined in any one of claims 12 to 13 for use in treating a disease or condition in a mammal associated with β2 adrenergic receptor activity.

15. The compound or pharmaceutical composition of claim 14 wherein the disease or condition is (a) a pulmonary disease, preferably asthma or chronic obstructive pulmonary disease, or (b) a disease or condition selected from the group consisting of pre-term labor, glaucoma, neurological disorders, cardiac disorders, and inflammation.

## Patentansprüche

1. Verbindung der Formel (I): 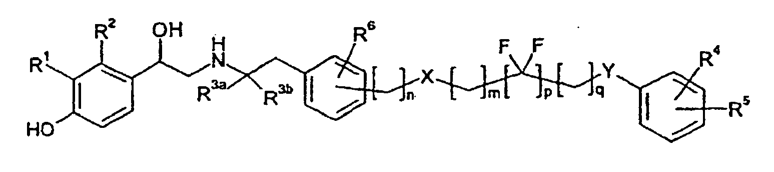 worin:
• R¹ eine Gruppe ist, ausgewählt aus -CH₂OH, -NH(CO)H und
• R² ein Wasserstoffatom ist oder
• R¹ zusammen mit R² die Gruppe -NH-C(O)-CH=CH- bilden, wobei das Stickstoffatom an das Kohlenstoffatom in dem Phenylring, das R¹ trägt, gebunden ist und das Kohlenstoffatom an das Kohlenstoffatom in dem Phenylring, das R² trägt, gebunden ist,
• R^{3a} und R^{3b} unabhängig ausgewählt sind aus der Gruppe, bestehend aus Wasserstoffatomen und C₁₋₄-Alkylgruppen,
• X und Y unabhängig ausgewählt sind aus der Gruppe, bestehend aus einer direkten Bindung und einem Sauerstoffatom,
• n, m und q jeweils unabhängig einen Wert, ausgewählt aus 0, 1, 2 und 3 haben,
• p einen Wert hat, der ausgewählt ist aus 1, 2 und 3,
• R⁴ und R⁵ unabhängig ausgewählt sind aus Wasserstoffatomen, Halogenatomen, C₁₋₄-Alkyl, C₁₋₄-Alkoxy, -CONH₂, -NHCONH₂, -SR⁷, -SOR⁷, -SO₂R⁷, -SO₂NHR⁸ und den Gruppen 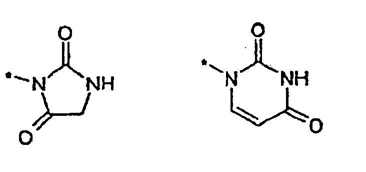 worin R⁷ aus C₁₋₄-Alkyl und C₃₋₈-Cycloalkyl ausgewählt ist und R⁸ aus Wasserstoffatomen und C₁₋₄-Alkylgruppen ausgewählt ist,
• R⁶ aus der Gruppe, bestehend aus Wasserstoffatomen, Halogenatomen, C₁₋₄-Alkyl und C₁₋₄-Alkoxy, ausgewählt ist,
oder ein pharmazeutisch verträgliches Salz oder Solvat oder Stereoisomer davon.

2. Verbindung nach Anspruch 1, wobei p 1 ist und/oder n 0 ist und/oder X ein Sauerstoffatom darstellt und/oder Y eine direkte Bindung darstellt.

3. Verbindung nach einem vorangehenden Anspruch, wobei m den Wert 1 oder 2, vorzugsweise 1, hat.

4. Verbindung nach einem vorangehenden Anspruch, wobei q den Wert 0 oder 1, vorzugsweise 0, hat.

5. Verbindung nach einem vorangehenden Anspruch, wobei R^{3a} ein Wasserstoffatom darstellt und R^{3b} ein Wasserstoffatom oder eine Methylgruppe, vorzugsweise ein Wasserstoffatom, ist.

6. Verbindung nach einem vorangehenden Anspruch, wobei R⁴ ein Wasserstoffatom darstellt.

7. Verbindung nach einem vorangehenden Anspruch, wobei R⁴ ein Wasserstoffatom darstellt und R⁵ ein Wasserstoffatom, Halogenatom, eine -CONH₂-, -NHCONH₂-, -SR⁷-, -SOR⁷-, -SO₂R⁷- oder -SO₂NHR⁸-Gruppe, vorzugsweise ein Wasserstoffatom, eine -CONH₂- oder -NHCONH₂-Gruppe, darstellt.

8. Verbindung nach einem vorangehenden Anspruch, wobei R⁶ ein Wasserstoffatom oder eine Methoxygruppe, vorzugsweise ein Wasserstoffatom, ist.

9. Verbindung nach einem vorangehenden Anspruch, wobei R¹ zusammen mit R² die Gruppe -NH-C(O)-CH=CH- bildet, wobei das Stickstoffatom an das Kohlenstoffatom in dem Phenylring, das R¹ trägt, gebunden ist, und das Kohlenstoffatom an das Kohlenstoffatom in dem Phenylring, das R² trägt, gebunden ist, und wobei n und q vorzugsweise den Wert 0 haben und m und p vorzugsweise den Wert 1 haben.

10. Verbindung nach einem vorangehenden Anspruch, wobei X ein Sauerstoffatom darstellt, Y eine direkte Bindung darstellt und R⁹, R⁵ und R⁶ unabhängig Wasserstoffatome darstellen.

11. Verbindung nach Anspruch 1, welche ausgewählt ist aus der Gruppe, bestehend aus:
5-[(1R,S)-2-({(1R,S)-2-[4-(2,2-Difluor-2-phenylethoxy)phenyl]-1-methylethyl}amino)-1-hydroxyethyl]-8-hydroxychinolin-2(1H)-on
4-[(1R,S)-2-({(1R,S)-2-[4-(2,2-Difluor-2-phenylethoxy)phenyl]-1-methylethyl}amino)-1-hydroxyethyl]-2-(hydroxymethyl)phenolacetat
Ameisensäure-{5-[(1R,S)-2-({(1R,S)-2-[4-(2,2-Difluor-2-phenylethoxy)phenyl]-1-methylethyl}amino)-1-hydroxyethyl]-2-hydroxyphenyl}formamid (1:1)
5-((1R)-2-({(1R,S)-2-[3-(2,2-Difluor-2-phenylethoxy)phenyl]-1-methylethyl}amino)-1-hydroxyethyl]-8-hydroxychinolin-2(1H)-on
4-[(1R)-2-({(1R,S)-2-[3-(2,2-Difluor-2-phenylethoxy)phenyl]-1-methylethyl}amino)-1-hydroxyethyl]-2-(hydroxymethyl)phenol 4-{(1R)-2-[((1R,S)-2-{3-[(2,2-Difluor-2-phenylethoxy)methyl]phenyl}-1-methylethyl)amino]-1-hydroxyethyl}-2-(hydroxymethyl)phenolacetat
5-[2-({2-[(1R,S)-4-(2,2-Difluor-2-phenylethoxy)phenyl]ethyl}amino)-1-hydroxyethyl]-8-hydroxychinolin-2(1H)-on
5-[(1R)-2-({2-[4-(2,2-Difluor-2-phenylethoxy)phenyl]ethyl}amino)-1-hydroxyethyl]-8-hydroxychinolin-2(1H)-on
4-[(1R,S)-2-({2-[4-(2,2-Difluor-2-phenylethoxy)phenyl]ethyl}amino)-1-hydroxyethyl]-2-(hydroxymethyl)phenol
{5-[(1R,S)-2-({2-[4-(2,2-Difluor-2-phenylethoxy)phenyl]-1-methylethyl}amino)-1-hydroxyethyl]-2-hydroxyphenyl}formamid-formiat
5-[(1R,S)-2-({2-[3-(2,2-Difluor-2-phenylethoxy)phenyl]ethyl}amino)-1-hydroxyethyl]-8-hydroxychinolin-2(1H)-on
5-[(1R)-2-({2-[3-(2,2-Difluor-2-phenylethoxy)phenyl]ethyl}amino)-1-hydroxyethyl]-8-hydroxychinolin-2(1H)-on
4-[(1R,S)-2-({2-[3-(2,2-Difluor-2-phenylethoxy)phenyl]ethyl}amino)-1-hydroxyethyl]-2-(hydroxymethyl)phenol
{5-[(1R,S)-2-({2-[3-(2,2-Difluor-2-phenylethoxy)phenyl]ethyl}amino)-1-hydroxyethyl]-2-hydroxyphenyl}formamid
5-{(1R,S)-2-[(2-{4-[2,2-Difluor-2-(2-methoxyphenyl)ethoxy]phenyl}ethyl)amino]-1-hydroxyethyl}-8-hydroxychinolin-2(1H)-on
5-[(1R)-2-({(1R,S)-2-[4-(2,2-Difluor-3-phenylpropoxy)phenyl]-1-methylethyl}amino)-1-hydroxyethyl]-8-hydroxychinolin-2(1H)-on
5-[(1R,S)-2-{[4-(3,3-Difluor-3-phenylpropoxy)benzyl]amino}-1-hydroxyethyl)]-8-hydroxychinolin-2(1H)-on
5-[(1R,S)-[2-({2-[4-(2,2-Difluor-3-phenoxypropoxy)phenyl]ethyl}-amino)-1-hydroxyethyl]-8-hydroxychinolin-2(1H)-on
5-[(1R,S)-[[2-({2-[4-(2,2-Difluor-2-phenylethoxy)-3-methoxyphenyl]ethyl}amino)-1-hydroxyethyl]-8-hydroxychinolin-2(1H)-on, Formiat
5-[(1R,S)-2-({2-[4-(2,2-Difluor-3-phenylpropoxy)phenyl]ethyl}amino)-1-hydroxyethyl]-8-hydroxychinolin-2(1H)-on, Format
5-[(1R,S)-2-({2-[4-(2,2-Difluor-4-phenylbutoxy)phenyl]-1-methylethyl}amino)-1-hydroxyethyl]-8-hydroxychinolin-2(1H)-on,
5-[(1R)-2-({(1R,S)-2-[4-(1,1-Difluor-2-phenoxyethyl)phenyl]-1-methylethyl}amino)-1-hydroxyethyl]-8-hydroxychinolin-2(1H)-on
5-[(1R)-2-({2-[4-(3,3-Difluor-3-phenylpropoxy)phenyl]ethyl}amino)-1-hydroxyethyl]-8-hydroxychinolin-2(1H)-on
5-[(1R,S)-2-({2-[4-(4,4-Difluor-4-phenylbutoxy)phenyl]ethyl}amino)-1-hydroxyethyl]-8-hydroxychinolin-2(1H)-on
5-[(1R,S)-2-({2-[4-(2,2-Difluor-2-phenylethoxy)-3-methylphenyl]ethyl}amino)-1-hydroxyethyl]-8-hydroxychinolin-2(1H)-on
5-[(1R,S)-2-({2-[4-(2,2-Difluor-2-phenylethoxy)-3-fluorphenyl]ethyl}amino)-1-hydroxyethyl]-8-hydroxychinolin-2(1H)-on
3-{1,1-Difluor-2-[4-((1R,S)-2-{[2-hydroxy-2-(8-hydroxy-2-oxo-1,2-dihydrochinolin-5-yl)ethyl]amino}ethyl)phenoxy]ethyl}benzamid
N-((1R,S)-3-{1,1-Difluor-2-[3-(2-{[(2R)-2-hydroxy-2-(8-hydroxy-2-oxo-1,2-dihydrochinolin-5-yl)ethyl]amino}propyl)phenoxy]ethyl}phenyl)harnstoff 5-{(1R,S)-2-[(2-{4-[2,2-Difluor-2-(3-fluorphenyl)ethoxy]phenyl}ethyl)amino]-1-hydroxyethyl}-8-hydroxychinolin-2(1H)-on
5-((1R,S)-(2-{[2-(4-{2-[3-(Cyclopentylthio)phenyl]-2,2-difluorethoxy}phenyl)ethyl]amino}-1-hydroxyethyl)-8-hydroxychinolin-2(1H)-on
5-((1R,S)-(2-{[2-(4-{2-[3-(Cyclopentylsulfonyl)phenyl]-2,2-difluorethoxy}phenyl)ethyl]amino}-1-hydroxyethyl)-8-hydroxychinolin-2(1H)-on
5-[(1R,S)-2-({2-[4-(2,2-Difluor-2-phenylethoxy)phenyl]-1,1-dimethylethyl}amino)-1-hydroxyethyl]-8-hydroxychinolin-2(1H)-on
und pharmazeutisch verträglichen Salzen und Solvaten davon.

12. Pharmazeutische Zusammensetzung, umfassend eine therapeutisch wirksame Menge einer Verbindung nach einem der Ansprüche 1 bis 11 und einen pharmazeutisch verträglichen Träger.

13. Pharmazeutische Zusammensetzung nach Anspruch 12, wobei die Zusammensetzung außerdem eine therapeutisch wirksame Menge eines anderen therapeutischen Mittels oder mehrerer anderer therapeutischer Mittel umfasst, wobei das andere therapeutische Mittel vorzugsweise ein Corticosteroid, ein anticholinerges Mittel oder ein PDE4-Inhibitor ist.

14. Verbindung, wie sie in einem der Ansprüche 1 bis 11 definiert ist, oder eine pharmazeutische Zusammensetzung, wie sie in einem der Ansprüche 12 bis 13 definiert ist, zur Verwendung bei der Behandlung einer Erkrankung oder eines Zustandes bei einem Säuger, die/der mit β2-adrenerger Rezeptoraktivität assoziiert ist.

15. Verbindung oder pharmazeutische Zusammensetzung nach Anspruch 14, wobei die Erkrankung oder der Zustand (a) eine Lungenerkrankung, vorzugsweise Asthma oder chronischobstruktive Lungenerkrankung, oder (b) eine Erkrankung oder ein Zustand, ausgewählt aus der Gruppe, bestehend aus Frühgeburt, Glaukom, neurologischen Störungen, Störungen des Herzens und Entzündung, ist.

## Revendications

1. Composé de formule (I) : 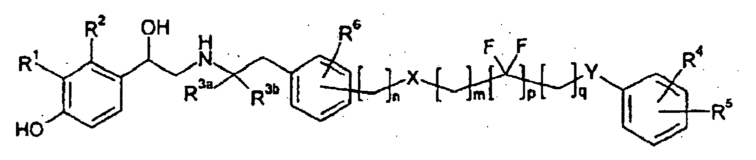 dans laquelle :
• R¹ est un groupe choisi parmi -CH₂OH, -NH(CO)H et
• R² est un atome d'hydrogène ; ou
• R¹ conjointement avec R² forment le groupe -NH-C(O)-CH=CH-, où l'atome d'azote est lié à l'atome de carbone dans le noyau phényle contenant R¹ et l'atome de carbone est lié à l'atome de carbone dans le noyau phényle contenant R²
• R^{3a} et R^{3b} sont indépendamment choisis dans le groupe constitué par les atomes d'hydrogène et les groupes alkyles en C₁₋₄
• X et Y sont indépendamment choisis dans le groupe constitué par une liaison directe et un atome d'oxygène
• n, m et q ont chacun indépendamment une valeur choisie parmi 0, 1, 2 et 3
• p a une valeur choisie parmi 1, 2 et 3
• R⁴ et R⁵ sont indépendamment choisis parmi des atomes d'hydrogène, des atomes d'halogène, un alkyle en C₁₋₄, un alcoxy en C₁₋₄, -CONH₂, -NHCONH₂, -SR⁷, -SOR⁷, -SO₂R⁷, -SO₂NHR⁸ et les groupes 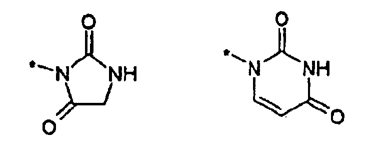 où R⁷ est choisi parmi un alkyle en C₁₋₄ et un cycloalkyle en C₃₋₈ et R⁸ est choisi parmi des atomes d'hydrogène et des groupes alkyles en C₁₋₄
• R⁶ est choisi dans le groupe constitué par des atomes d'hydrogène, des atomes d'halogène, un alkyle en C₁₋₄ et un alcoxy en C₁₋₄
ou un sel ou solvate ou stéréo-isomère pharmaceutiquement acceptable de celui-ci.

2. Composé selon la revendication 1, dans lequel p est 1 et/ou n est 0 et/ou X représente un atome d'oxygène et/ou Y représente une liaison directe.

3. Composé selon l'une quelconque des revendications précédentes, dans lequel m a une valeur de 1 ou 2, de préférence 1.

4. Composé selon l'une quelconque des revendications précédentes, dans lequel q a une valeur de 0 ou 1, de préférence 0.

5. Composé selon l'une quelconque des revendications précédentes, dans lequel R^{3a} représente un atome d'hydrogène et R^{3b} est un atome d'hydrogène ou un groupe méthyle, de préférence un atome d'hydrogène.

6. Composé selon l'une quelconque des revendications précédentes, dans lequel R⁴ représente un atome d'hydrogène.

7. Composé selon l'une quelconque des revendications précédentes, dans lequel R⁴ représente un atome d'hydrogène et R⁵ est un atome d'hydrogène, un atome d'halogène, un groupe -CONH₂, -NHCONH₂, -SR⁷, -SOR⁷, -SO₂R⁷, -SO₂NHR⁸, de préférence un atome d'hydrogène, un groupe -CONH₂ ou -NHCONH₂.

8. Composé selon l'une quelconque des revendications précédentes, dans lequel R⁶ est un atome d'hydrogène ou un groupe méthoxy, de préférence un atome d'hydrogène.

9. Composé selon l'une quelconque des revendications précédentes, dans lequel R¹ conjointement avec R² forment le groupe -NH-C(O)-CH=CH-, dans lequel l'atome d'azote est lié à l'atome de carbone dans le noyau phényle contenant R¹ et l'atome de carbone est lié à l'atome de carbone dans le noyau phényle contenant R², et dans lequel n et q ont de préférence une valeur de 0 et m et p ont de préférence une valeur de 1.

10. Composé selon l'une quelconque des revendications précédentes, dans lequel X représente un atome d'oxygène, Y représente une liaison directe et R⁴, R⁵ et R⁶ représentent indépendamment des atomes d'hydrogène.

11. Composé selon la revendication 1, qui est choisi dans le groupe constitué par :
la 5-[(1*R,S*)-2-({(1*R,S*)-2-[4-(2,2-difluoro-2-phényl-éthoxy)phényl]-1-méthyléthyl}amino)-1-hydroxyéthyl]-8-hydroxyquinoléin-2(1*H*)one
l'acétate de 4-[(1*R,S*)-2-({(1*R,S*)-2-[4-(2,2-difluoro-2-phényléthoxy)phényl]-1-méthyléthyl}amino)-1-hydroxyéthyl]-2-(hydroxyméthyl)phénol
l'acide formique - formamide de {5-[(1*R,S*)-2-({(1*R,S*)-2-[4-(2,2-difluoro-2-phényléthoxy)phényl]-1-méthyléthyl}-amino)-1-hydroxyéthyl]-2-hydroxyphényle} (1:1)
la 5-((1*R*)-2-({(1*R,S*)-2-[3-(2,2-difluoro-2-phényl-éthoxy)phényl]-1-méthyléthyl}amino)-1-hydroxyéthyl]-8-hydroxyquinoléin-2(1*H*)one
le 4-[(1*R*)-2-({(1*R,S*)-2-[3-(2,2-difluoro-2-phényl-éthoxy)phényl]-1-méthyléthyl}amino)-1-hydroxyéthyl]-2-(hydroxyméthyl)phénol
l'acétate de 4-{(1*R*)-2-[((1*R,S*)-2-{3-[(2,2-difluoro-2-phényléthoxy)méthyl]phényl]-1-méthyléthyl)amino]-1-hydroxy-éthyl}-2-(hydroxyméthyl)phénol
la 5-[2-({2-[(1R,S)-4-(2,2-difluoro-2-phényléthoxy)-phényl]éthyl}amino)-1-hydroxyéthyl]-8-hydroxyquinoléin-2(1H)one
la 5-[(1*R*)-2-({-2-[4-(2,2-difluoro-2-phényléthoxy)-phényl]éthyl}amino)-1-hydroxyéthyl]-8-hydroxyquinoléin-2(1*H*)one
le 4-[(1*R,S*)-2-({2-[4-(2,2-difluoro-2-phényléthoxy)-phényl]éthyl}amino)-1-hydroxyéthyl]-2-(hydroxyméthyl)phénol
le formamide {5-[(1*R,S*)-2-({2-[4-(2,2-difluoro-2-phényléthoxy)phényl]-1-méthyléthyl}amino)-1-hydroxyéthyl]-2-hydroxyphényle} - formiate
la 5-[(1*R,S*)-2-({2-[3-(2,2-difluoro-2-phényléthoxy)-phényl]éthyl}amino)-1-hydroxyéthyl]-8-hydroxyquinoléin-2(1*H*)one
la 5-[(1R)-2-({2-[3-(2,2-difluoro-2-phényléthoxy)-phényl]éthyl}amino)-1-hydroxyéthyl]-8-hydroxyquinoléin-2(1H)one
le 4-[(1*R,S*)-2-({2-[3-(2,2-difluoro-2-phényléthoxy)-phényl]éthyl}amino)-1-hydroxyéthyl]-2-(hydroxyméthyl)phénol
le formamide de {5-[(1*R,S*)-2-({2-[3-(2,2-difluoro-2-phényléthoxy)phényl]éthyl}amino)-1-hydroxyéthyl]-2-hydroxyphényle}
la 5-{(1*R,S*)-2-[(2-{4-[2,2-difluoro-2-(2-méthoxy-phényl)éthoxy)phényl}éthyl)amino]-1-hydroxyéthyl}-8-hydroxyquinoléin-2(1*H*)one
la 5-[(1*R*)-2-({(1*R,S*)-2-[4-(2,2-difluoro-3-phényl-propoxy)phényl]-1-méthyléthyl}amino)-1-hydroxyéthyl]-8-hydroxyquinoléin-2(1*H*)one
la 5-[(1*R,S*)-2-{[4-(3,3-difluoro-3-phénylpropoxy)-benzyl]amino}-1-hydroxyéthyl)]-8-hydroxyquinoléin-2(1H)one
la 5-[(1*R,S*)-[2-({2-[4-(2,2-difluoro-3-phénoxypropoxy)-phényl]éthyl}amino)-1-hydroxyéthyl]-8-hydroxyquinoléin-2(1*H*)one
le 5-[(1*R,S*)-[[2-({2-[4-(2,2-difluoro-2-phényléthoxy)-3-méthoxyphényl]éthyl}amino)-1-hydroxyéthyl]-8-hydroxy-quinoléin-2(1*H*)-one, formiate
le 5-[(1*R,S*)-2-({2-[4-(2,2-difluoro-3-phénylpropoxy)-phényl]éthyl}amino)-1-hydroxyéthyl]-8-hydroxyquinoléin-2(1*H*)-one, formiate
la 5-[(1R,S)-2-({2-[4-(2,2-difluoro-4-phénylbutoxy)-phényl]-1-méthyléthyl}amino)-1-hydroxyéthyl]-8-hydroxy-quinoléin-2(1H)-one
la 5-[(1R)-2-({(1*R,S*)-2-[4-(1,1-difluoro-2-phénoxy-éthyl)phényl]-1-méthyléthyl}amino)-1-hydroxyéthyl]-8-hydroxyquinoléin-2(1H)-one
la 5-[(1R)-2-({2-[4-(3,3-difluoro-3-phénylpropoxy)-phényl]éthyl}amino)-1-hydroxyéthyl]-8-hydroxyquinoléin-2(1H)-one
la 5-[(1*R,S*)-2-({2-[4-(4,4-difluoro-4-phénylbutoxy)-phényl]éthyl}amino)-1-hydroxyéthyl]-8-hydroxyquinoléin-2(1H)-one
la 5-[(1*R,S*)-2-({2-[4-(2,2-difluoro-2-phényléthoxy)-3-méthylphényl]éthyl}amino)-1-hydroxyéthyl]-8-hydroxy-quinoléin-2(1H)-one
la 5-[(1*R,S*)-2-({2-[4-(2,2-difluoro-2-phényléthoxy)-3-fluorophényl]éthyl}amino)-1-hydroxyéthyl]-8-hydroxy-quinoléin-2(1H)-one
le 3-{1,1-difluoro-2-[4-((1*R,S*)-2-{[2-hydroxy-2-(8-hydroxy-2-oxo-1,2-dihydroquinoléin-5-yl)éthyl]amino}éthyl)-phénoxy]éthyl}benzamide
la N-((1*R,S*)-3-{1,1-difluoro-2-[3-(2-{[(2R)-2-hydroxy-2-(8-hydroxy-2-oxo-1,2-dihydroquinoléin-5-yl)éthyl]amino}-propyl)phénoxy]éthyl}phényl)urée
la 5-{(1*R,S*)-2-[(2-{4-[2,2-difluoro-2-(3-fluorophényl)-éthoxy]phényl}éthyl)amino]-1-hydroxyéthyl}-8-hydroxy-quinoléin-2(1H)-one
la 5-((1*R,S*)-(2-{[2-(4-{2-[3-(cyclopentylthio)phényl]-2,2-difluoroéthoxy}phényl)éthyl]amino}-1-hydroxyéthyl)-8-hydroxyquinoléin-2(1H)-one
la 5-((1R,S)-(2-{[2-(4-{2-[3-(cyclopentylsulfonyl)-phényl]-2,2-difluoroéthoxy}phényl)éthyl]amino}-1-hydroxy-éthyl)-8-hydroxyquinoléin-2(1H)-one
la 5-[(1*R,S*)-2-({2-[4-(2,2-difluoro-2-phényléthoxy)-phényl]-1,1-diméthyléthyl}amino)-1-hydroxyéthyl]-8-hydroxy-quinoléin-2(1H)-one
et les sels et solvates pharmaceutiquement acceptable de celui-ci.

12. Composition pharmaceutique comprenant une quantité thérapeutiquement efficace d'un composé selon l'une quelconque des revendications 1 à 11 et un support pharmaceutiquement acceptable.

13. Composition pharmaceutique selon la revendication 12, laquelle composition comprend en outre une quantité thérapeutiquement efficace d'un ou plusieurs autres agents thérapeutiques, dans laquelle l'autre agent thérapeutique est de préférence un corticostéroïde, un agent anticholinergique ou un inhibiteur de la PDE4.

14. Composé selon l'une quelconque des revendications 1 à 11 ou composition pharmaceutique selon l'une quelconque des revendications 12 à 13, pour une utilisation dans le traitement d'une maladie ou affection chez un mammifère associée à l'activité du récepteur β2 adrénergique.

15. Composé ou composition pharmaceutique selon la revendication 14, dans laquelle la maladie ou affection est (a) une maladie pulmonaire, de préférence un asthme ou une bronchopneumopathie obstructive chronique, ou (b) une maladie ou affection choisie dans le groupe constitué par un travail prématuré, un glaucome, des troubles neurologiques, des troubles cardiaques et une inflammation.
